(19) Europäisches Patentamt
European Patent Office
Office européen des brevets

(11) **EP 1 155 013 B1**

(12) # FASCICULE DE BREVET EUROPEEN

(45) Date de publication et mention de la délivrance du brevet:
**23.07.2003 Bulletin 2003/30**

(21) Numéro de dépôt: **00906414.8**

(22) Date de dépôt: **17.02.2000**

(51) Int Cl.[7]: **C07D 401/14**, C07D 401/12, A61K 31/47

(86) Numéro de dépôt international:
**PCT/FR00/00396**

(87) Numéro de publication internationale:
**WO 00/050418 (31.08.2000 Gazette 2000/35)**

(54) **COMPOSES HETEROCYCLIQUES DE BENZENESULFONAMIDE EN TANT QU'ANTAGONISTES DE LA BRADYKININE**

HETEROCYCLISCHE BENZOLSULFONAMIDDERIVATE ALS BRADYKININ ANTAGONISTEN

HETEROCYCLIC BENZENESULPHONAMIDE COMPOUNDS AS BRADYKININE ANTAGONISTS

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **26.02.1999 FR 9902412**

(43) Date de publication de la demande:
**21.11.2001 Bulletin 2001/47**

(73) Titulaire: **FOURNIER INDUSTRIE ET SANTE**
**F-75008 Paris (FR)**

(72) Inventeurs:
- **DODEY, Pierre**
  **F-21121 Fontaine-lès-Dijon (FR)**
- **BARTH, Martine**
  **F-21380 Asnieres les Dijon (FR)**
- **BONDOUX, Michel**
  **F-21121 Fontaine-lès-Dijon (FR)**

(74) Mandataire: **Hubert, Philippe**
**Cabinet Beau de Loménie**
**158, rue de l'Université**
**75340 Paris Cédex 07 (FR)**

(56) Documents cités:
**WO-A-97/41104**
**FR-A- 2 743 073**
**FR-A- 2 756 562**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 155 013 B1

## Description

### *Domaine de l'invention*

La présente invention concerne de nouveaux composés de benzènesulfonamide, leur procédé de préparation et leur utilisation en thérapeutique.

Ces nouveaux composés présentent une action antagoniste vis-à-vis de la bradykinine et sont utiles en thérapeutique, particulièrement pour le traitement de la douleur et de l'inflammation, et notamment dans le traitement de l'asthme, du choc traumatique cérébral et des rhinites allergiques.

### *Art antérieur*

On sait que l'une des possibilités de traitement de certaines pathologies à caractère douloureux et/ou inflammatoire (telles que l'asthme, la rhinite, le choc septique, la douleur dentaire, etc.) est d'inhiber l'action de certaines hormones telles que la bradykinine ou la kallidine. En effet ces hormones peptidiques sont impliquées dans un grand nombre de processus physiologiques dont certains sont liés de façon étroite à ces pathologies.

Bien qu'actuellement aucun produit possédant ce mode d'action ne soit encore commercialisé, de nombreuses études ont été entreprises pour comprendre le mode d'action des kinines et en particulier de la bradykinine et de ses homologues, puis pour créer des composés susceptibles d'être antagonistes des récepteurs de la bradykinine. Parmi les nombreuses publications relatant ces travaux, on peut citer Pharmacological Reviews Vol. 44 n° 1, pages 1-80 (1992) et Biopolymers (Peptide Science) vol. 37 pages 143-155 (1995).

La bradykinine est une hormone peptidique constituée de 9 aminoacides (Arg-Pro-Pro-Gly-Phe-Ser-Pro-Phe-Arg) et la kallidine est une hormone peptidique (Lys-Arg-Pro-Pro-Gly-Phe-Ser-Pro-Phe-Arg) qui comporte un aminoacide supplémentaire (Lys) par rapport à la bradykinine. On sait que des études antérieures ont permis d'obtenir des peptides qui interagissent avec les récepteurs de la bradykinine : certains comme le Bradycor (CP.0127 de la société Cortech), l'Icatibant (HOE 140 de la société Hoechst) ["Bradycor" et "Icatibant" sont des dénominations communes internationales (DCI)] ou encore le NPC 17761 (de la société Scios-Nova) présentent une action inhibitrice de la fixation de la bradykinine sur son récepteur $B_2$. Des publications récentes font état d'autres peptides susceptibles d'avoir une action antagoniste de la bradykinine vis-à-vis de son récepteur $B_2$ ; parmi celles-ci on peut citer par exemple WO-A-97/09347, WO-A-97/09346, US-A-5610140, US-A-5620958, US-A-5610142 et US-A-5597803. Par ailleurs, des composés non peptidiques ont été proposés comme antagonistes vis-à-vis de la fixation de la bradykinine sur son récepteur $B_2$, notamment dans EP-A-0596406, EP-A-0622361, US-A-5578601, US-A-5510380, FR-A-2735128, JP-A-09/040662, FR-A-2737892, WO-A-97/11069, WO-A-97/41104, WO-A-96/13485, FR-A- 2743073, FR-A-2756562 et FR-A-2765222. On sait en outre que certains composés de structure plus ou moins apparentée à celles des composés visés dans la présente demande ont déjà été décrits, notamment dans DE-A-3617183 et EP-A-0261539, eu égard à leurs éventuelles propriétés antithrombotiques.

### *But de l'invention*

Il existe un besoin d'atténuer ou de supprimer chez les mammifères et surtout chez l'homme les douleurs et les inflammations.

Pour satisfaire ce besoin, on a recherché une nouvelle solution technique qui soit efficace dans le traitement des algies quelle que soit leur origine, notamment dans le traitement des algies liées à des phénomènes inflammatoires ou à des traumatismes.

Selon l'invention, on se propose de fournir une nouvelle solution technique, qui met en oeuvre, au niveau du récepteur $B_2$ de la bradykinine, une fixation compétitive entre (i) la bradykinine et les hormones apparentées ou analogues, et (ii) une substance antagoniste, et qui fait appel à des composés de type benzènesulfonamide, structurellement différents des produits connus précités, et capables de limiter ou d'inhiber substantiellement la fixation de la bradykinine et des hormones analogues sur ledit récepteur $B_2$ de la bradykinine.

Suivant cette solution technique, les nouveaux composés se fixent de façon compétitive sur le récepteur $B_2$ de la bradykinine sans provoquer les effets de la bradykinine sur ce récepteur (ces nouveaux composés sont des substances dites antagonistes). Il s'en suit l'apparition d'un état analogue à celui observé en l'absence de bradykinine, à savoir une diminution de la douleur, des réactions inflammatoires et des autres effets néfastes provoqués par les récepteurs activés par la bradykinine.

Conformément à cette nouvelle solution technique on se propose de fournir, selon un premier aspect de l'invention, des composés dérivés de benzènesulfonamide en tant que produits industriels nouveaux ; selon un second aspect de l'invention, un procédé de préparation de ces composés ; et selon un troisième aspect de l'invention, une utilisation de ces composés notamment en thérapeutique en tant que principes actifs de spécialités ou compositions

médicamenteuses.

***Objet de l'invention***

Selon la nouvelle solution technique de l'invention, on préconise, en tant que produit industriel nouveau, un composé de benzènesulfonamide qui est caractérisé en ce qu'il est choisi parmi l'ensemble constitué par :

(i) les composés de formule I :

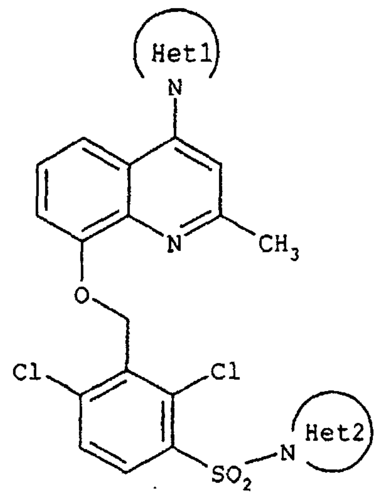

(I)

dans laquelle :

Het1 représente un hétérocycle azoté à 5 sommets, notamment l'imidazole, le pyrazole ou le triazole,
Het2 représente un hétérocycle azoté à 4, 5 ou 6 sommets de structure :

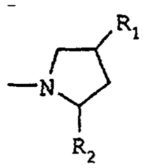 , 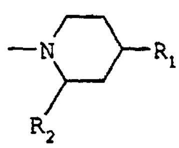 , 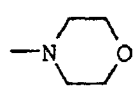 ,

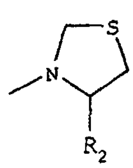 , 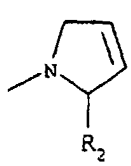 ou 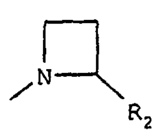

dans lesquels

$R_1$ représente un atome d'hydrogène ou un groupe hydroxy, alcoxy en $C_1$-$C_4$, phénoxy, phénylméthoxy, $-CH_2OH$, cycloalkyloxy, cycloalkylalcoxy (où chaque fragment cycloalkyle est en $C_3$-$C_8$ et le fragment alcoxy en $C_1$-$C_4$), $-NH-CO-CH_3$, $-CO-NH_2$ ou $-CO-NH-CH_3$,
$R_2$ représente un atome d'hydrogène ou un groupe $-CH_2OH$, $-CH_2-O-CH_3$, $-CONR_3R_4$,

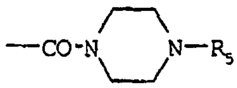 ou 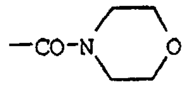

$R_3$ représente un atome d'hydrogène, un groupe alkyle en $C_1$-$C_3$, un groupe cycloalkyle en $C_3$-$C_8$, un groupe cycloalkyl (en $C_3$-$C_8$)-alkyle (en $C_1$-$C_3$), un groupe phényle, ou un groupe phénylméthyle,
$R_4$ représente un atome d'hydrogène, un groupe alkyle en $C_1$-$C_3$, -$(CH_2)$n-$CH_2OH$, -$(CH_2)$n-COOH, -$(CH_2)$n-$CH_2$-$NR_5R_6$,

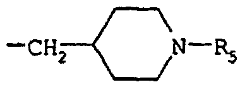 , 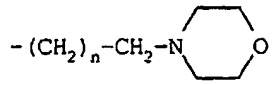 ou 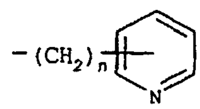

$R_5$ représente un atome d'hydrogène, un groupe alkyle en $C_1$-$C_3$, phényle, phénylméthyle, pyridinyle, pyridinylméthyle, pyridinyléthyle, benzoyle, 4-(aminoiminométhyl)benzoyle, -$(CH_2)_m$-$CH_2OH$, -$(CH_2)_m$-COOH, -$(CH_2)_m CH_2$-O-$(CH_2)_m$-$CH_2OH$, -CO-$(CH_2)_m$-COOH, ou

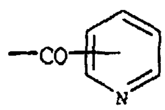

$R_6$ représente un atome d'hydrogène ou un groupe alkyle en $C_1$-$C_3$,

ou, $R_5$ et $R_6$ considérés ensemble forment, avec l'atome d'azote auquel ils sont attachés, un N-hétérocycle de 5 à 6 sommets,

n = 1, 2, 3 ou 4,

m = 1, 2 ou 3 ; et,

(ii) leurs sels d'addition.

Selon l'invention, on préconise aussi un procédé de préparation des composés de formule I et de leurs sels d'addition.
On préconise également l'utilisation d'une substance antagoniste du récepteur $B_2$ de la bradykinine, choisie parmi les composés de formule I de la présente invention et leurs sels d'addition non toxiques, pour l'obtention d'un médicament destiné à une utilisation en thérapeutique humaine ou animale, vis-à-vis de pathologies impliquant la bradykinine ou ses homologues, en particulier vis-à-vis des algies, notamment dans le traitement ou la prévention de pathologies liées à des états inflammatoires ou douloureux, et vis-à-vis des chocs traumatiques sévères, en particulier les chocs traumatiques cérébraux.

***Description détaillée de l'invention***

Dans la formule générale I des composés de l'invention, on entend par groupe alkyle en $C_1$-$C_3$ un groupe méthyle, éthyle, propyle ou 1-méthyléthyle.
Par groupe alcoxy en $C_1$-$C_4$, on entend préférentiellement ici les groupes méthoxy, éthoxy, propoxy, butoxy, 1-méthyléthoxy, et 1,1-diméthyl-éthoxy. Par groupe cycloalkyle en $C_3$-$C_8$, on comprend les groupes cyclopropyle, cyclobutyle, cyclopentyle, cyclohexyle, et par groupes (cycloalkyl)alkyle on entend notamment les groupes cyclopropylméthyle, cyclopropyléthyle, cyclohexylméthyle et cyclohexyléthyle.

Lorsqu'un groupe tel que $R_5$ comprend un hétérocycle, par exemple la pyridine, et que la position de substitution n'est pas précisée, il faut comprendre que la liaison avec l'hétérocycle peut se faire par l'un quelconque des sommets substituables.

Par hétérocycle $NR_5R_6$ de 5 à 6 sommets, on entend un cycle pyrrolidine, pipéridine, pipérazine ou morpholine, et plus particulièrement un groupe 1-pyrrolidinyle, 1-pipéridiynyle, 1-pipérazinyle ou 1-morpholinyle.

L'hétérocycle Het1 qui a cinq sommets comprend un ou plusieurs hétéroatomes. De façon avantageuse, il comprend 1 à 4 sommets azotés. Comme représenté par la formule I ci-dessus, Het1 est lié par son sommet azoté ou l'un de ses sommets azotés à la position 4 de la quinoléine.

L'hétérocycle Het2 est lié par son sommet azoté à l'atome de soufre du groupe $SO_2$ pour constituer la fonction sulfonamide.

Lorsque, sur l'hétérocycle Het2, le substituant $R_2$ n'est pas un atome d'hydrogène, le carbone du cycle porteur du substituant $R_2$ peut présenter une configuration S ou R. Dans ce cas, les composés selon l'invention peuvent être de configuration indéterminée (c'est-à-dire, un mélange des isomères R et S) ou, de préférence, l'un des isomères R ou S, ou, préférentiellement, l'isomère S. De même, le substituant $R_1$, lorsqu'il n'est pas l'hydrogène, introduit un centre d'asymétrie et peut se trouver dans une configuration indéterminée, ou déterminée R ou S, la configuration « trans » par rapport au groupe $R_2$ étant préférée

Par « sels d'addition », on entend les sels d'addition d'acide, obtenus par réaction d'un composé de formule I sous sa forme non salifiée, avec un acide minéral ou un acide organique. Les acides minéraux préférés pour salifier un composé basique de formule I sont les acides chlorhydrique, bromhydrique, phosphorique et sulfurique. Les acides organiques préférés pour salifier un composé basique de formule I sont les acides méthanesulfonique, benzène-sulfonique, maléique, fumarique, oxalique, citrique, lactique, tartrique et trifluoroacétique.

Parmi les composés selon la présente invention, on préfère ceux dans lesquels l'hétérocycle Het1 est un groupe 1-(1*H*)-imidazolyle. On préfère également les composés dans lesquels l'hétérocycle Het2 comprend un groupe 2(S)-pyrrolidinecarboxamide,

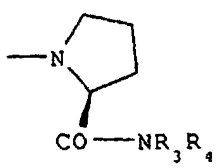 ou 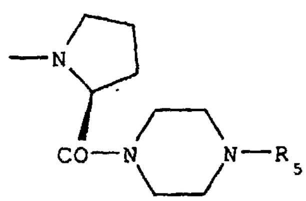

et plus particulièrement lorsque

$R_3$ représente un atome d'hydrogène ou un groupe alkyle en $C_1$-$C_3$, et
$R_4$ représente un groupe alkyle en $C_1$-$C_3$, un groupe $-(CH_2)_n-CH_2-NR_5R_6$, un groupe pyridinylméthyle ou un groupe

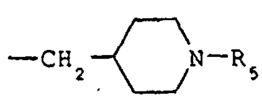 , et

$R_5$ représente un groupe alkyle en $C_1$-$C_3$, un groupe $-(CH_2)_m-CH_2OH$, un groupe (2-pyridinyl)méthyle ou un groupe 4-(aminoiminométhyl)benzoyle,
$R_6$ représente un groupe méthyle ou forme, avec $R_5$ et l'azote auquel ils sont liés, un hétérocycle saturé à 5 ou 6 sommets.

Par "température ambiante" on entend une température de l'ordre de 15 à 25°C, et par "température voisine de la température ambiante" une température d'environ 0 à 40°C.

Un procédé général de préparation des composés de formule I, que l'on préconise selon l'invention, comprend :

selon une première variante A, les étapes consistant à :

(1) faire réagir un dérivé de la 8-hydroxyquinoléine de formule II :

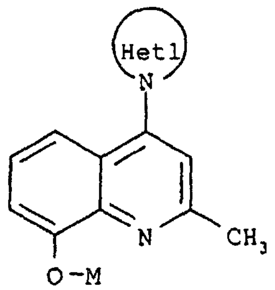

(II)

dans laquelle :

Het1 représente un hétérocycle azoté à cinq sommets comprenant au total 1, 2, 3 ou 4 atomes d'azote et M représente un métal alcalin, notamment le sodium ou le potassium,
avec un composé de formule III :

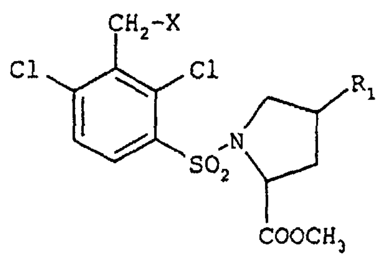

(III)

dans laquelle :

X représente un atome d'halogène, de préférence un atome de brome, et
$R_1$ représente un atome d'hydrogène ou un groupe OH, un groupe alcoxy ou un groupe phénoxy,
dans un solvant anhydre comme par exemple le diméthylformamide, à une température comprise entre 0 et 50°C, pendant 0,5 à 10 heures, pour obtenir un composé de formule IV :

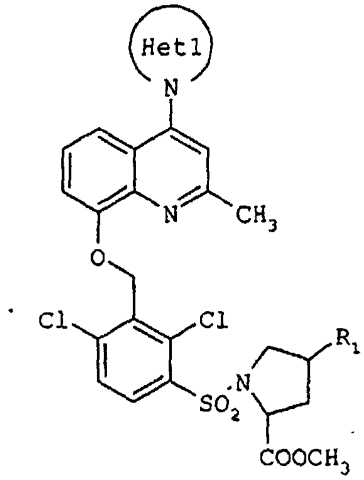

(IV)

dans laquelle :

Het1 et $R_1$ conservent la même signification que précédemment ;

(2) hydrolyser la fonction ester du composé de formule IV ainsi obtenu selon l'étape (1) ci-dessus, notamment par réaction avec une solution aqueuse d'hydroxyde de sodium, dans un solvant miscible tel que par exemple du dioxanne, à une température de l'ordre de 20 à 60°C et pendant 1 à 5 heures, pour obtenir après acidification un composé de formule V :

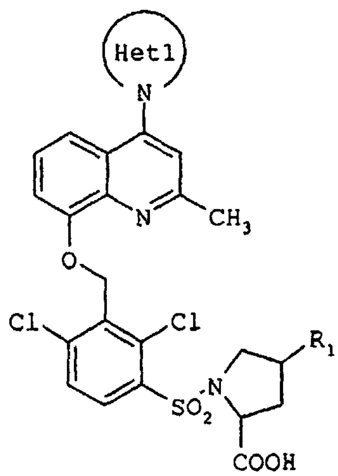

(V)

dans laquelle :

Het1 et $R_1$ conservent la même signification que ci-dessus ;

(3) faire réagir le composé de formule V, ainsi obtenu, avec une amine de formule :

$$HNR_3R_4 \quad (VI)$$

dans laquelle :

$R_3$ représente un atome d'hydrogène ou un groupe alkyle en $C_1$-$C_3$,
$R_4$ représente un atome d'hydrogène, un groupe alkyle en $C_1$-$C_3$, $-(CH_2)_n-CH_2OH$, $-(CH_2)_n-COOR_{11}$, $-(CH_2)_n-CH_2-NR_5R_6$,

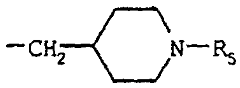 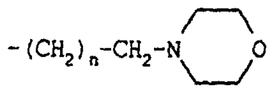 ou 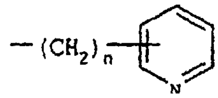

$R_5$ représente un groupe alkyle en $C_1$-$C_3$, $-(CH_2)_m-CH_2OH$, $-(CH_2)_m-COOR_{11}$, $-(CH_2)_m-CH_2-O-(CH_2)_m-CH_2OH$, ou un groupe aminoprotecteur tel que par exemple un groupe 1,1-diméthyléthoxycarbonyle (BOC), ($R_5$ et $R_6$ n'étant pas simultanément des groupes aminoprotecteurs),
$R_6$ représente un groupe alkyle en $C_1$-$C_3$ ou un groupe aminoprotecteur, par exemple du type BOC,
$R_{11}$ représente un groupe protecteur de la fonction acide facilement hydrolysable tel que par exemple le groupe *t*-butyle (ou 1,1-diméthyléthyle),

$$n = 1, 2, 3 \text{ ou } 4,$$

$$m = 1, 2 \text{ ou } 3,$$

dans un solvant approprié, notamment le dichlorométhane, en présence d'activateurs tels que notamment le 1-hydroxy-7-aza-benzotriazole (HOAT) et le chlorhydrate de 1-[3-(diméthylaminopropyl)-3-éthyl]carbodiimide (EDCI), à une température voisine de la température ambiante (0-40°C, de préférence 10-35°C), pendant 2 à 50 heures, pour obtenir un composé de formule :

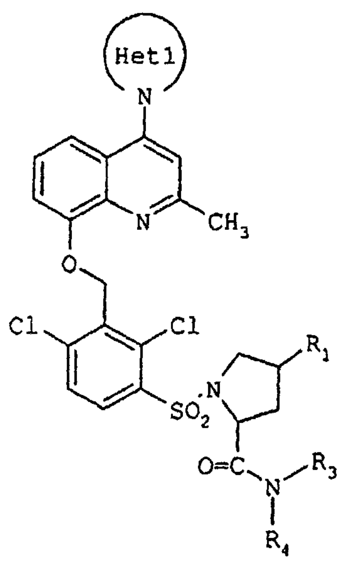

(VII)

dans laquelle :

Het1, $R_1$, $R_3$, $R_4$ conservent la même signification que précédemment ; et,
(4) si nécessaire, faire réagir le composé de formule VII, ainsi obtenu, pour éliminer les groupes amino- ou acidoprotecteurs de façon à remplacer ces groupes par un atome d'hydrogène, par exemple par réaction dudit composé VII avec l'acide trifluoroacétique pour éliminer un groupe amino protecteur du type BOC ou pour éliminer un groupe acidoprotecteur du type *t*-butyle, de façon à obtenir le composé de formule I':

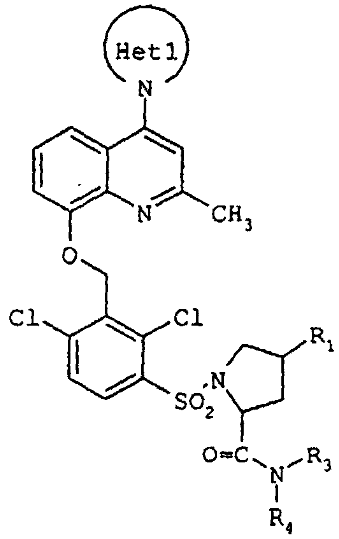

(I')

dans laquelle :

Het1, $R_1$, $R_3$ et $R_4$ conservent la même signification que ci-dessus, à l'exception des groupes protecteurs remplacés par des atomes d'hydrogène; puis,

(5) si nécessaire, faire réagir le composé de formule I,[1] ainsi obtenu, avec un acide pour obtenir le sel d'addition d'acide correspondant ; selon une seconde variante B consistant à :
(1) faire réagir un composé de formule $I^1$ tel qu'obtenu à l'étape (4) de la variante A ci-dessus,

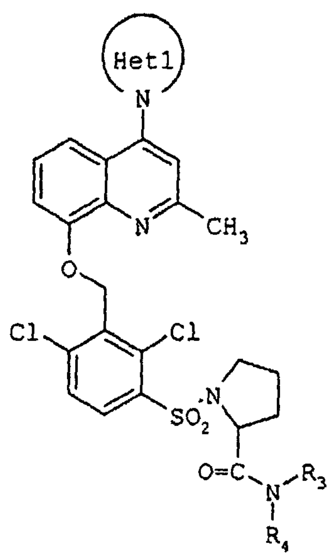

dans laquelle :

Het1 représente un groupe 1-imidazolyle, un groupe 1-pyrazolyle ou un groupe 1-(1,2,4-triazolyle),
$R_3$ représente H, ou un groupe alkyle en $C_1$-$C_3$,
$R_4$ représente un groupe porteur d'une fonction amine primaire ou secondaire choisi parmi : $-(CH_2)_n-CH_2-NHR_6$ ou

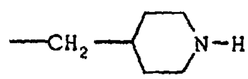

où $R_6$ représente H ou un groupe alkyle et n représente 1, 2, 3 ou 4, avec un composé halogéné de formule : $Y-(CH_2)_m-CH_2OR_{13}$, $Y-(CH_2)_m-COOR_{11}$, ou $Y-(CH_2)_m-CH_2-O-(CH_2)_m-CH_2OR_{13}$,
où

Y est un halogène, préférentiellement Br ou I,
m représente 1, 2, ou 3
$R_{11}$ est un groupe acidoprotecteur, tel que par exemple t-butyle, et
$R_{13}$ est un groupe protecteur de la fonction alcool, notamment le groupe acétyle,

dans un solvant tel que par exemple le diméthylformamide ou l'acétonitrile, en présence d'un agent à caractère alcalin comme par exemple le carbonate de potassium, à température voisine de la température ambiante et pendant 5 à 20 heures, pour obtenir le composé de formule VII :

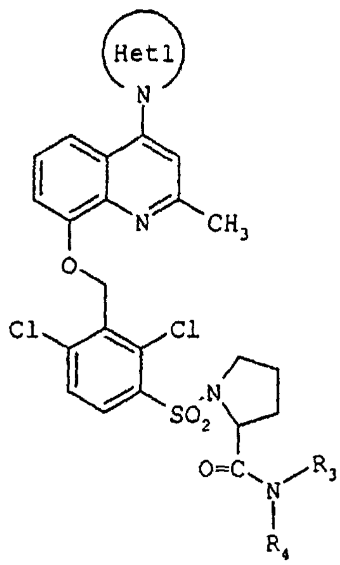

(VII)

dans laquelle :

$R_3$ représente H ou un groupe alkyle en $C_1$-$C_3$,
$R_4$ représente un groupe $-(CH_2)_n-CH_2-NR_5R_6$ ou

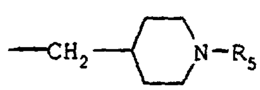

où

$R_5$ représente un groupe : $-(CH_2)_m-CH_2OR_{13}$, $-(CH_2)_m-COOR_{11}$, ou $-(CH_2)_m-CH_2-O-(CH_2)_m-CH_2OR_{13}$,
Het1, $R_6$, $R_1$ et $R_{13}$ conservant la même signification que ci-dessus ;

(2) effectuer une réaction de déprotection de chaque groupe alcool ou acide afin de remplacer les groupes $R_{13}$ et $R_{11}$ par un atome d'hydrogène, et ainsi obtenir les composés de formule I correspondants ;
(3) si nécessaire, faire réagir le composé de formule I ainsi obtenu avec un acide minéral ou organique pour obtenir le sel correspondant ;

<u>selon une troisième variante C, les étapes consistant à</u> :

(1) faire réagir le chlorure d'acide de formule VIII :

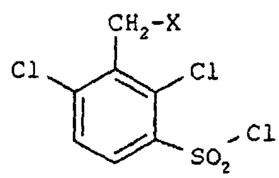

(VIII)

dans laquelle :

X représente un halogène, préférentiellement le brome, avec un dérivé hétérocyclique répondant à la formule :

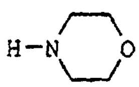
,
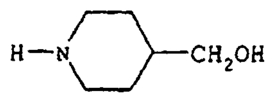
ou
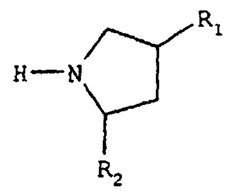

où :

$R_1$ représente H, OH, alcoxy, phénoxy, phénylméthoxy, $CH_2OH$, cycloalkyloxy en $C_3$-$C_8$ ou cycloalkylalcoxy où le fragment cycloalkyle est en $C_3$-$C_8$ et le fragment alcoxy en $C_1$-$C_4$,
$R_2$ représente H, ou un groupe $-CH_2OH$, $-CH_2OCH_3$, $-CONH(CH_2)_nCH_2NR_5R_{12}$, $-CONH(CH_2)_nCH_2OH$, $-CONH(CH_2)_nCOOR_{11}$ ou

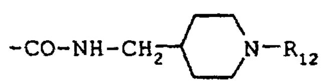
,

n = 1, 2, 3 ou 4,

$R_5$ représente H ou un groupe alkyle,
$R_{11}$ représente un groupe acidoprotecteur, et
$R_{12}$ représente un groupe aminoprotecteur,

dans un solvant comme par exemple l'acétonitrile, en présence d'une base comme par exemple le carbonate de potassium ou la triéthylamine, à une température proche de la température ambiante, pendant 10 à 30 heures, pour obtenir un composé de formule IX :

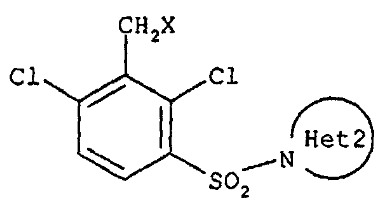

(IX)

dans laquelle :

Het2 représente un groupe

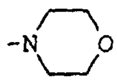 , 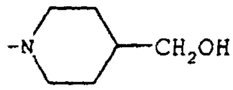 ou 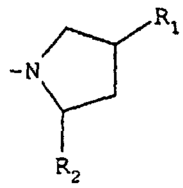 .

et X, $R_1$, $R_2$, $R_{11}$, $R_{12}$ et n conservent la même signification que ci-dessus ;

(2) faire réagir le composé de formule IX, ainsi obtenu, avec un dérivé de la 8-hydroxyquinoléine de formule II:

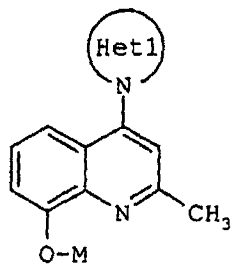

(II)

dans laquelle :

Het1 représente un hétérocycle azoté à cinq sommets comprenant 1, 2, 3 ou 4 atomes d'azote et M représente un métal alcalin, notamment le sodium ou le potassium,
dans des conditions analogues à celles mises en oeuvre à l'étape (1) de la variante A précédente, pour obtenir un composé de formule X :

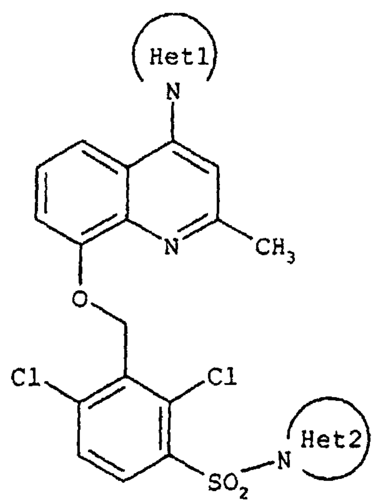

(X)

dans laquelle :

Het1 et Het2 conservent la même signification que ci-dessus ;

(3) si nécessaire, effectuer une réaction de déprotection, par exemple par action de l'acide trifluoroacétique, pour remplacer chaque groupe protecteur $R_{11}$ ou $R_{12}$ des fonctions acide ou amine par un atome d'hydrogène, pour obtenir un composé de formule I :

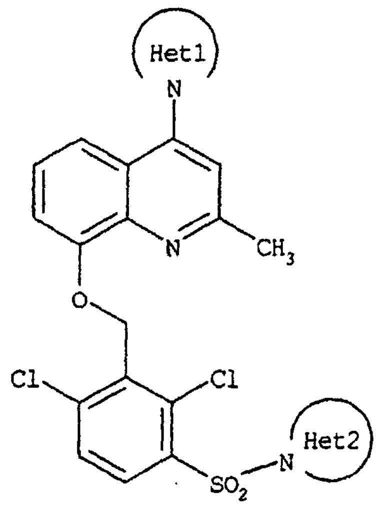

dans laquelle :

Het1 conserve la même signification que ci-dessus, et
Het2 représente un groupe

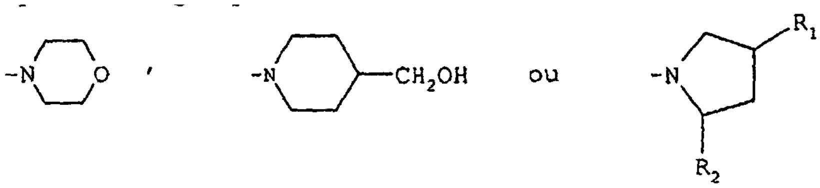

$R_1$ a la même signification que ci-dessus,
$R_2$ représente un groupe $-CH_2OH$, $-CH_2OCH_3$, $-CONH(CH_2)_nCH_2NHR_5$, $-CONH(CH_2)_nCH_2OH$, $-CONH(CH_2)_nCOOH$ ou

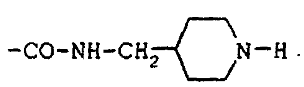

n = 1, 2, 3 ou 4, et

$R_5$ représente H ou un groupe alkyle ; et,

(4) si nécessaire, faire réagir le composé de formule I, ainsi obtenu, avec un acide pour obtenir le sel correspondant.

L'invention sera mieux comprise à la lecture qui va suivre (i) d'exemples de préparation et (ii) de résultats d'essais pharmacologiques réalisés avec des composés selon l'invention. Bien entendu l'ensemble de ces éléments n'est pas limitatif mais est donné à titre d'illustration.
Dans le cas de composés présentant dans leur structure un carbone asymétrique, l'absence d'indication

particulière ou la mention (R,S) signifie qu'il s'agit de composés racémiques ; dans le cas de composés présentant une chiralité, celle-ci est indiquée à la suite immédiate de l'indexation du substituant porté par ledit carbone asymétrique ; on utilise alors les signes (R) ou (S), conformément aux règles de Cahn, Ingold et Prelog. La nomenclature utilisée dans les exemples est celle préconisée par les Chemical Abstracts : ainsi certains dérivés de la L-proline peuvent devenir, après réaction de la fonction acide avec une amine, des dérivés de 2(S)-pyrrolidinecarboxamide.

**PREPARATION I**

**1-[[2,4-dichloro-3-[[[4-(1*H*-imidazol-1-yl)-2-méthyl-8-quinolinyl]oxy]méthyl]-phényl]sulfonyl]-L-proline, méthyl ester**

On prépare une solution de 0,7 g ($3{,}11.10^{-3}$ mole) de 8-hydroxy-4-[1H-imidazol-1-yl]-2-méthylquinoléine dans 20 ml de diméthylformamide (DMF) et on ajoute 0,11 g ($3{,}42.10^{-3}$ mole) d'hydrure de sodium à 75 % dans l'huile. Après 10 minutes sous agitation à température ambiante, on ajoute 1,47 g ($3{,}42.10^{-3}$ mole) de l'ester méthylique de la N-[3-(bromométhyl)-2,4-dichlorophénylsulfonyl]-L-proline. Après 15 heures sous agitation à température ambiante, le mélange réactionnel est hydrolysé sur de l'eau glacée et extrait par de l'acétate d'éthyle. La phase organique est lavée à l'eau, séchée sur sulfate de magnésium puis concentrée sous pression réduite. Le résidu obtenu est purifié par chromatographie sur gel de silice en éluant à l'aide d'un mélange toluène/propanol (95/5 ; v/v). On obtient ainsi 1,07 g du produit attendu sous forme d'un solide beige (Rendement = 68 %).
F = 100°C
$[\alpha]^{27}_D$ = - 14,4° (c = 0,33 ; $CH_3OH$)

**PREPARATION II**

**1-[[2,4-dichloro-3-[[[4-(1*H*-imidazol-1-yl)-2-méthyl-8-quinolinyl]oxy]méthyl]-phényl]sulfonyl]-L-proline**

On ajoute 1,6 ml ($1{,}6.10^{-3}$ mole) d'une solution normale d'hydroxyde de sodium dans l'eau à une solution de 0,44 g ($0{,}763.10^{-3}$ mole) du composé obtenu selon la préparation I dans 30 ml de dioxanne. Le mélange réactionnel est chauffé à doux reflux pendant 8 heures puis le solvant est chassé sous pression réduite. Le résidu est repris dans de l'eau et la solution est acidifiée doucement jusqu'à pH 4,5 à l'aide d'une solution d'acide chlorhydrique. L'acide attendu précipite. Le précipité est filtré, lavé à l'eau sur le filtre et séché à 40°C sous pression réduite. On obtient ainsi 0,36 g du produit attendu sous forme de poudre blanche (Rendement = 89 %).
F = 172°C

**Exemple 1**

**1-[[2,4-dichloro-3-[[[4-(1*H*-imidazol-1-yl)-2-méthyl-8-quinolyl]oxy]méthyl]-phényl]sulfonyl]-N-méthyl-2-(S)-pyrrolidinecarboxamide**

On prépare une solution de 0,35 g ($0{,}633.10^{-3}$ mole) d'acide obtenu selon la préparation II dans 25 ml de dichlorométhane et on ajoute 0,13 g ($0{,}686.10^{-3}$ mole) de chlorhydrate de 1-(3-diméthylaminopropyl)3-éthylcarbodiimide (EDCI), 0,1 g ($0{,}686.10^{-3}$ mole) de 1-hydroxy-7-azabenzotriazole (HOAT), 0,138 g ($1{,}37.10^{-3}$ mole) de triéthylamine, puis 0,05 g ($0{,}748.10^{-3}$ mole) de chlorhydrate de méthylamine. Le mélange réactionnel est agité à température ambiante pendant 20 heures. On hydrolyse ensuite sur de l'eau froide et extrait avec du dichlorométhane. La phase organique est séchée sur sulfate de magnésium puis concentrée sous pression réduite. Le résidu est purifié par chromatographie sur gel de silice en éluant à l'aide d'un mélange dichlorométhane/méthanol (98/2 ; v/v). On obtient ainsi 0,29 g du produit attendu sous forme d'un solide écru (Rendement = 81 %).
F = 90°C
$[\alpha]^{27}_D$ = - 28° (c = 0,46 ; $CH_3OH$)

**Exemple 2**

**1-[[2,4-dichloro-3-[[[4-(1*H*-imidazol-1-yl)-2-méthyl-8-quinolyl]oxy]méthyl]-phényl]sulfonyl]-N-méthyl-2-(S)-pyrrolidinecarboxamide, tartrate**

On prépare une solution de 0,28 g ($0{,}487.10^{-3}$ mole) du composé obtenu selon l'exemple 1 dans 3 ml de méthanol et on ajoute 0,073 g ($0{,}487.10^{-3}$ mole) d'acide L-tartrique. Le mélange réactionnel est maintenu sous agitation

à température ambiante pendant 10 minutes puis concentré sous pression réduite. Le résidu est repris en solution dans 10 ml d'eau distillée et la solution obtenue est lyophilisée. On obtient ainsi 0,34 g du sel attendu sous forme d'un solide blanc fin et léger (Rendement = 96 %).
F = 119°C
$[\alpha]^{27}_D$ = - 19° (c = 0,45 ; $CH_3OH$)

**PREPARATION III**

**N-(3-aminopropyl)-4-cyanobenzamide, trifluoroacétate**

On prépare une solution de 51 g (0,168 mole) d'acide [3-[(4-cyanobenzoyl)amino]propyl]carbamique, 1,1-diméthyléthylester dans 300 ml de dichlorométhane et on ajoute, à 0°C, 25 ml d'acide trifluoroacétique sous agitation. Le milieu réactionnel est ramené à température ambiante et maintenu 4 heures sous agitation. Le mélange est concentré sous pression réduite et le résidu est repris dans de l'éther éthylique. Le produit attendu cristallise. On le filtre, lave avec un peu d'éther éthylique sur le filtre et sèche sous pression réduite. On obtient ainsi 52 g du produit sous forme de cristaux blancs (Rendement = 97 %).
F=160°C

**PREPARATION IV**

**N-[3-[(4-cyanobenzoyl)amino]propyl]-1-[[2,4-dichloro-3-[[[4-(1*H*-imidazol-1-yl)-2-méthyl-8-quinolinyl]oxy]méthyl]phényl]sulfonyl]-2(S)-pyrrolidine carboxamide**

En opérant de façon analogue à l'exemple 1, au départ des composés obtenus selon les préparations II et III, on obtient le produit attendu sous forme d'un solide beige, (Rendement = 81 %).
F=118°C
$[\alpha]^{27}_D$ = - 33,2° (c = 0,32 ; $CH_3OH$)

**PREPARATION V**

**N-[3-[[4-[amino(hydroxyimino)méthyl]benzoyl]amino]propyl]-1-[[2,4-dichloro-3-[[[4-(1*H*-imidazol-1-yl)-2-méthyl-8-quinolinyl]oxy]méthyl]phényl]-sulfonyl]-2(S)-pyrrolidinecarboxamide**

On prépare une solution de 0,058 g ($0{,}843.10^{-3}$ mole) de chlorhydrate d'hydroxylamine dans 2 ml de DMSO et on ajoute 0,170 g ($1{,}69.10^{-3}$ mole) de triéthylamine, puis 0,36 g ($0{,}48.10^{-3}$ mole) du composé obtenu selon la préparation IV. Le mélange réactionnel est maintenu sous agitation, à température ambiante pendant 1 heure, puis on ajoute à nouveau une même quantité de chlorhydrate d'hydroxylamine et de triéthylamine. Après 15 heures sous agitation à température ambiante, le mélange réactionnel est versé sur de l'eau. Le précipité formé est séparé par filtration, puis lavé à l'eau et séché sous pression réduite à 30°C. On obtient ainsi 0,37 g du produit attendu sous forme d'une poudre blanche (Rendement = 98 %).
F = 160°C
$[\alpha]^{27}_D$ = - 22,5° (c = 0,35 ; $CH_3OH$)

**PREPARATION VI**

**N-[3-[[4-[[(acétyloxy)imino]aminométhyl]benzoyl]amino]propyl]-1-[[2,4-dichloro-3-[[[4-(1*H*-imidazol-1-yl)-2-méthyl-8-quinolinyl]oxy]méthyl]phényl]-sulfonyl]-2(S)-pyrrolidinecarboxamide**

On prépare une suspension de 0,32 g ($0{,}41.10^{-3}$ mole) du composé obtenu selon la préparation V, dans 10 ml de dichlorométhane et on ajoute 0,134 g ($1{,}23.10^{-3}$ mole) d'anhydride acétique. On agite le mélange pendant 3 heures à température ambiante puis on ajoute à nouveau 0,134 g d'anhydride acétique et on laisse sous agitation pendant 15 heures. Le milieu réactionnel est hydrolysé, extrait avec du dichlorométhane. La phase organique est lavée à l'eau puis séchée sur sulfate de magnésium et concentrée sous pression réduite. On obtient ainsi 0,32 g du produit attendu sous forme d'un solide blanc (Rendement = 95 %).
F = 96°C
$[\alpha]^{27}_D$ = - 20,3° (c = 0,32 ; $CH_3OH$)

**Exemple 3**

**N-[3-[[4-(aminoiminométhyl)benzoyl]amino]propyl]-1-[[2,4-dichloro-3-[[[4-(1*H*-imidazol-1-yl)-2-méthyl-8-quinolinyl]oxy]méthyl]phényl]sulfonyl]-2(S)-pyrrolidinecarboxamide**

On prépare une solution de 0,31 g ($0{,}377.10^{-3}$ mole) du composé obtenu selon la préparation VI, dans 20 ml de méthanol et on ajoute 0,12 g de catalyseur de Lindlar (à 5 % de Palladium). Le mélange est agité sous atmosphère d'hydrogène, à pression atmosphérique et à température ambiante pendant 6 heures. On élimine le catalyseur par filtration puis on concentre sous pression réduite. Le résidu est repris par de l'eau et la solution obtenue est amenée à pH légèrement alcalin à l'aide de soude IN. Le précipité blanc formé est filtré, lavé à l'eau puis séché sous pression réduite. On effectue ensuite une purification de ce produit par chromatographie sur gel de silice greffé $NH_2$ (Lichroprep® $NH_2$), en éluant à l'aide d'un mélange dichlorométhane/méthanol (98/2 ; v/v). On obtient ainsi 0,19 g du produit attendu sous forme d'un solide jaune (Rendement = 66 %).
F = 148°C
$[\alpha]^{27}_D$ = - 28,3° (c = 0,36 ; $CH_3OH$)

**Exemple 4**

**N-[3-[[4-(aminoiminométhyl)benzoyl]amino]propyl]-1-[[2,4 dichloro-3-[[[4-(1*H*-imidazol-1-yl)-2-méthyl-8-quinolinyl]oxy]méthyl]phényl]sulfonyl]-2(S)-pyrrolidinecarboxamide, bis méthanesulfonate**

On prépare une solution de 0,17 g ($0{,}22.10^{-3}$ mole) du composé obtenu selon l'exemple 3, dans 4 ml de méthanol et on ajoute 0,0428 g ($0{,}44.10^{-3}$ mole) d'acide méthanesulfonique. On agite le mélange réactionnel pendant 10 mn à température ambiante puis on le concentre sous pression réduite. Le résidu est repris en solution dans de l'eau distillée; la solution obtenue est filtrée puis lyophilisée. On obtient ainsi 0,16 g du produit attendu sous forme d'un solide cotonneux blanc (Rendement = 75 %).
F=176°C
$[\alpha]^{28}_D$ = - 28,3° (c = 0,32 ; $CH_3OH$)

**PREPARATION VII**

**1-[[3-(bromométhyl)-2,4-dichlorophényl]sulfonyl]-2(S)-pyrrolidineméthanol**

On prépare une solution de 1 g ($2{,}95.10^{-3}$ mole) de chlorure de 3-bromométhyl-2,4-dichlorobenzènesulfonyle dans 10 ml d'acétonitrile et 4 ml d'eau. On ajoute 292 µl ($2{,}95.10^{-3}$ mole) de L-(+)-prolinol et une solution de 886 mg de carbonate de potassium dans 4 ml d'eau. Après 20 heures sous agitation à température ambiante, le mélange réactionnel est versé sur de l'eau et extrait par l'acétate d'éthyle. La phase organique est lavée à l'eau, séchée sur sulfate de magnésium et concentrée sous pression réduite. On obtient 1,2 g de produit brut que l'on purifie par chromatographie sur gel de silice en éluant par un mélange toluène/acétate d'éthyle (80/20 ; v/v). On obtient ainsi 0,92 g du produit attendu sous forme d'une huile incolore (Rendement = 77 %).
$[\alpha]^{26}_D$ = - 16,5° (c = 0,5 ; $CH_3OH$)

**Exemple 5**

**1-[[2,4-dichloro-3-[[[4-(1*H*-imidazol-1-yl)-2-méthyl-8-quinolinyl]oxy]méthyl]-phényl]sulfonyl]-2(S)-pyrrolidineméthanol**

En opérant de façon analogue à la préparation I, au départ de 8-hydroxy-4-(1*H*-imidazol-1-yl)-2-méthylquinoléïne et du composé obtenu selon la préparation VII, on obtient le produit attendu sous forme de cristaux blancs (Rendement = 35 %).
F = 76°C
$[\alpha]^{26}_D$ = - 14,9° (c = 0,8 ; $CH_3OH$)

**Exemple 6**

**1-[[2,4-dichloro-3-[[[4-(1*H*-imidazol-1-yl)-2-méthyl-8-quinolinyl]oxy]méthyl]-phényl]sulfonyl]-2(S)-pyrrolidineméthanol, méthanesulfonate**

En opérant de façon analogue à l'exemple 4, au départ du composé obtenu selon l'exemple 5 et d'un équivalent molaire d'acide méthanesulfonique, on obtient le produit attendu sous forme de cristaux jaune pâle (Rendement = 90 %).
F = 134°C
$[\alpha]^{26}_{D}$ = + 3,1° (c = 0,84 ; $CH_3OH$)

**Exemple 7**

**1-[[2,4-dichloro-3-[[[2-méthyl-4-(1*H*-pyrazol-1-yl)-8-quinolinyl]oxy]méthyl]-phényl]sulfonyl]-2(S)-pyrrolidineméthanol**

En opérant de façon analogue à l'exemple 5, au départ de 8-hydroxy-2-méthyl-4-(1*H*-pyrazol-1-yl)quinoléine, on obtient le produit attendu sous forme de cristaux blancs (Rendement = 77%).
F = 65°C
$[\alpha]^{26}_{D}$ = -14,9° (c = 0,7 ; $CH_3OH$)

**Exemple 8**

**1-[[2,4-dichloro-3-[[[2-méthyl-4-(1*H*-pyrazol-1-yl)-8-quinolinyl]oxy]méthyl]-phényl]sulfonyl]-2(S)-pyrrolidineméthanol, méthanesulfonate**

En opérant de façon analogue à l'exemple 6, au départ du composé obtenu selon l'exemple 7, on obtient le sel attendu sous forme de cristaux jaunes (Rendement = 99 %).
F = 120°C
$[\alpha]^{26}_{D}$ = + 11,2° (c = 0,75 ; $CH_3OH$)

**PREPARATION VIII**

**Acide 4-[[[[1-[(phénylméthoxy)carbonyl]-2(S)-pyrrolidinyl]carbonyl]amino]-méthyl]-1-pipéridinecarboxylique, 1,1-diméthyléthyl ester**

En opérant de façon analogue à l'exemple 1, au départ de N-[(phényl-méthoxy)carbonyl]-L-proline et d'acide 4-(aminométhyl)-1-pipéridinecarboxylique, 1,1-diméthyléthyl ester, on obtient le produit attendu sous forme d'un solide blanc crème (Rendement = 99 %).
F = 50°C
$[\alpha]^{26}_{D}$ = -31° (c = 0,80 ; $CH_3OH$)

**PREPARATION IX**

**Acide 4-[[[(2(S)-pyrrolidinyl)carbonyl]amino]méthyl]-1-pipéridinecarboxylique, 1,1-diméthyléthyl ester (acétate)**

On prépare une solution de 100,9 g (0,23 mole) du composé obtenu selon la préparation VIII, dans l'acide acétique. Sous atmosphère d'azote, on ajoute 96,4 ml (1,02 mole) de cyclohexadiène, puis 2 g de charbon palladié à 10 %. Le mélange réactionnel est porté à reflux pendant 5 heures. Après refroidissement à 10-15°C, le milieu réactionnel est filtré et concentré sous pression réduite. Le résidu est purifié par chromatographie sur gel de silice en éluant avec un mélange dichlorométhane/éthanol (6/4 ; v/v). On obtient ainsi 60 g du produit attendu sous forme d'une huile orange (Rendement = 72 %, exprimé en sel avec l'acide acétique).
$[\alpha]^{22}_{D}$ = - 36,8° (c = 0,63 ; $CH_3OH$)

**PREPARATION X**

**Acide 4-[[[[1-[[3-(bromométhyl)-2,4-dichlorophényl]sulfonyl]-2(S)-pyrrolidinyl]carbonyl]amino]méthyl]-1-pipéridinecarboxylique,1,1-diméthyléthyl ester**

En opérant de façon analogue à la préparation VII, au départ du chlorure de 3-(bromométhyl)-2,4-dichlorobenzènesulfonyle et du composé obtenu selon la préparation IX, on obtient le produit attendu sous forme d'une poudre blanche (Rendement = 97 %).
F = 80°C
$[\alpha]^{22}_D$ = - 31° (c = 0,92 ; $CH_3OH$)

**PREPARATION XI**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridinecarboxylique, 1,1-diméthyléthyl ester**

En opérant de façon analogue à la préparation I, au départ du composé obtenu selon la préparation X, on obtient le produit attendu sous forme d'un solide blanc crème (Rendement = 44 %).
F = 100°C
$[\alpha]^{27}_D$ = - 28,8° (c = 0,36 ; $CH_3OH$)

**Exemple 9**

**1-[[2,4-dichloro-3-[[[4-(1*H*-imidazol-1-yl)-2-méthyl-8-quinolinyl]oxy]méthyl]-phényl]sulfonyl]-N-(4-pipéridinylméthyl)-2(S)-pyrrolidinecarboxamide**

On prépare une solution de 6 g ($7,92.10^{-3}$ mole) du composé obtenu selon la préparation XI dans 100 ml de dichlorométhane et on ajoute 0,856 g ($7,92.10^{-3}$ mole) d'anisole. On refroidit le mélange à 0°C et on ajoute 5 ml d'acide trifluoroacétique. La solution est ensuite agitée pendant 15 heures à température ambiante, puis concentrée sous pression réduite. Le résidu est repris avec de l'eau et la solution obtenue est amenée à pH basique avec une solution de soude normale. Le mélange est extrait avec de l'acétate d'éthyle, puis la phase organique est séchée sur sulfate de magnésium et concentrée. Le produit brut est purifié par chromatographie sur gel de silice en éluant à l'aide d'un mélange dichlorométhane/méthanol/ammoniaque (95/5/0,02 ; v/v/v). On obtient ainsi 4,4 g du produit attendu sous forme d'un solide jaune (Rendement = 84 %).
F = 150°C
$[\alpha]^{22}_D$ = - 47° (c = 0,35 ; $CH_3OH$)

**Exemple 10**

**1-[[2,4-dichloro-3-[[[4-(1*H*-imidazol-1-yl)-2-méthyl-8-quinolinyl]oxy]méthyl]-phényl]sulfonyl]-N-(4-pipéridinylméthyl)-2(S)-pyrrolidinecarboxamide, ditartrate**

En opérant de façon analogue à l'exemple 2, au départ du composé obtenu selon l'exemple 9 et en utilisant 2 moles d'acide tartrique pour une mole dudit composé, on obtient le produit attendu sous forme d'un solide cotonneux blanc (Rendement = 81 %).
F =145°C
$[\alpha]^{27}_D$ = - 23,7° (c = 0,31 ; $CH_3OH$)

**PREPARATION XII**

**Acide acétique, 2-(2-iodoéthoxy)éthyl ester**

On prépare une solution de 2,4 g ($14.10^{-3}$ mole) d'acétate de 2-(2-chloroéthoxy)éthyle dans 60 ml d'acétone et on ajoute 22 g (0,144 mole) d'iodure de sodium. On porte le mélange réactionnel à reflux pendant 6 heures, puis on le concentre sous pression réduite. Le résidu est repris par de l'eau et de l'acétate d'éthyle. La phase organique est séchée sur sulfate de magnésium et concentrée sous pression réduite. On obtient ainsi 2,81 g du produit attendu, qui est utilisé sans autre purification, sous forme d'une huile orangée (Rendement = 78 %).
$n_D$ = 1,468

## PREPARATION XIII

**1-[[2,4-dichloro-3-[[[4-(1*H*-imidazol-1-yl)-2-méthyl-8-quinolinyl]oxy]méthyl] phényl]sulfonyl]-N-[[1-[2-[2-(acétoxy)éthoxy]éthyl]-4-pipéridinyl]méthyl]-2(S)-pyrrolidinecarboxamide**

On prépare un mélange de 0,3 g ($0,456.10^{-3}$ mole) du composé obtenu selon l'exemple 9 dans 10 ml d'acétonitrile et 4 ml de diméthylformamide. On ajoute 95 mg ($0,68.10^{-3}$ mole) de carbonate de potassium puis 130 mg ($0,5.10^{-3}$ mole) du composé obtenu selon la préparation XII en solution dans 2 ml d'acétonitrile. Le mélange réactionnel est maintenu sous agitation à température ambiante pendant 15 heures puis concentré sous pression réduite. Le résidu est repris par du dichlorométhane et la phase organique ainsi obtenue est lavée à l'eau, séchée sur sulfate de magnésium et concentrée sous pression réduite. Le produit brut est purifié par chromatographie sur gel de silice en éluant à l'aide d'un mélange dichlorométhane/méthanol/ammoniaque (9/1/0,02; v/v/v). On obtient ainsi 0,18 g du produit attendu sous forme d'un solide écru (Rendement = 50 %).
F = 90°C
$[\alpha]^{25}_D$ = 35,8° (c = 0,31 ; $CH_3OH$)

## Exemple 11

**1-[[2,4-dichloro-3-[[[4-(1*H*-imidazol-1-yl)-2-méthyl-8-quinolinyl]oxy]méthyl]-phényl]sulfonyl]-N-[[1-[2-(2-hydroxyéthoxy)éthyl]-4-pipéridinyl]méthyl]-2(S)-pyrrolidinecarboxamide**

On prépare une solution de 0,17 g ($0,216.10^{-3}$ mole) du composé obtenu selon la préparation XIII dans 7 ml de méthanol et on ajoute 1 g de résine Amberlite® IRA 400 (sous forme $OH^-$). Le mélange réactionnel est agité à température ambiante pendant 15 heures, puis filtré de façon à éliminer la résine. Après concentration du filtrat sous pression réduite, on obtient 0,14 g du produit attendu sous forme d'un solide pulvérulent blanc (Rendement = 88 %).
F = 96°C
$[\alpha]^{25}_D$ = - 38,5° (c = 0,32 ; $CH_3OH$)

## Exemple 12

**1-[[2,4-dichloro-3-[[[4-(1*H*-imidazol-1-yl)-2-méthyl-8-quinolinyl]oxy]méthyl]-phényl]sulfonyl]-N-[[1-[2-(2-hydroxyéthoxy)éthyl]-4-pipéridinyl)méthyl]-2(S)-pyrrolidinecarboxamide, tartrate**

En opérant de façon analogue à l'exemple 2, au départ du composé obtenu selon l'exemple 11, on obtient le sel attendu sous forme d'un produit blanc cotonneux (Rendement = 99 %).
F = 135°C
$[\alpha]^{25}_D$ = - 38° (c = 0,43 ; $CH_3OH$)

## PREPARATION XIV

**N-[[1-[[2,4-dichloro-3-[[[4-(1*H*-imidazol-1-yl)-2-méthyl-8-quinolinyl]oxy]-méthyl]phényl]sulfonyl]-2(S)-pyrrolidinyl]carbonyl]glycine, 1,1-diméthyléthyl ester**

En opérant de façon analogue à l'exemple 1, au départ du composé obtenu selon la préparation II et de glycinate de 1,1-diméthyléthyle, on obtient le produit attendu sous forme d'un solide blanc (Rendement = 93 %).
F = 110°C
$[\alpha]^{22}_D$ = - 49° (c = 0,3 ; $CH_3OH$)

## Exemple 13

**N-[[1-[[2,4-dichloro-3-[[[4-(1*H*-imidazol-1-yl)-2-méthyl-8-quinolinyl]oxy]-méthyl]phényl]sulfonyl]-2(S)-pyrrolidinyl]carbonyl]glycine, trifluoroacétate**

On prépare un mélange de 0,27 g ($0,4.10^{-3}$ mole) du composé obtenu selon la préparation XIV dans 5 ml de dichiorométhane et 43 mg ($0,4.10^{-3}$ mole) d'anisole, puis on ajoute, à 0°C, 1,5 ml d'acide trifluoroacétique. La solution est agitée à 0°C pendant 1 heure puis à température ambiante pendant 24 heures. Le mélange réactionnel est ensuite concentré sous pression réduite et le résidu est trituré avec de l'éther éthylique. Le solvant est éliminé avec les produits solubles et le résidu et repris en solution dans de l'eau distillée. La solution est filtrée, puis lyophilisée. On obtient ainsi

0,285 g du produit attendu sous forme d'un solide fin jaunâtre (Rendement = 86 %).
F = 132°C
$[\alpha]^{22}_D$ = - 90° (c=0,64 ; $CH_3OH$)

**Exemple 14**

**1-[[2,4-dichloro-3-[[[4-(1*H*-imidazol-1-yl)-2-méthyl-8-quinolinyl]oxy]méthyl]-phényl]sulfonyl]-N-[2-(diméthylamino)éthyl]-2(S)-pyrrolidinecarboxamide**

En opérant de façon analogue à l'exemple 1, au départ du composé obtenu selon la préparation II et de N, N-diméthyléthylènediamine, on obtient le produit attendu sous forme d'une poudre beige (Rendement = 40 %).
F = 88°C
$[\alpha]^{23}_D$ = - 44° (c = 0,37 ; $CH_3OH$)

**Exemple 15**

**1-[[2,4-dichloro-3-[[[4-(1*H*-imidazol-1-yl)-2-méthyl-8-quinolinyl]oxy]méthyl]-phényl]sulfonyl]-N-[2-(diméthylamino)éthyl]-2(S)-pyrrolidinecarboxamide, tartrate**

En opérant de façon analogue à l'exemple 2, au départ du composé obtenu selon l'exemple 14, on obtient le produit attendu sous forme d'un solide cotonneux blanc cassé (Rendement de 93 %).
F=132°C
$[\alpha]^{24}_D$ = - 41° (c = 0,58 ; $CH_3OH$)

**Exemple 16**

**1-[[2,4-dichloro-3-[[[4-(1*H*-imidazol-1-yl)-2-méthyl-8-quinolinyl]oxy]méthyl]-phényl]sulfonyl]-N-[3-(diméthylamino)propyl]-2(S)-pyrrolidinecarboxamide**

En opérant de façon analogue à l'exemple 1, au départ du composé obtenu selon la préparation II et de N, N-diméthyl-1,3-propanediamine, on obtient le produit attendu sous forme d'un solide blanc (Rendement = 40 %).
F = 80°C
$[\alpha]^{24}_D$ = - 47° (c = 0,33 ; $CH_3OH$)

**Exemple 17**

**1-[[2,4-dichloro-3-[[[4-(1*H*-imidazol-1-yl)-2-méthyl-8-quinolinyl]oxy]méthyl]-phényl]sulfonyl]-N-[3-(diméthylamino)propyl]-2(S)-pyrrolidinecarboxamide, tartrate**

En opérant de façon analogue à l'exemple 2, au départ du composé obtenu selon l'exemple 16, on obtient le produit attendu sous forme d'un solide blanc (Rendement = 87 %).
F = 131°C
$[\alpha]^{24}_D$ = - 43° (c = 0,42 ; $CH_3OH$)

**Exemple 18**

**1-[[2,4-dichloro-3-[[[4-(1*H*-imidazol-1-yl)-2-méthyl-8-quinolinyl]oxy]méthyl]-phényl]sulfonyl]-N-[4-(diméthylamino)butyl]-2(S)-pyrrolidinecarboxamide**

En opérant de façon analogue à l'exemple 1, au départ du composé obtenu selon la préparation II et de N, N-diméthyl-1,4-butanediamine, on obtient le produit attendu sous forme d'un solide blanc (Rendement = 51 %).
F = 75°C
$[\alpha]^{22}_D$ = - 49° (c = 0,31 ; $CH_3OH$)

**Exemple 19**

**1-[[2,4-dichloro-3-[[[4-(1*H*-imidazol-1-yl)-2-méthyl-8-quinolinyl]oxy]méthyl]-phényl]sulfonyl]-N-[4-(diméthylamino)butyl]-2(S)-pyrrolidinecarboxamide, tartrate**

En opérant de façon analogue à l'exemple 2, au départ du composé obtenu selon l'exemple 18, on obtient le produit attendu sous forme d'un solide cotonneux blanc (Rendement = 90 %).
F = 125°C
$[\alpha]^{24}_D$ = - 33° (c = 0,35 ; $CH_3OH$)

**Exemple 20**

**1-[[2,4-dichloro-3-[[[4-(1*H*-imidazol-1-yl)-2-méthyl-8-quinolinyl]oxy]méthyl]-phényl]sulfonyl]-N-[(1-méthyl-4-pipéridinyl)méthyl]-2(S)-pyrrolidine-carboxamide**

On prépare un mélange de 0,6 g ($0,677.10^{-3}$ mole) du composé obtenu selon l'exemple 9 dans 20 ml de dichlorométhane et on ajoute 0,15 g ($1,49.10^{-3}$ mole) de triéthylamine, puis 0,08 g de paraformaldéhyde et 0,37 g ($27,1.10^{-3}$ mole) de chlorure de zinc. Le mélange réactionnel est maintenu sous agitation pendant 1 heure, puis on ajoute 0,1 g ($2,64.10^{-3}$ mole) de borohydrure de sodium et 2 ml de méthanol. On continue l'agitation à température ambiante pendant 15 heures puis on concentre le mélange sous pression réduite. Le résidu est repris avec de l'eau et la phase aqueuse ainsi obtenue, amenée à pH alcalin à l'aide d'une solution d'ammoniaque, est extraite par de l'acétate d'éthyle. La phase organique est séchée puis concentrée sous pression réduite. Le résidu obtenu est purifié par chromatographie sur gel de silice en éluant par un mélange dichlorométhane/méthanol/ammoniaque (95/5/0,2 ; v/v/v). On obtient ainsi 0,25 g du produit attendu sous forme d'une poudre blanche (Rendement = 55 %).
F = 86°C
$[\alpha]^{27}_D$ = - 36° (c = 0,33 ; $CH_3OH$)

**Exemple 21**

**1-[[2,4-dichloro-3-[[[4-(1*H*-imidazol-1-yl)-2-méthyl-8-quinolinyl]oxy]méthyl]-phényl]sulfonyl]-N-[(1-méthyl-4-pipéridinyl)méthyl]-2(S)-pyrrolidine carboxamide, tartrate**

En opérant de façon analogue à l'exemple 2, au départ du composé obtenu selon l'exemple 20, on obtient le produit attendu sous forme d'un solide blanc (Rendement = 97 %).
F=135°C
$[\alpha]^{27}_D$ = - 34,3° (c = 0,58 ; $CH_3OH$)

**PREPARATION XV**

**1-[[2,4-dichloro-3-[[[4-(1*H*-imidazol-1-yl)-2-méthyl-8-quinolinyl]oxy]méthyl]-phényl]sulfonyl]-N-[[1-[3-(acétoxy)propyl]-4-pipéridinyl]méthyl]-2(S)-pyrrolidinecarboxamide**

En opérant de façon analogue à la préparation XIII, au départ du composé obtenu selon l'exemple 9 et d'acétate de 3-iodopropyle, on obtient le produit attendu (Rendement = 52 %).
F = 96°C
$[\alpha]^{27}_D$ = - 32,2° (c = 0,30 ; $CH_3OH$)

**Exemple 22**

**1-[[2,4-dichloro-3-[[[4-(1*H*-imidazol-1-yl)-2-méthyl-8-quinolinyl]oxy]méthyl]-phényl]sulfonyl]-N-[[1-(3-hydroxypropyl)-4-pipéridinyl]méthyl]-2(S)-pyrrolidinecarboxamide**

On prépare une solution de 0,13 g ($0,167.10^{-3}$ mole) du composé obtenu selon la préparation XV dans 5 ml de méthanol. On ajoute 1 ml d'eau et 50 mg ($0,385.10^{-3}$ mole) de carbonate de potassium. Le mélange est agité pendant 15 heures à température ambiante puis concentré sous pression réduite. Le résidu est repris dans du dichlorométhane et la phase organique est lavée à l'eau puis séchée sur sulfate de magnésium et concentrée sous pression réduite. Le produit brut est purifié par chromatographie sur gel de silice en éluant à l'aide d'un mélange dichlorométhane/méthanol (98/2 ;v/v). On obtient ainsi 0,09 g du produit attendu sous forme d'un fin solide blanc (Rendement = 74 %).

F = 90°C
$[\alpha]^{27}_D$ = - 35,5° (c = 0,35 ; $CH_3OH$)

**Exemple 23**

**1-[[2,4-dichloro-3-[[[4-(1*H*-imidazol-1-yl)-2-méthyl-8-quinolinyl]oxy]méthyl]-phényl]sulfonyl]-N-[[1-(3-hydroxypropyl)-4-pipéridinyl]méthyl]-2(S)-pyrrolidinecarboxamide, tartrate**

En opérant de façon analogue à l'exemple 2, au départ du composé obtenu selon l'exemple 22, on obtient le produit attendu sous forme de poudre blanche (Rendement = 95 %).
F = 145°C
$[\alpha]^{26}_D$ = - 10,4° (c = 0,32 ; $CH_3OH$)

**PREPARATION XVI**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridineacétique, 1,1-diméthyléthyl ester**

On prépare un mélange de 0,45 g ($0,684.10^{-3}$ mole) du composé obtenu selon l'exemple 9 dans 4 ml de diméthylfonnamide et 30 ml d'acétonitrile. On ajoute 0,11 g ($0,752.10^{-3}$ mole) de carbonate de potassium, puis 0,133 g ($0,684.10^{-3}$ mole) de bromoacétate de *t*-butyle. Le mélange réactionnel est maintenu sous agitation à température ambiante pendant 15 heures puis concentré sous pression réduite. Le résidu est repris dans de l'eau et le précipité formé est extrait par de l'acétate d'éthyle. La phase organique est lavée à l'eau puis séchée et concentrée sous pression réduite. Le produit brut est purifié par chromatographie sur gel de silice en éluant à l'aide d'un mélange dichlorométhane/méthanol (98/2 ;v/v). On obtient ainsi 0,31 g du produit attendu sous forme d'un solide blanc écru (Rendement = 60 %).
F = 100°C
$[\alpha]^{23}_D$ = - 40° (c = 0,30 ; $CH_3OH$)

**Exemple 24**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridineacétique, bis trifluoroacétate**

En opérant de façon analogue à l'exemple 13, au départ du composé obtenu selon la préparation XVI, on obtient le produit attendu sous forme d'un solide léger jaune (Rendement = 90 %).
F = 149°C
$[\alpha]^{21}_D$ = - 41° (c = 0,40 ; $CH_3OH$)

**PREPARATION XVII**

**1-[[2,4-dichloro-3-[[[2-méthyl-4-(1*H*-pyrazol-1-yl)--8-quinolinyl]oxy]méthyl]-phényl]sulfonyl]-L-proline, méthyl ester**

En opérant de façon analogue à la préparation I, au départ de 8-hydroxy-4-(1*H*-pyrazol-1-yl)-2-méthyl-quinoléine, on obtient le produit attendu sous forme d'un solide blanc (Rendement = 96 %).
F = 90°C
$[\alpha]^{25}_D$ = - 19° (c = 0,50 ; $CHCl_3$)

**PREPARATION XVIII**

**1-[[2,4-dichloro-3-[[[2-méthyl-4-(1*H*-pyrazol-1-yl)-8-quinolinyl]oxy]méthyl]-phényl]sulfonyl]-L-proline**

En opérant de façon analogue à la préparation II, au départ du composé obtenu selon la préparation XVII, on obtient le produit attendu sous forme d'une poudre blanc écru (Rendement = 91 %).
F = 148°C
$[\alpha]^{27}_D$ = + 1° (c = 0,40 ; DMSO)

**PREPARATION XIX**

**Acide 4-[[[[1-[[2,4-dichloro-3-[[[2-méthyl-4-(1*H*-pyrazol-1-yl)-8-quinolinyl]-oxy]méthyl]phényl]sulfonyl]-2(S)-pyrrolidinyl]carbonyl]amino]méthyl]-1-pipéridinecarboxylique, 1,1-diméthyl-éthyl ester**

En opérant de façon analogue à l'exemple 1, au départ du composé obtenu selon la préparation XVIII et de l'ester *t*-butylique de l'acide 4-(aminométhyl)-1-pipéridinecarboxylique, on obtient le produit attendu sous forme d'un solide blanc (Rendement = 52 %).
F = 109°C
$[\alpha]^{25}_D$ = - 45° (c = 0,44 ; $CHCl_3$)

**Exemple 25**

**1-[[2,4-dichloro-3-[[[2-méthyl-4-(1*H*-pyrazol-1-yl)-8-quinolinyl]oxy]méthyl]-phényl]sulfonyl]-N-(4-pipéridinylméthyl)-2(S)-pyrrolidinecarboxamide, bis trifluoroacétate**

En opérant de façon analogue à l'exemple 13, au départ du composé obtenu selon la préparation XIX, on obtient le produit attendu sous forme d'un solide blanc (Rendement = 89 %).
F = 125°C
$[\alpha]^{26}_D$ = - 27° (c = 0,30 ; $CH_3OH$)

**PREPARATION XX**

**Acide 4-[[[[1-[[2,4-dichloro-3-[[[2-méthyl-4-(1*H*-1,2,4-triazol-1-yl)-8-quinolinyl]oxy]méthyl]phényl]sulfonyl]-2(S)-pyrrolidinyl]carbonyl]amino]méthyl]-1-pipéridinecarboxylique, 1,1-diméthyl-éthyl ester**

En opérant de façon analogue à la préparation XI, au départ du composé obtenu selon la préparation X et de 8-hydroxy-2-méthyl-4-(IH-1,2,4-triazol-1-yl)-quinoléine, on obtient le produit attendu sous forme d'une poudre beige (Rendement = 93 %).
F = 94°C
$[\alpha]^{24}_D$ = - 44° (c = 0,57 ; $CHCl_3$)

**Exemple 26**

**1-[[2,4-dichloro-3-[[[2-méthyl-4-(1*H*-1,2,4-triazol-1-yl)-8-quinolinyl]oxy]-méthyl]phényl]sulfonyl]-N-(4-pipéridinylméthyl)-2(S)-pyrrolidinecarboxamide, bis trifluoroacétate**

En opérant de façon analogue à l'exemple 25, au départ du composé obtenu selon la préparation XX, on obtient le produit attendu sous forme d'un solide blanc-crème (Rendement = 89 %).
F = 130°C
$[\alpha]^{18}_D$ = - 28° (c = 0,63 ; $CH_3OH$)

**PREPARATION XXI**

**1-[[2,4-dichloro-3-[[[2-méthyl-4-(1*H*-pyrazol-1-yl)-8-quinolinyl]oxy]méthyl]-phényl]sulfonyl]-N-[[1-[3-(acétoxy)propyl]-4-pipéridinyl]méthyl]-2(S)-pyrrolidinecarboxamide**

En opérant de façon analogue à la préparation XV, au départ du composé obtenu selon l'exemple 25 et d'acétate de 3-iodopropyle, on obtient le produit attendu sous forme d'un solide blanc (Rendement = 64 %).
F = 89°C
$[\alpha]^{24}_D$ = - 41°(c = 0,60 ; $CHCl_3$)

**Exemple 27**

**1-[[2,4-dichloro-3-[[[2-méthyl-4-(1*H*-pyrazol-1-yl)-8-quinolinyl]oxy]méthyl]-phényl]sulfonyl]-N-[[1-(3-hydroxypropyl)-4-pipéridinyl]méthyl]-2(S)-pyrrolidinecarboxamide**

En opérant de façon analogue à l'exemple 22, au départ du composé obtenu selon la préparation XXI, on

obtient le produit attendu sous forme de poudre fine blanche (Rendement = 95 %).
F = 112°C
$[\alpha]^{24}_D$ = - 42° (c = 0,38 ; $CHCl_3$)

**Exemple 28**

**1-[[2,4-dichloro-3-[[[2-méthyl-4-(1*H*-pyrazol-1-yl)-8-quinolinyl]oxy]méthyl]-phényl]sulfonyl]-N-[[1-(3-hydroxypropyl)-4-pipéridinyl]méthyl]-2(S)-pyrrolidinecarboxamide, tartrate**

En opérant de façon analogue à l'exemple 2, au départ du composé obtenu selon l'exemple 27, on obtient le produit attendu sous forme d'un solide blanc (Rendement = 99 %).
F=136°C
$[\alpha]^{22}_D$ = - 39° (c = 0,50 ; $CH_3OH$)

**PREPARATION XXII**

**1-[[2,4-dichloro-3-[[[2-méthyl-4-(1*H*-1,2,4-triazol-1-yl)-8-quinolinyl]oxy]-méthyl]phényl]sulfonyl]-N-[[1-[3-(acétoxy)propyl]-4-pipéridinyl]méthyl]-2(S)-pyrrolidinecarboxamide**

En opérant de façon analogue à la préparation XV, au départ du composé obtenu selon l'exemple 26 et d'acétate de 3-iodopropyle, on obtient le produit attendu sous forme d'un solide blanc crème (Rendement = 47 %).
F = 120°C
$[\alpha]^{26}_D$ = - 61° (c = 0,4 ; $CHCl_3$)

**Exemple 29**

**1-[[2,4-dichloro-3-[[[2-méthyl-4-(1*H*-1,2,4-triazol-1-yl)-8-quinolinyl]oxy]-méthyl]phényl]sulfonyl]-N-[[1-(3-hydroxypropyl)-4-pipéridinyl]méthyl]-2(S)-pyrrolidinecarboxamide**

En opérant de façon analogue à l'exemple 22, au départ du composé obtenu selon la préparation XXII, on obtient le produit attendu sous forme d'une poudre blanche (Rendement = 83 %).
F=136°C
$[\alpha]^{22}_D$ = - 48° (c = 0;55 ; $CHCl_3$)

**Exemple 30**

**1-[[2,4-dichloro-3-[[[2-méthyl-4-(1*H*-1,2,4-triazol-1-yl)-8-quinolinyl]oxy]-méthyl]phényl]sulfonyl]-N-[[1-(3-hydroxypropyl)-4-pipéridinyl]méthyl]-2(S)-pyrrolidinecarboxamide, tartrate**

En opérant de façon analogue à l'exemple 2, au départ du composé obtenu selon l'exemple 29, on obtient le produit attendu sous forme d'une poudre blanche (Rendement = 98 %).
F = 123°C
$[\alpha]^{23}_D$ = - 43° (c = 0;48 ; $CH_3OH$)

**PREPARATION XXIII**

**1-[[2,4-dichloro-3-[[[4-(1*H*-imidazol-1-yl)-2-méthyl-8-quinolinyl]oxy]méthyl]-phényl]sulfonyl]-N-[[1-[4-(acétoxy)butyl]-4-pipéridinyl]méthyl]-2(S)-pyrrolidinecarboxamide**

En opérant de façon analogue à la préparation XV, au départ du composé obtenu selon l'exemple 9 et d'acétate de 4-iodobutyle, on obtient le produit attendu (Rendement = 63 %).
F = 86°C
$[\alpha]^{22}_D$ = - 44° (c = 0,86 ; $CHCl_3$)

**Exemple 31**

**1-[[2,4-dichloro-3-[[[4-(1*H*-imidazol-1-yl)-2-méthyl-8-quinolinyl]oxy]méthyl]-phényl]sulfonyl]-N-[[1-(4-hydroxybutyl)-4-pipéridinyl]méthyl]-2(S)-pyrrolidinecarboxamide**

En opérant de façon analogue à l'exemple 11, au départ du composé obtenu selon la préparation XXIII, on obtient le produit attendu sous forme d'un solide blanc (Rendement = 96 %).
F = 112°C
$[\alpha]^{22}_D$ = - 49° (c = 0,78 ; $CHCl_3$)

**Exemple 32**

**1-[[2,4-dichloro-3-[[[4-(1*H*-imidazol-1-yl)-2-méthyl-8-quinolinyl]oxy]méthyl]-phényl]sulfonyl]-N-[[1-(4-hydroxybutyl)-4-pipéridinyl]méthyl]-2(S)-pyrrolidinecarboxamide, tartrate**

En opérant de façon analogue à l'exemple 2, au départ du composé obtenu selon l'exemple 31, on obtient le produit attendu sous forme d'un solide blanc (Rendement = 96 %).
F = 133°C
$[\alpha]^{22}_D$ = - 41° (c = 0,82 ; $CH_3OH$)

**PREPARATION XXIV**

**4-[(3-bromométhyl-2,4-dichlorophényl)sulfonyl]-morpholine**

En opérant de façon analogue à la préparation VII, au départ de morpholine et du chlorure de 3-bromométhyl-2,4-dichlorobenzènesulfonyle, on obtient le produit attendu sous forme d'un solide jaune (Rendement = 85 %) (le produit contient en partie l'analogue chlorométhylé).
F = 128°C

**Exemple 33**

**8-[[2,6-dichloro-3-(4-morpholinylsulfonyl)phényl]méthoxy]-4-(1*H*-imidazol-1-yl)-2-méthylquinoléine**

En opérant de façon analogue à la préparation I, au départ du produit obtenu selon la préparation XXIV et de 8-hydroxy-4-(1*H*-imidazol-1-yl)-2-méthylquinoléine, on obtient le produit attendu sous forme d'un solide beige (Rendement = 34 %).
F = 148°C

**Exemple 34**

**8-[[2,6-dichloro-3-(4-morpholinylsulfonyl)phényl]méthoxy]-4-(1*H*-imidazol-1-yl)-2-méthylquinoléine, trifluoroacétate**

En opérant de façon analogue à l'exemple 1, au départ d'acide trifluoroacétique et du composé obtenu selon l'exemple 33, on obtient le produit attendu sous forme d'une poudre légère couleur crème (Rendement = 98 %).
F=93°C

**Exemple 35**

**8-[[2,6-dichloro-3-(4-morpholinylsulfonyl)phényl]méthoxy]-2-méthyl-4-(1*H*pyrazol-1-yl)quinoléine**

En opérant de façon analogue à l'exemple 33, au départ de 8-hydroxy-4-(1*H*-pyrazol-1-yl)-2-méthylquinoléïne, on obtient le produit attendu sous forme d'un solide blanc cassé (Rendement = 83 %).
F=178°C

**Exemple 36**

**8-[[2,6-dichloro-3-(4-morpholinylsulfonyl)phényl]méthoxy]-2-méthyl-4-(1*H*pyrazol-1-yl)quinoléine, trifluoroacétate**

En opérant de façon analogue à l'exemple 34, au départ de l'exemple 35, on obtient le produit attendu sous forme d'un solide blanc (Rendement = 89 %).
F = 110°C

**PREPARATION XXV**

**1-[(3-bromométhyl-2,4-dichlorophényl)sulfonyl]-4-(hydroxyméthyl)-pipéridine**

En opérant de façon analogue à la préparation XXIV, au départ de 4-(hydroxyméthyl)pipéridine, on obtient le produit attendu sous forme d'une poudre blanche (Rendement = 88 %).
F=121°C

**Exemple 37**

**1-[[2,4-dichloro-3-[[[4-(1*H*-imidazol-1-yl)-2-méthyl-8-quinolinyl]oxy]méthyl]-phényl]sulfonyl]-4-pipéridineméthanol**

En opérant de façon analogue à l'exemple 33, au départ du produit obtenu selon la préparation XXV et 8-hydroxy-4-(1*H*-imidazol-1-yl)-2-méthylquinoléïne, on obtient le produit attendu sous forme d'un solide blanc (Rendement = 40 %).
F = 100°C

**Exemple 38**

**1-[[2,4-dichloro-3-[[[4-(1*H*-imidazol-1-yl)-2-méthyl-8-quinolinyl]oxy]méthyl] phényl]sulfonyl]-4-pipéridineméthanol, trifluoroacétate**

En opérant de façon analogue à l'exemple 34, au départ du produit obtenu selon l'exemple 37, on obtient le produit attendu sous forme d'une poudre blanche (Rendement = 98 %).
F = 109°C

**Exemple 39**

**1-[[2,4-dichloro-3-[[[2-méthyl-4-(1*H*-pyrazol-1-yl)-8-quinolinyl]oxy]méthyl]-phényl]sulfonyl]-4-pipéridineméthanol**

En opérant de façon analogue à l'exemple 37, au départ du produit obtenu selon la préparation XXV et 8-hydroxy-2-méthyl-4-(1*H*-pyrazol-1-yl)-quinoléine, on obtient le produit attendu sous forme d'un solide beige (Rendement = 74 %).
F = 138°C

**Exemple 40**

**1-[[2,4-dichloro-3-[[[2-méthyl-4-(1*H*-pyrazol-1-yl)-8-quinolinyl]oxy]méthyl]-phényl]sulfonyl]-4-pipéridineméthanol, trifluoroacétate**

En opérant de façon analogue à l'exemple 34, au départ du produit obtenu selon l'exemple 39, on obtient le produit attendu sous forme d'une poudre blanc crème (Rendement = 98 %).
F=90°C

**PREPARATION XXVI**

**1-[[2,4-dichloro-3-[[[4-(1*H*-imidazol-1-yl)-2-méthyl-8-quinolinyl]oxy]méthyl]-phényl]sulfonyl]-4(R)-hydroxy-L-proline, méthyl ester**

En opérant de façon analogue à la préparation I, au départ de l'ester méthylique de la N-[(3-bromométhyl-2,4-dichlorophényl)sulfonyl]-4-(R)-hydroxy(L)-proline, on obtient le produit attendu sous forme d'un solide blanc écru (Rendement = 69 %).
F = 78°C
$[\alpha]^{22}_D$ = + 1° (c = 0,66 ;$CH_3OH$)

**PREPARATION XXVII**

**1-[[2,4-dichloro-3-[[[4-(1*H*-imidazol-1-yl)-2-méthyl-8-quinolinyl]oxy]méthyl]-phényl]sulfonyl]-4(R)-hydroxy-L-proline**

En opérant de façon analogue à la préparation II, au départ du produit obtenu selon la préparation XXVI, on obtient le produit attendu sous forme d'un solide blanc (Rendement = 51 %).
F = 140°C
$[\alpha]^{27}_D$ = + 13° (c = 0,38 ;$CH_3OH$)

**PREPARATION XXVIII**

**Acide 4-[[[[1-[[2,4-dichloro-3-[[[4-(1*H*-imidazol-1-yl)-2-méthyl-8-quinolinyl]-oxy]méthyl]phényl]sulfonyl]-4(R)-hydroxy-2(S)-pyrrolidinyl]carbonyl]-amino]méthyl]-1-pipéridinecarboxylique, 1,1-diméthyléthyl ester**

En opérant de façon analogue à l'exemple 1, au départ de l'acide obtenu selon la préparation XXVII et de l'ester *t*-butylique de l'acide 4-(aminométhyl)pipéridine carboxylique, on obtient le produit attendu sous forme d'un solide blanc (Rendement = 65 %).
F = 124°C
$[\alpha]^{24}_D$ = - 6° (c = 0,51 ; $CH_3OH$)

**Exemple 41**

**1-[[2,4-dichloro-3-[[[4-(1*H*-imidazol-1-yl)-2-méthyl-8-quinolinyl]oxy]méthyl]-phényl]sulfonyl]-4(R)-hydroxy-N-(4-pipéridineméthyl)-2(S)-pyrrolidine-carboxamide**

En opérant de façon analogue à l'exemple 9, au départ du composé obtenu selon la préparation XXVIII, on obtient le produit attendu sous forme d'un solide jaunâtre (Rendement = 80 %).
F=140°C
$[\alpha]^{27}_D$ = - 3° (c = 0,33 ; $CH_3OH$)

**Exemple 42**

**1-[[2,4-dichloro-3-[[[4-(1*H*-imidazol-1-yl)-2-méthyl-8-quinolinyl]oxy]méthyl]-phényl]sulfonyl]-4(R)-hydroxy-N-(4-pipéridineméthyl)-2(S)-pyrrolidine-carboxamide, ditartrate**

En opérant de façon analogue à l'exemple 2, au départ du composé obtenu selon l'exemple 41 et de deux équivalents molaires d'acide tartrique, on obtient le produit attendu sous forme d'un solide léger jaune (Rendement = 50 %).
F=156°C
$[\alpha]^{27}_D$ = + 30° (c = 0,38 ; DMSO)

**Exemple 43**

**1-[[2,4-dichloro-3-[[[4-(1*H*-imidazol-1-yl)-2-méthyl-8-quinolinyl]oxy]méthyl]-phényl]sulfonyl]-N-(2-hydroxyéthyl)-2(S)-pyrrolidinecarboxamide**

En opérant de façon analogue à l'exemple 1, au départ d'acide obtenu selon la préparation II et de 2-aminoéthanol, on obtient le produit attendu sous forme d'un solide blanc (Rendement = 93 %).
F = 120°C
$[\alpha]^{27}_D$ = - 31° (c = 0,37 ; $CH_3OH$)

**Exemple 44**

**1-[[2,4-dichloro-3-[[[4-(1*H*-imidazol-1-yl)-2-méthyl-8-quinolinyl]oxy]méthyl]-phényl]sulfonyl]-N-(2-hydroxyéthyl)-2(S)-pyrrolidinecarboxamide, hémisulfate**

En opérant de façon analogue à l'exemple 2, au départ du composé obtenu selon l'exemple 43 et d'un demi équivalent molaire d'acide sulfurique, on obtient le produit attendu sous forme d'une poudre blanche (Rendement = 98 %).
F = 140°C
$[\alpha]^{27}_D$ = - 24° (c = 0,35 ; $CH_3OH$)

**Exemple 45**

**1-[[2,4-dichloro-3-[[[4-(1*H*-imidazol-1-yl)-2-méthyl-8-quinolinyl]oxy]méthyl]-phényl]sulfonyl]-N,N-diméthyl-2(S)-pyrrolidinecarboxamide**

En opérant de façon analogue à l'exemple 1, au départ de l'acide obtenu selon la préparation II et de diméthylamine en solution dans l'éthanol, on obtient le produit attendu sous forme d'un solide blanc (Rendement = 69 %).
F = 88°C
$[\alpha]^{27}_D$ = - 8° (c = 0,32 ; $CH_3OH$)

**Exemple 46**

**1-[[2,4-dichloro-3-[[[4-(1*H*-imidazol-1-yl)-2-méthyl-8-quinolinyl]oxy]méthyl]-phényl]sulfonyl]-N,N-diméthyl-2(S)-pyrrolidinecarboxamide, tartrate**

En opérant de façon analogue à l'exemple 2, au départ du composé obtenu selon l'exemple 45, on obtient le produit attendu sous forme d'une poudre fine jaune (Rendement = 80 %).
F = 121°C
$[\alpha]^{24}_D$ = + 34° (c = 0,37 ; $CH_3OH$)

**Exemple 47**

**1-[[2,4-dichloro-3-[[[4-(1*H*-imidazol-1-yl)-2-méthyl-8-quinolinyl]oxy]méthyl]-phényl]sulfonyl]-N-[2-(4-morpholinyl)éthyl]-2(S)-pyrrolidinecarboxamide**

En opérant de façon analogue à l'exemple 1, au départ d'acide obtenu selon la préparation II et de 4-(2-aminoéthyl)morpholine, on obtient le produit attendu sous forme de cristaux blancs (Rendement = 73 %).
F=100°C
$[\alpha]^{24}_D$ = - 30° (c = 0,62 ; $CH_3OH$)

**Exemple 48**

**1-[[2,4-dichloro-3-[[[4-(1*H*-imidazol-1-yl)-2-méthyl-8-quinolinyl]oxy]méthyl]-phényl]sulfonyl]-N-[2-(4-morpholinyl)éthyl]-2(S)-pyrrolidinecarboxamide, hémisulfate**

En opérant de façon analogue à l'exemple 44, au départ du composé obtenu selon l'exemple 47, on obtient le produit attendu sous forme de cristaux blancs (Rendement = 97 %).

F = 145°C
$[\alpha]^{25}_D$ = - 46° (c = 0,75 ; $CH_3OH$)

**Exemple 49**

**1-[[2,4-dichloro-3-[[[4-(1*H*-imidazol-1-yl)-2-méthyl-8-quinolinyl]oxy]méthyl]-phényl]sulfonyl]-N-[3-(4-morpholinyl)propyl]-2(S)-pyrrolidinecarboxamide**

En opérant de façon analogue à l'exemple 1, au départ d'acide obtenu selon la préparation II et de 4-(3-aminopropyl)morpholine, on obtient le produit attendu sous forme de cristaux blancs (Rendement = 69 %).
F = 96°C
$[\alpha]^{25}_D$ = - 32,5° (c = 0,64 ; $CH_3OH$)

**Exemple 50**

**1-[[2,4-dichloro-3-[[[4-(1*H*-imidazol-1-yl)-2-méthyl-8-quinolinyl]oxy]méthyl]-phényl]sulfonyl]-N-[3-(4-morpholinyl)propyl]-2(S)-pyrrolidinecarboxamide, hémisulfate**

En opérant de façon analogue à l'exemple 44, au départ du composé obtenu selon l'exemple 49, on obtient le produit attendu sous forme de cristaux blancs (Rendement = 98 %).
F = 150°C
$[\alpha]^{25}_D$ = - 46,5° (c = 0,84 ; $CH_3OH$)

**PREPARATION XXIX**

**1-[[2,4-dichloro-3-[[[2-méthyl-4-(1*H*-1,2,4-triazol-1-yl)-8-quinolinyl]oxy]-méthyl]phényl]sulfonyl]-N-[[1-[2-[2-(acétoxy)éthoxy]éthyl]-4-pipéridinyl]-méthyl]-2(S)-pyrrolidinecarboxamide**

En opérant de façon analogue à la préparation XIII, au départ du composé obtenu selon l'exemple 26, on obtient le produit attendu sous forme de cristaux beiges (Rendement = 60 %).
F = 86°C
$[\alpha]^{26}_D$ = - 37,5° (c = 0,78 ; $CH_3OH$)

**Exemple 51**

**1-[[2,4-dichloro-3-[[[2-méthyl-4-(1*H*-1,2,4-triazol-1-yl)-8-quinolinyl]oxy]-méthyl]phényl]sulfonyl]-N-[[1-[2-(2-hydroxyéthoxy)éthyl]-4-pipéridinyl]-méthyl]-2(S)-pyrrolidinecarboxamide**

En opérant de façon analogue à l'exemple 11, au départ du composé obtenu selon la préparation XXIX, on obtient le produit attendu sous forme de cristaux blancs (Rendement = 89 %).
F = 82°C
$[\alpha]^{27}_D$ = - 33,2° (c = 0,76 ; $CH_3OH$)

**Exemple 52**

**1-[[2,4-dichloro-3-[[[2-méthyl-4-(1*H*-1,2,4-triazol-1-yl)-8-quinolinyl]oxy]-méthyl]phényl]sulfonyl]-N-[[1-[2-(2-hydroxyéthoxy)éthyl]-4-pipéridinyl]-méthyl]-2(S)-pyrrolidinecarboxamide, méthanesulfonate**

En opérant de façon analogue à l'exemple 4, au départ du composé obtenu selon l'exemple 51, on obtient le produit attendu sous forme de cristaux jaune pâle (Rendement = 99 %).
F = 135°C
$[\alpha]^{26}_D$ = - 36° (c = 0,70 ; $CH_3OH$)

**PREPARATION XXX**

**N-[[1-[[2,4-dichloro-3-[[[4-(1*H*-imidazol-1-yl)-2-méthyl-8-quinolinyl]oxy]-méthyl]phényl]sulfonyl]-2(S)-pyrrolidinyl]carbonyl]-β-alanine, 1,1-diméthyléthylester**

En opérant de façon analogue à l'exemple 1, au départ de l'acide obtenu selon la préparation II et de l'ester *t*-butylique de la β-alanine, on obtient le produit attendu sous forme d'un solide blanc écru (Rendement = 80 %).
F = 68°C
$[\alpha]^{27}_D$ = - 23° (c = 0,41 ; $CH_3OH$)

**Exemple 53**

**N-[[1-[[2,4-dichloro-3-[[[4-(1*H*-imidazol-1-yl)-2-méthyl-8-quinolinyl]oxy]-méthyl]phényl]sulfonyl]-2(S)-pyrrolidinyl]carbonyl]-β-alanine, trifluoroacétate**

En opérant de façon analogue à l'exemple 13, au départ du composé l'acide obtenu selon la préparation XXX, on obtient le produit attendu sous forme d'un solide fin jaune (Rendement = 94 %).
F = 113°C
$[\alpha]^{27}_D$ = - 8° (c = 0,44 ; $CH_3OH$)

**PREPARATION XXXI**

**1-[[3-(bromométhyl)-2,4-dichlorophényl]sulfonyl]-2(S)-(méthoxyméthyl)-pyrrolidine**

En opérant de façon analogue à la préparation VII, au départ de 2(S)-(méthoxyméthyl)pyrrolidine, on obtient le produit attendu sous forme d'une huile incolore (Rendement = 78 %).
$[\alpha]^{26}_D$ = - 5,5° (c = 0,73 ; $CH_3OH$)

**Exemple 54**

**1-[[2,4-dichloro-3-[[[4-(1*H*-imidazol-1-yl)-2-méthyl-8-quinolinyl)oxy]méthyl]-phényl]sulfonyl]-2(S)-(méthoxyméthyl)pyrrolidine**

En opérant de façon analogue à la préparation I, au départ de 8-hydroxy-4-(1*H*-imidazol-1-yl)-2-méthylquinoléïne et du composé obtenu selon la préparation XXXI, on obtient le produit attendu sous forme de cristaux blancs (Rendement = 44 %).
F = 66°C
$[\alpha]^{27}_D$ = - 31,5° (c = 0,80 ; $CH_3OH$)

**Exemple 55**

**1-[[2,4-dichloro-3-[[[4-(1*H*-imidazol-1-yl)-2-méthyl-8-quinolinyl)oxy]méthyl]-phényl]sulfonyl]-2(S)-(méthoxyméthyl)pyrrolidine, méthanesulfonate**

En opérant de façon analogue à l'exemple 4, au départ du composé obtenu selon l'exemple 54, on obtient le produit attendu sous forme de cristaux jaune pâle (Rendement = 99 %).
F = 123°C
$[\alpha]^{26}_D$ = + 21° (c = 0,85 ; $CH_3OH$)

**Exemple 56**

**1-[[2,4-dichloro-3-[[[2-méthyl-4-(1*H*-pyrazol-1-yl)-8-quinolinyl)oxy]méthyl]-phényl]sulfonyl]-2(S)-(méthoxyméthyl)pyrrolidine**

En opérant de façon analogue à l'exemple 54, au départ de 8-hydroxy-2-méthyl-4-(1*H*-pyrazol-1-yl)-quinoléine, on obtient le produit attendu sous forme de cristaux blancs (Rendement = 73 %).
F = 75°C
$[\alpha]^{24}_D$ = + 1,6° (c = 0,69 ; $CH_3OH$)

**Exemple 57**

**1-[[2,4-dichloro-3-[[[2-méthyl-4-(1*H*-pyrazol-1-yl)-8-quinolinyl)oxy]méthyl]-phényl]sulfonyl]-2(S)-(méthoxyméthyl)pyrrolidine, méthanesulfonate**

En opérant de façon analogue à l'exemple 4, au départ du composé obtenu selon l'exemple 56, on obtient le produit attendu sous forme de cristaux jaunes (Rendement = 97 %).
F = 110°C
$[\alpha]^{26}_{D}$ = + 36° (c = 0,80 ; $CH_3OH$)

**PREPARATION XXXII**

**1-[[2,4-dichloro-3-[[[4-(1*H*-imidazol-1-yl)-2-méthyl-8-quinolinyl]oxy]méthyl]-phényl]sulfonyl]-D-proline, méthyl ester**

En opérant de façon analogue à la préparation I, au départ de l'ester méthylique de la N-[[3-(bromométhyl)-2,4-dichloro-phényl]sulfonyl]-D-proline, on obtient le produit attendu sous forme d'un solide jaune (Rendement = 97 %).
F = 74°C
$[\alpha]^{22}_{D}$ = + 10° (c = 0,60 ; $CH_3OH$)

**PREPARATION XXXIII**

**1-[[2,4-dichloro-3-[[[4-(1*H*-imidazol-1-yl)-2-méthyl-8-quinolinyl]oxy]méthyl]-phényl]sulfonyl]-D-proline**

En opérant de façon analogue à la préparation II, au départ du composé obtenu selon la préparation XXXII, on obtient le produit attendu sous forme d'un solide blanc écru (Rendement = 73 %).
F = 175°C

**Exemple 58**

**1-[[2,4-dichloro-3-[[[4-(1*H*-imidazol-1-yl)-2-méthyl-8-quinolyl]oxy]méthyl]-phényl]sulfonyl]-N-méthyl-2(R)-pyrrolidinecarboxamide**

En opérant de façon analogue à l'exemple 1, au départ du composé obtenu selon la préparation XXXIII, on obtient le produit attendu sous forme d'un solide blanc (Rendement = 83 %).
F = 128°C
$[\alpha]^{25}_{D}$ = + 25° (c = 0,30 ; $CH_3OH$)

**Exemple 59**

**1-[[2,4-dichloro-3-[[[4-(1*H*-imidazol-1-yl)-2-méthyl-8-quinolyl]oxy]méthyl]-phényl]sulfonyl]-N-méthyl-2(R)-pyrrolidinecarboxamide, tartrate**

En opérant de façon analogue à l'exemple 2, au départ du composé obtenu selon l'exemple 58, on obtient le produit attendu sous forme d'un solide fin jaune (Rendement = 80 %).
F=114°C
$[\alpha]^{25}_{D}$ = +25° (c = 0,80 ; $CH_3OH$)

**Exemple 60**

**1-[[2,4-dichloro-3-[[[4-(1*H*-imidazol-1-yl)-2-méthyl-8-quinolinyl]oxy]méthyl]-phényl]sulfonyl]-N-[3-(diméthylamino)propyl]-N-méthyl-2(S)-pyrrolidine-carboxamide**

En opérant de façon analogue à l'exemple 1, au départ de N,N,N'-triméthyl-1,3-propanediamine, on obtient le produit attendu sous forme d'un solide blanc (Rendement = 64 %).
F = 80°C
$[\alpha]^{25}_{D}$ = - 16° (c = 0,34 ; $CH_3OH$)

## Exemple 61

**1-[[2,4-dichloro-3-[[[4-(1*H*-imidazol-1-yl)-2-méthyl-8-quinolinyl]oxy]méthyl]-phényl]sulfonyl]-N-[3-(diméthylamino)propyl]-N-méthyl-2(S)-pyrrolidine-carboxamide, hémisulfate**

En opérant de façon analogue à l'exemple 48, au départ du composé obtenu selon l'exemple 60, on obtient le produit attendu sous forme d'un solide fin blanc (Rendement = 98 %).
F = 133°C
$[\alpha]^{25}_D$ = - 34° (c = 0,40 ; $CH_3OH$)

## Exemple 62

**1-[[2,4-dichloro-3-[[[4-(1*H*-imidazol-1-yl)-2-méthyl-8-quinolinyl]oxy]méthyl]-phényl]sulfonyl]-N-[[1-[2-(acétoxy)éthyl]-4-pipéridinyl]méthyl]-2(S)-pyrrolidinecarboxamide**

En opérant de façon analogue à la préparation XV, au départ d'acétate de 2-bromoéthyle, on obtient le produit attendu sous forme d'un solide beige (Rendement = 38 %).
F = 93°C
$[\alpha]^{22}_D$ = - 48° (c = 0,50 ; $CHCl_3$)

## Exemple 63

**1-[[2,4-dichloro-3-[[[4-(1*H*-imidazol-1-yl)-2-méthyl-8-quinolinyl]oxy]méthyl]-phényl]sulfonyl]-N-[[1-(2-hydroxyéthyl)-4-pipéridinyl]méthyl]-2(S)-pyrrolidinecarboxamide**

En opérant de façon analogue à l'exemple 11, au départ du composé obtenu selon l'exemple 62, on obtient le produit attendu sous forme d'un solide blanc écru (Rendement = 88 %).
F = 104°C
$[\alpha]^{18}_D$ = - 53° (c = 0,75 ; $CHCl_3$)

## Exemple 64

**1-[[2,4-dichloro-3-[[[4-(1*H*-imidazol-1-yl)-2-méthyl-8-quinolinyl]oxy]méthyl]-phényl]sulfonyl]-N-[[1-(2-hydroxyéthyl)-4-pipéridinyl]méthyl]-2(S)-pyrrolidinecarboxamide, hémisulfate**

En opérant de façon analogue à l'exemple 48, au départ du composé obtenu selon l'exemple 63, on obtient le produit attendu sous forme d'un solide blanc (Rendement = 98 %).
F = 160°C
$[\alpha]^{22}_D$ = -48° (c =0,54; $CH_3OH$)

## Exemple 64 bis

**1-[[2,4-dichloro-3-[[[4-(1*H*-imidazol-1-yl)-2-méthyl-8-quinolinyl]oxy]méthyl]-phényl]sulfonyl]-N-[[1-(2-hydroxyéthyl)-4-pipéridinyl]méthyl]-2(S)-pyrrolidinecarboxamide, méthanesulfonate**

En opérant de façon analogue à l'exemple 4, au départ du composé obtenu selon l'exemple 63, on obtient le produit attendu sous forme d'un solide jaune pâle (Rendement = 99 %).
F=127°C
$[\alpha]^{29}_D$ = - 46° (c = 0,82 ; $CH_3OH$)

## PREPARATION XXXIV

**Acide 4-[[[[1-[[2,4-dichloro-3-[[[2-méthyl-4-(1*H*-1,2,4-triazol-1-yl)-8-quinolinyl]oxy]méthyl]phényl]sulfonyl]-2(S)-pyrrolidinyl]carbonyl]amino]méthyl]-1-pipéridineacétique, 1,1-diméthyléthyl ester**

En opérant de façon analogue à la préparation XVI, au départ du composé obtenu selon l'exemple 26, on obtient le produit attendu sous forme d'un solide beige (Rendement = 58 %).
F = 50°C

$[\alpha]^{25}_D$ = - 40° (c = 0,50 ; $CHCl_3$)

**Exemple 65**

**Acide 4-[[[[1-[[2,4-dichloro-3-[[[2-méthyl-4-(1*H*-1,2,4-triazol-1-yl)-8-quinolinyl]oxy]méthyl]phényl]sulfonyl]-2 (S)-pyrrolidinyl]carbonyl]amino]méthyl]-1-pipéridineacétique, bis-trifluoroacétate**

En opérant de façon analogue à l'exemple 24, au départ du composé obtenu selon la préparation XXXIV, on obtient le produit attendu sous forme d'une poudre blanc écru (Rendement = 96 %).
F = 142°C
$[\alpha]^{19}_D$ = - 37° (c = 0,80 ; $CH_3OH$)

**Exemple 66**

**1-[[2,4-dichloro-3-[[[4-(1*H*-imidazol-1-yl)-2-méthyl-8-quinolinyl]oxy]méthyl]-phényl]sulfonyl]-2(S)-pyrrolidine-carboxamide**

En opérant de façon analogue à l'exemple 1, au départ de l'acide obtenu selon la préparation II et d'ammoniac introduit sous forme gazeuse dans le milieu réactionnel, on obtient le produit attendu sous forme d'un solide beige (Rendement = 98 %).
F = 110°C
$[\alpha]^{27}_D$ = - 22,9° (c = 0,31 ; $CH_3OH$)

**Exemple 67**

**1-[[2,4-dichloro-3-[[[4-(1*H*-imidazol-1-yl)-2-méthyl-8-quinolinyl]oxy]méthyl]-phényl]sulfonyl]-2(S)-pyrrolidine-carboxamide, tartrate**

En opérant de façon analogue à l'exemple 2, au départ du composé obtenu selon l'exemple 66, on obtient le produit attendu sous forme d'un solide jaune (Rendement = 97 %).
F = 124°C
$[\alpha]^{27}_D$ = - 12,6° (c = 0,41 ; $CH_3OH$)

**Exemple 68**

**N-cyclopropyl-1-[[2,4-dichloro-3-[[[4-(1*H*-imidazol-1-yl)-2-méthyl-8-quinolinyl]oxy]méthyl]phényl]sulfonyl]-2 (S)-pyrrolidinecarboxamide**

En opérant de façon analogue à l'exemple 1, au départ de cyclopropylamine, on obtient le produit attendu sous forme d'un solide blanc écru (Rendement = 87 %).
F=108°C
$[\alpha]^{24}_D$ = - 19,2° (c = 0,32 ; $CH_3OH$)

**Exemple 69**

**N-cyclopropyl-1-[[2,4-dichloro-3-[[[4-(1*H*-imidazol-1-yl)-2-méthyl-8-quinolinyl]oxy]méthyl]phényl]sulfonyl]-2 (S)-pyrrolidinecarboxamide, chlorhydrate**

En opérant de façon analogue à l'exemple 2, au départ du composé obtenu selon l'exemple 68 et d'une solution de chlorure d'hydrogène dans le méthanol, on obtient le produit attendu sous forme d'une poudre jaune (Rendement = 100 %).
F=160°C
$[\alpha]^{24}_D$ = - 10,9° (c = 0,36 ; $CH_3OH$)

**Exemple 70**

**N-(cyclopropylméthyl)-1-[[2,4-dichloro-3-[[[4-(1*H*-imidazol-1-yl)-2-méthyl-8-quinolinyl]oxy]méthyl]phényl]sulfonyl]-2(S)-pyrrolidinecarboxamide**

En opérant de façon analogue à l'exemple 1, au départ d'(aminométhyl)cyclopropane, on obtient le produit attendu sous forme d'un solide blanc (Rendement = 98 %).
F = 100°C
$[\alpha]^{24}_D$ = - 22,4° (c = 0,42 ; $CH_3OH$)

**Exemple 71**

**N-(cyclopropylméthyl)-1-[[2,4-dichloro-3-[[[4-(1*H*-imidazol-1-yl)-2-méthyl-8-quinolinyl]oxy]méthyl]phényl]sulfonyl]-2(S)-pyrrolidinecarboxamide, chlorhydrate**

En opérant de façon analogue à l'exemple 69, au départ du composé obtenu selon l'exemple 70, on obtient le produit attendu sous forme d'une poudre jaune (Rendement = 99 %).
F = 155°C
$[\alpha]^{24}_D$ = - 5,9° (c = 0,33 ; $CH_3OH$)

**Exemple 72**

**1-[[2,4-dichloro-3-[[[4-(1*H*-imidazol-1-yl)-2-méthyl-8-quinolinyl]oxy]méthyl]-phényl]sulfonyl]-N-[2-(diméthylamino)éthyl]-N-méthyl-2(S)-pyrrolidinecarboxamide**

En opérant de façon analogue à l'exemple 1, au départ du N,N,N'-triméthyléthylènediamine, on obtient le produit attendu sous forme d'un solide blanc (Rendement = 33 %).
F = 98°C
$[\alpha]^{24}_D$ = - 20,2° (c = 0,36 ; $CH_3OH$)

**Exemple 73**

**1-[[2,4-dichloro-3-[[[4-(1*H*-imidazol-1-yl)-2-méthyl-8-quinolinyl]oxy]méthyl]-phényl]sulfonyl]-N-[2-diméthylamino)éthyl]-N-méthyl-2(S)-pyrrolidinecarboxamide, tartrate**

En opérant de façon analogue à l'exemple 1, au départ du composé obtenu selon l'exemple 72, on obtient le produit attendu sous forme d'un solide cotonneux blanc écru (Rendement = 93 %).
F = 125°C
$[\alpha]^{24}_D$ = - 30° (c = 0,43 ; $CH_3OH$)

**Exemple 74**

**1-[[2,4-dichloro-3-[[[4-(1*H*-imidazol-1-yl)-2-méthyl-8-quinolinyl]oxy]methyl]-phényl]sulfonyl]-N-[2-(1-pyrrolidinyl)éthyl]-2(S)-pyrrolidinecarboxamide**

En opérant de façon analogue à l'exemple 1, au départ de 1-(2-aminoéthyl)-pyrrolidine, on obtient le produit attendu sous forme d'un solide blanc (Rendement = 62 %).
F = 105°C
$[\alpha]^{24}_D$ = - 50° (c = 0,35 ; $CH_3OH$)

**Exemple 75**

**1-[[2,4-dichloro-3-[[[4-(1*H*-imidazol-1-yl)-2-méthyl-8-quinolinyl]oxy]méthyl]-phényl]sulfonyl]-N-[2-(1-pyrrolidinyl)éthyl]-2(S)-pyrrolidinecarboxamide, tartrate**

En opérant de façon analogue à l'exemple 2, au départ du composé obtenu selon l'exemple 74, on obtient le produit attendu sous forme d'un solide fin jaune (Rendement = 97 %).
F = 129°C

$[\alpha]^{24}_D$ = - 30° (c = 0,45 ; $CH_3OH$)

**Exemple 76**

**1-[[2,4-dichloro-3-[[[4-(1*H*-imidazol-1-yl)-2-méthyl-8-quinolinyl]oxy]méthyl]-phényl]sulfonyl]-N-[3-(1-pyrrolidinyl)propyl]-2(S)-pyrrolidinecarboxamide**

En opérant de façon analogue à l'exemple 1, au départ de 1-(3-aminopropyl)-pyrrolidine, on obtient le produit attendu sous forme d'un solide blanc écru (Rendement = 51 %).
F = 120°C
$[\alpha]^{24}_D$ = - 51° (c = 0,35 ; $CH_3OH$)

**Exemple 77**

**1-[[2,4-dichloro-3-[[[4-(1*H*-imidazol-1-yl)-2-méthyl-8-quinolinyl]oxy]méthyl]-phényl]sulfonyl]-N-[3-(1-pyrrolidinyl)propyl)-2(S)-pyrrolidinecarboxamide, tartrate**

En opérant de façon analogue à l'exemple 2, au départ du composé obtenu selon l'exemple 76, on obtient le produit attendu sous forme d'un solide fin jaune (Rendement = 95 %).
F = 128°C
$[\alpha]^{24}_D$ = - 32° (c = 0,35 ; $CH_3OH$)

**Exemple 78**

**1-[[2,4-dichloro-3-[[[4-(1*H*-imidazol-1-yl)-2-méthyl-8-quinolinyl]oxy]méthyl]-phényl]sulfonyl]-N-[2-(1-pipéridinyl)éthyl]-2(S)-pyrrolidinecarboxamide**

En opérant de façon analogue à l'exemple 1, au départ de 1-(2-aminoéthyl)-pipéridine, on obtient le produit attendu sous forme d'un solide blanc (Rendement = 63 %).
F = 108°C
$[\alpha]^{22}_D$ = - 35,1° (c = 0.37 ; $CH_3OH$)

**Exemple 79**

**1-[[2,4-dichloro-3-[[[4-(1*H*-imidazol-1-yl)-2-méthyl-8-quinolinyl]oxy]méthyl]-phényl]sulfonyl]-N-[2-(1-pipéridinyl)éthyl]-2(S)-pyrrolidinecarboxamide, tartrate**

En opérant de façon analogue à l'exemple 2, au départ du composé obtenu selon l'exemple 78, on obtient le produit attendu sous forme d'un solide jaunâtre (Rendement = 98 %).
F = 125°C
$[\alpha]^{20}_D$ = - 43,7° (c = 0,42 ; $CH_3OH$)

**Exemple 80**

**N-cyclopentyl-1-[[2,4-dichloro-3-[[[4-(1*H*-imidazol-1-yl)-2-méthyl-8-quinolinyl]oxy]méthyl]phényl]sulfonyl]-pyrrolidinecarboxamide**

En opérant de façon analogue à l'exemple 1, au départ de cyclopentylamine, on obtient le produit attendu sous forme d'un solide blanc (Rendement = 86 %).
F = 75°C
$[\alpha]^{25}_D$ = - 17,5° (c = 1,05 ; $CH_3OH$)

**Exemple 81**

**N-cyclopentyl-1-[[2,4-dichloro-3-[[[4-(1*H*-imidazol-1-yl)-2-méthyl-8-quinolinyl]oxy]méthyl]phényl]sulfonyl]-pyrrolidinecarboxamide, méthanesulfonate**

En opérant de façon analogue à l'exemple 6, au départ du composé obtenu selon l'exemple 80, on obtient le

produit attendu sous forme d'un solide blanc cassé (Rendement = 91 %).
F=134°C
$[\alpha]^{25}_{D}$ = +3,8° (c = 0,83 ; DMSO)

**PREPARATION XXXV**

**1-[[2,4-dichloro-3-[[[2-méthyl-4-(1*H*-1,2,4-triazol-1-yl)-8-quinolinyl]oxy]-méthyl]phényl]sulfonyl]-L-proline, méthyl ester**

En opérant de façon analogue à la préparation I, au départ de 8-hydroxy-2-méthyl-4-(1*H*-1,2,4-triazol-1-yl)-quinoléine, on obtient le produit attendu sous forme d'un solide jaune pâle (Rendement = 98 %).
F=130°C
$[\alpha]^{22}_{D}$ = - 35° (c =0,68 ; $CHCl_3$)

**PREPARATION XXXVI**

**1-[[2,4-dichloro-3-[[[2-méthyl-4-(1*H*-1,2,4-triazol-1-yl)-8-quinolinyl]oxy]-méthyl]phényl]sulfonyl]-L-proline**

En opérant de façon analogue à la préparation II, au départ du composé obtenu selon la préparation XXXV, on obtient le produit attendu sous forme d'une poudre jaune pâle (Rendement = 75 %).
F=146°C
$[\alpha]^{24}_{D}$ = - 5° (c = 0,65 ; $CH_3OH$)

**Exemple 82**

**1-[[2,4-dichloro-3-[[[2-méthyl-4-(1*H*-1,2,4-triazol-1-yl)-8-quinolinyl]oxy]-méthyl]phényl]sulfonyl]-N-[2-(diméthylamino)éthyl]-2(S)-pyrrolidinecarboxamide**

En opérant de façon analogue à l'exemple 1, au départ du composé obtenu selon la préparation XXXVI et de N,N-diméthyl-éthylènediamine, on obtient le produit attendu sous forme de cristaux blancs (Rendement = 60 %).
F = 106°C
$[\alpha]^{25}_{D}$ = - 35,2° (c = 0,45 ; $CH_3OH$)

**Exemple 83**

**1-[[2,4-dichloro-3-[[[2-méthyl-4-(1*H*-1,2,4-triazol-1-yl)-8-quinolinyl]oxy]-méthyl]phényl]sulfonyl]-N-[2-(diméthylamino)éthyl]-2(S)-pyrrolidinecarboxamide, tartrate**

En opérant de façon analogue à l'exemple 2, au départ du composé obtenu selon l'exemple 82, on obtient le produit attendu sous forme de cristaux blancs (Rendement = 97 %).
F = 128°C
$[\alpha]^{25}_{D}$ = - 38,1° (c = 1 ; $CH_3OH$)

**Exemple 84**

**1-[[2,4-dichloro-3-[[[2-méthyl-4-(1*H*-1,2,4-triazol-1-yl)-8-quinolinyl]oxy]-méthyl]phényl]sulfonyl]-N-[3-(diméthylamino)propyl]-2(S)-pyrrolidinecarboxamide**

En opérant de façon analogue à l'exemple 82, au départ de N,N-diméthyl-1,3propylènediamine, on obtient le produit attendu sous forme d'un solide blanc (Rendement = 74 %).
F = 105°C
$[\alpha]^{25}_{D}$ = - 51° (c = 0,75 ; $CHCl_3$)

**Exemple 85**

**1-[[2,4-dichloro-3-[[[2-méthyl-4-(1*H*-1,2,4-triazol-1-yl)-8-quinolinyl]oxy]-méthyl]phényl]sulfonyl]-N-[3-(diméthylamino)propyl]-2(S)-pyrrolidinecarboxamide, tartrate**

En opérant de façon analogue à l'exemple 2, au départ du composé obtenu selon l'exemple 84, on obtient le produit attendu sous forme de cristaux blancs (Rendement = 99 %).
F = 124°C
$[\alpha]^{25}_D$ = - 42,4° (c = 1 ; $CH_3OH$)

**Exemple 86**

**1-[[2,4-dichloro-3-[[[2-méthyl-4-(1*H*-1,2,4-triazol-1-yl)-8-quinolinyl]oxy]-méthyl]phényl]sulfonyl]-N-[3-(1-pyrrolidinyl)propyl]-2(S)-pyrrolidinecarboxamide**

En opérant de façon analogue à l'exemple 82, au départ de 1(3-aminopropyl)-pyrrolidine, on obtient le produit attendu sous forme de cristaux blancs (Rendement = 65 %).
F = 86°C
$[\alpha]^{25}_D$ = - 37,8° (c = 0,67 ; $CH_3OH$)

**Exemple 87**

**1-[[2,4-dichloro-3-[[[2-méthyl-4-(1*H*-1,2,4-triazol-1-yl)-8-quinolinyl]oxy]-méthyl]phényl]sulfonyl]-N-[3-(1-pyrrolidinyl)propyl]-2(S)-pyrrolidinecarboxamide, méthanesulfonate**

En opérant de façon analogue à l'exemple 6, au départ du composé obtenu selon l'exemple 86, on obtient le produit attendu sous forme d'un solide fin blanc (Rendement = 99 %).
F = 110°C
$[\alpha]^{25}_D$ = - 54.6° (c = 0,63 ; $CH_3OH$)

**Exemple 88**

**1-[[2,4-dichloro-3-[[[4-(1*H*-imidazol-1-yl)-2-méthyl-8-quinolinyl]oxy]méthyl]-phényl]sulfonyl]-N-[(2-pyridinyl)méthyl]-2(S)-pyrrolidinecarboxamide**

En opérant de façon analogue à l'exemple 1, au départ de 2-(aminométhyl)-pyridine, on obtient le produit attendu sous forme d'un solide blanc (Rendement = 72 %).
F = 102°C
$[\alpha]^{25}_D$ = - 37,5° (c = 0,61 ; $CH_3OH$)

**Exemple 89**

**1-[[2,4-dichloro-3-[[[4-(1*H*-imidazol-1-yl)-2-méthyl-8-quinolinyl]oxy]méthyl]-phényl]sulfonyl]-N-[(2-pyridinyl)méthyl]-2(S)-pyrrolidinecarboxamide, méthanesulfonate**

En opérant de façon analogue à l'exemple 6, au départ du composé obtenu selon l'exemple 88, on obtient le produit attendu sous forme de flocons blanc cassé (Rendement = 95 %).
F = 130°C
$[\alpha]^{25}_D$ = - 32,6° (c = 0,54 ; $CH_3OH$)

**Exemple 90**

**1-[[2,4-dichloro-3-[[[4-(1*H*-imidazol-1-yl)-2-méthyl-8-quinolinyl]oxy]méthyl]-phényl]sulfonyl]-N-[(3-pyridinyl)méthyl]-2(S)-pyrrolidinecarboxamide**

En opérant de façon analogue à l'exemple 1, au départ de 3-(aminométhyl)-pyridine, on obtient le produit attendu sous forme d'un solide blanc (Rendement = 84 %).
F=104°C

$[\alpha]^{25}_D$ = - 41,9° (c = 0,59 ; $CH_3OH$)

**Exemple 91**

**1-[[2,4-dichloro-3-[[[4-(1*H*-imidazol-1-yl)-2-méthyl-8-quinolinyl]oxy]méthyl]-phényl]sulfonyl]-N-[(3-pyridinyl) méthyl]-2(S)-pyrrolidinecarboxamide, méthanesulfonate**

En opérant de façon analogue à l'exemple 6, au départ du composé obtenu selon l'exemple 90, on obtient le produit attendu sous forme de cristaux blancs (Rendement = 99 %).
F = 126°C
$[\alpha]^{25}_D$ = - 35,5° (c = 0,56 ; $CH_3OH$)

**Exemple 92**

**1-[[2,4-dichloro-3-[[[4-(1*H*-imidazol-1-yl)-2-méthyl-8-quinolinyl]oxy]méthyl]-phényl]sulfonyl]-N-[(4-pyridinyl) méthyl]-2(S)-pyrrolidinecarboxamide**

En opérant de façon analogue à l'exemple 1, au départ de 4-(arninométhyl)-pyridine, on obtient le produit attendu sous forme d'un solide blanc (Rendement = 90 %).
F=114°C
$[\alpha]^{25}_D$ = - 50,3° (c = 0,51 ; $CH_3OH$)

**Exemple 93**

**1-[[2,4-dichloro-3-[[[4-(1*H*-imidazol-1-yl)-2-méthyl-8-quinolinyl]oxy]méthyl]-phényl]sulfonyl]-N-[(4-pyridinyl) méthyl]-2(S)-pyrrolidinecarboxamide, tartrate**

En opérant de façon analogue à l'exemple 2, au départ du composé obtenu selon l'exemple 92, on obtient le produit attendu sous forme d'un solide jaune (Rendement = 98 %).
F = 128°C
$[\alpha]^{25}_D$ = - 34,5° (c = 0,49 ; $CH_3OH$)

**Exemple 94**

**8-[[2,6-dichloro-3-[[2(S)-[[4-(4-pyridinyl)-1-pipérazinyl]carbonyl]-1-pyrrolidinyl]sulfonyl]phényl]méthoxy]-4-(1*H*-imidazol-1-yl)-2-méthyl-quinoléïne**

En opérant de façon analogue à l'exemple 1, au départ de 1-(4-pyridinyl)pipérazine, on obtient le produit attendu sous forme d'un solide blanc (Rendement = 45 %).
F = 136°C
$[\alpha]^{25}_D$ = - 32,3° (c = 0,46 ; $CH_3OH$)

**Exemple 95**

**8-[[2,6-dichloro-3-[[2(S)-[[4-(4-pyridinyl)-1-pipérazinyl]carbonyl]-1-pyrrolidinyl]sulfonyl]phényl]méthoxy]-4-(1*H*-imidazol-1-yl)-2-méthyl-quinoléïne, tartrate**

En opérant de façon analogue à l'exemple 2, au départ du composé obtenu selon l'exemple 94, on obtient le produit attendu sous forme de flocons jaune pâle (Rendement = 89 %).
F = 146°C
$[\alpha]^{25}_D$ = - 23,2° (c =0,52 ; $CH_3OH$)

**Exemple 96**

**8-[[2,6-dichloro-3-[[2(S)-[[4-(2-pyridinyl)-1-pipérazinyl]carbonyl]-1-pyrrolidinyl]sulfonyl]phényl]méthoxy]-4-(1*H*-imidazol-1-yl)-2-méthyl-quinoléïne**

En opérant de façon analogue à l'exemple 1, au départ de 1-(2-pyridinyl)pipérazine, on obtient le produit

attendu sous forme d'un solide blanc (Rendement = 34 %).
F=108°C
$[\alpha]^{25}_D$ = - 27,6° (c =0,4 ; $CH_3OH$)

**Exemple 97**

**8-[[2,6-dichloro-3-[[2(S)-[[4-(2-pyridinyl)-1-pipérazinyl]carbonyl]-1-pyrrolidinyl]sulfonyl]phényl]méthoxy]-4-(1*H*-imidazol-1-yl)-2-méthyl-quinoléïne, méthanesulfonate**

En opérant de façon analogue à l'exemple 6, au départ du composé obtenu selon l'exemple 96, on obtient le produit attendu sous forme d'un solide blanc (Rendement = 99 %).
F=138°C
$[\alpha]^{25}_D$ = - 17,3° (c = 0,37 ; $CH_3OH$)

**PREPARATION XXXVII**

**Acide [2-[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl](méthyl)amino] éthyl](méthyl)carbamique, 1,1-diméthyléthyl ester**

En opérant de façon analogue à l'exemple 1, au départ de l'ester t-butylique de l'acide [2-(méthylamino)éthyl](méthyl)carbamique, on obtient le produit attendu sous forme de cristaux blancs (Rendement = 93 %).
F = 75°C
$[\alpha]^{25}_D$ = - 21,4° (c = 0,67 ; $CH_3OH$)

**Exemple 98**

**1-[[2,4-dichloro-3-[[[4-(1*H*-imidazol-1-yl)-2-méthyl-8-quinolinyl]oxy]méthyl]-phényl]sulfonyl]-N-méthyl-N-[2-(méthylamino)éthyl]-2(S)-pyrrolidine carboxamide**

En opérant de façon analogue à l'exemple 9, au départ du composé obtenu selon la préparation XXXVII, on obtient le produit attendu sous forme de cristaux jaunes (Rendement = 97 %).
F = 116°C
$[\alpha]^{25}_D$ = - 22,6° (c = 0,6 ; $CH_3OH$)

**PREPARATION XXXVIII**

**Acide [3-[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl](méthyl)amino]-propyl](méthyl)carbamique, 1,1-diméthyléthyl ester**

En opérant de façon analogue à l'exemple 1, au départ de l'ester t-butylique de l'acide [3-(méthylamino)propyl](méthyl)carbamique, on obtient le produit attendu sous forme de cristaux jaune pâle (Rendement = 86 %).
F = 70°C
$[\alpha]^{25}_D$ = - 16,4° (c = 0,6 ; $CH_3OH$)

**Exemple 99**

**1-[[2,4-dichloro-3-[[[4-(1*H*-imidazol-1-yl)-2-méthyl-8-quinolinyl]oxy]méthyl]-phényl]sulfonyl]-N-méthyl-N-[3-(méthylamino)propyl]-2(S)-pyrrolidine carboxamide**

En opérant de façon analogue à l'exemple 9, au départ du composé obtenu selon la préparation XXXVIII, on obtient le produit attendu sous forme de cristaux jaunes (Rendement = 99 %).
F=125°C
$[\alpha]^{25}_D$ = - 34,5° (c = 0,54 ; $CH_3OH$)

**PREPARATION XXXIX**

**1-[[2,4-dichloro-3-[[[4-(1*H*-imidazol-1-yl)-2-méthyl-8-quinolinyl]oxy]méthyl]-phényl]sulfonyl]-N-méthyl-N-[2-[[3-(acétoxy)propyl](méthyl)amino]éthyl]-2(S)-pyrrolidinecarboxamide**

En opérant de façon analogue à la préparation XIII, au départ du composé obtenu selon l'exemple 98 et d'acétate de 3-iodopropyle, on obtient le produit attendu sous forme de cristaux blancs (Rendement = 44 %).
F = 75°C
$[\alpha]^{25}_D$ = - 16,1° (c = 0,6 ; $CH_3OH$)

**PREPARATION XL**

**1-[[2,4-dichloro-3-[[[4-(1*H*-imidazol-1-yl)-2-méthyl-8-quinolinyl]oxy]méthyl]-phényl]sulfonyl]-N-méthyl-N-[2-[[4-(acétoxy)butyl](méthyl)amino]éthyl]-2(S)-pyrrolidinecarboxamide**

En opérant de façon analogue à la préparation XXXIX, au départ d'acétate de 4-bromobutyle, on obtient le produit attendu sous forme de cristaux blancs (Rendement = 55 %).
F = 76°C
$[\alpha]^{25}_D$ = -14,2° (c = 0,53 ; $CH_3OH$)

**PREPARATION XLI**

**1-[[2,4-dichloro-3-[[[4-(1*H*-imidazol-1-yl)-2-méthyl-8-quinolinyl]oxy]méthyl]-phényl]sulfonyl]-N-méthyl-N-[3-[[3-(acétoxy)propyl](méthyl)amino]propyl]-2(S)-pyrrolidinecarboxamide**

En opérant de façon analogue à la préparation XXXIX, au départ du composé obtenu selon l'exemple 99, on obtient le produit attendu sous forme de cristaux beiges (Rendement = 73 %).
F = 90°C
$[\alpha]^{25}_D$ = - 28,3° (c = 0,68 ; $CH_3OH$)

**PREPARATION XLII**

**1-[[2,4-dichloro-3-[[[4-(1*H*-imidazol-1-yl)-2-méthyl-8-quinolinyl]oxy]méthyl]-phényl]sulfonyl]-N-méthyl-N-[3-[[4-(acétoxy)butyl](méthyl)amino]propyl]-2(S)-pyrrolidinecarboxamide**

En opérant de façon analogue à la préparation XL, au départ du composé obtenu selon l'exemple 99, on obtient le produit attendu sous forme de cristaux jaune pâle (Rendement = 69 %).
F = 88°C
$[\alpha]^{25}_D$ = - 29,10 ° (c = 0,7 ; $CH_3OH$)

**Exemple 100**

**1-[[2,4-dichloro-3-[[[4-(1*H*-imidazol-1-yl)-2-méthyl-8-quinolinyl]oxy]méthyl]-phényl]sulfonyl]-N-méthyl-N-[2-[[3-(hydroxy)propyl](méthyl)amino]éthyl]-2(S)-pyrrolidinecarboxamide**

En opérant de façon analogue à l'exemple 11, au départ du composé obtenu selon la préparation XXXIX, on obtient le produit attendu sous forme de cristaux blancs (Rendement = 92 %).
F = 98°C
$[\alpha]^{25}_D$ = - 15,9° (c = 0,6 ; $CH_3OH$)

**Exemple 101**

**1-[[2,4-dichloro-3-[[[4-(1*H*-imidazol-1-yl)-2-méthyl-8-quinolinyl]oxy]méthyl]-phényl]sulfonyl]-N-méthyl-N-[2-[[3-(hydroxy)propyl](méthyl)amino]éthyl]-2(S)-pyrrolidinecarboxamide, méthanesulfonate**

En opérant de façon analogue à l'exemple 4, au départ du composé obtenu selon l'exemple 100, on obtient le produit attendu sous forme de flocons blancs (Rendement = 99.%).
F = 118°C

$[\alpha]^{25}_D$ = - 37,1° (c=0,6 ; $CH_3OH$)

**Exemple 102**

**1-[[2,4-dichloro-3-[[[4-(1*H*-imidazol-1-yl)-2-méthyl-8-quinolinyl]oxy]méthyl]-phényl]sulfonyl]-N-méthyl-N-[2-[[4-(hydroxy)butyl](méthyl)amino]éthyl]-2(S)-pyrrolidinecarboxamide**

En opérant de façon analogue à l'exemple 11, au départ du composé obtenu selon la préparation XL, on obtient le produit attendu sous forme de cristaux blancs (Rendement = 74 %).
F = 84°C
$[\alpha]^{25}_D$ = - 18,1° (c = 0,62 ; $CH_3OH$)

**Exemple 103**

**1-[[2,4-dichloro-3-[[[4-(1*H*-imidazol-1-yl)-2-méthyl-8-quinolinyl]oxy]méthyl]-phényl]sulfonyl]-N-méthyl-N-[2-[[4-(hydroxy)butyl](méthyl)amino]éthyl]-2(S)-pyrrolidinecarboxamide, méthanesulfonate**

En opérant de façon analogue à l'exemple 6, au départ du composé obtenu selon l'exemple 102, on obtient le produit attendu sous forme de flocons blancs (Rendement = 99 %).
F = 120°C
$[\alpha]^{25}_D$ = - 43,2° (c = 0,65 ; $CH_3OH$)

**Exemple 104**

**1-[[2,4-dichloro-3-[[[4-(1*H*-imidazol-1-yl)-2-méthyl-8-quinolinyl]oxy]méthyl]-phényl]sulfonyl]-N-méthyl-N-[3-[[3-(hydroxy)propyl](méthyl)amino]propyl]-2(S)-pyrrolidinecarboxamide**

En opérant de façon analogue à l'exemple 11, au départ du composé obtenu selon la préparation XLI, on obtient le produit attendu sous forme de cristaux blancs (Rendement = 84 %).
F = 92°C
$[\alpha]^{25}_D$ = - 18,1° (c = 0,56; $CH_3OH$)

**Exemple 105**

**1-[[2,4-dichloro-3-[[2(L)-[[4-(2-pyridinyl)-1-pipérazinyl]carbonyl]-1-pyrrolidinyl]sulfonyl]-N-méthyl-N-[3-[[3-(hydroxy)propyl](méthyl)amino]propyl]-2(S)-pyrrolidinecarboxamide, méthanesulfonate**

En opérant de façon analogue à l'exemple 6, au départ du composé obtenu selon l'exemple 104, on obtient le produit attendu sous forme de flocons blancs (Rendement = 99 %).
F = 116°C
$[\alpha]^{25}_D$ = - 49,1° (c = 0,69 ; $CH_3OH$)

**Exemple 106**

**1-[[2,4-dichloro-3-[[[4-(1*H*-imidazol-1-yl)-2-méthyl-8-quinolinyl]oxy]méthyl]-phényl]sulfonyl]-N-méthyl-N-[3-[[4-(hydroxy)butyl](méthyl)amino]propyl]-2(S)-pyrrolidinecarboxamide**

En opérant de façon analogue à l'exemple 11, au départ du composé obtenu selon la préparation XLII, on obtient le produit attendu sous forme de cristaux blancs (Rendement = 77 %).
F = 82°C
$[\alpha]^{25}_D$ = - 22,1° (c = 0,62 ; $CH_3OH$)

**Exemple 107**

**1-[[2,4-dichloro-3-[[[4-(1*H*-imidazol-1-yl)-2-méthyl-8-quinolinyl]oxy]méthyl]-phényl]sulfonyl]-N-méthyl-N-[3-[[4-(hydroxy)butyl](méthyl)amino]propyl]-2(S)-pyrrolidinecarboxamide, méthanesulfonate**

En opérant de façon analogue à l'exemple 6, au départ du composé obtenu selon l'exemple 106, on obtient

le produit attendu sous forme de flocons blancs (Rendement = 98 %).
F=100°C
$[\alpha]^{25}_D$ = - 49,5° (c = 0,58 ; $CH_3OH$)

**Exemple 108**

**1-[[2,4-dichloro-3-[[[4-(1*H*-imidazol-1-yl)-2-méthyl-8-quinolinyl]oxy]méthyl]-phényl]sulfonyl]-N-[[1-(2-pyridinylméthyl)-4-pipéridinyl]méthyl]-2(S)-pyrrolidinecarboxamide**

En opérant de façon analogue à la préparation XIII, au départ du chlorhydrate de 2-(chlorométhyl)pyridine, on obtient le produit attendu sous forme d'un solide blanc (Rendement = 31 %).
F = 100°C
$[\alpha]^{25}_D$ = - 43,2° (c = 0,4 ; $CH_3OH$)

**Exemple 109**

**1-[[2,4-dichloro-3-[[[4-(1*H*-imidazol-1-yl)-2-méthyl-8-quinolinyl]oxy]méthyl]-phényl]sulfonyl]-N-[[1-(2-pyridinylméthyl)-4-pipéridinyl]méthyl]-2(S)-pyrrolidinecarboxamide, méthanesulfonate**

En opérant de façon analogue à l'exemple 6, au départ du composé obtenu selon l'exemple 108, on obtient le produit attendu sous forme d'un solide blanc (Rendement = 90 %).
F = 130°C
$[\alpha]^{25}_D$ = - 44,8° (c = 0,3 ; $CH_3OH$)

**Exemple 110**

**1-[[2,4-dichloro-3-[[[4-(1*H*-imidazol-1-yl)-2-méthyl-8-quinolinyl]oxy]méthyl]-phényl]sulfonyl]-N-[[1-(3-pyridinylméthyl)-4-pipéridinyl]méthyl]-2(S)-pyrrolidinecarboxamide**

En opérant de façon analogue à la préparation XIII, au départ du chlorhydrate de 3-(chlorométhyl)pyridine, on obtient le produit attendu sous forme d'une poudre blanche (Rendement = 80 %).
F = 107°C
$[\alpha]^{25}_D$ = - 30,7° (c = 0,35 ; $CH_3OH$)

**Exemple 111**

**1-[[2,4-dichloro-3-[[[4-(1*H*-imidazol-1-yl)-2-méthyl-8-quinolinyl]oxy]méthyl]-phényl]sulfonyl]-N-[[1-(3-pyridinylméthyl)-4-pipéridinyl]méthyl]-2(S)-pyrrolidinecarboxamide, méthanesulfonate**

En opérant de façon analogue à l'exemple 6, au départ du composé obtenu selon l'exemple 110, on obtient le produit attendu sous forme de flocons blancs (Rendement = 99 %).
F=141°C
$[\alpha]^{25}_D$ = - 44,4° (c =0,36 ; $CH_3OH$)

**Exemple 112**

**1-[[2,4-dichloro-3-[[[4-(1*H*-imidazol-1-yl)-2-méthyl-8-quinolinyl]oxy]méthyl]-phényl]sulfonyl]-N-[[1-(4-pyridinylméthyl)-4-pipéridinyl]méthyl]-2(S)-pyrrolidinecarboxamide**

En opérant de façon analogue à la préparation XIII, au départ du chlorhydrate de 4-(chlorométhyl)pyridine, on obtient le produit attendu sous forme d'un solide jaune (Rendement = 83 %).
F = 113°C
$[\alpha]^{25}_D$ = - 27,7° (c = 0,39 ; $CH_3OH$)

**Exemple 113**

**1-[[2,4-dichloro-3-[[[4-(1*H*-imidazol-1-yl)-2-méthyl-8-quinolinyl]oxy]méthyl]-phényl]sulfonyl]-N-[[1-(4-pyridinylméthyl)-4-pipéridinyl]méthyl]-2(S)-pyrrolidinecarboxamide, méthanesulfonate**

En opérant de façon analogue à l'exemple 6, au départ du composé obtenu à l'exemple 112, on obtient le produit attendu sous forme d'un solide blanc (Rendement = 92 %).
F = 96°C
$[\alpha]^{25}_D$ = - 39,8° (c = 0,34 ; $CH_3OH$)

**PREPARATION XLIII**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridinepropanoïque, 1,1-diméthyléthyl ester**

On prépare une suspension de 0,7 g ($1,06.10^{-3}$ mole) du composé obtenu selon l'exemple 9 dans 20 ml de tétrahydrofurane. On ajoute, à 50°C, 0,66 g ($5,1.10^{-3}$ mole) d'acrylate de t-butyle et on maintient sous agitation pendant 100 heures à 50°C. Le solvant est chassé sous pression réduite et le résidu est purifié par chromatographie sur gel de silice en éluant à l'aide d'un mélange dichlorométhane/méthanol/ammoniaque (97/3/0,1 ; v/v/v). On obtient ainsi 0,5 g du produit attendu sous forme d'un solide blanc (Rendement = 61 %).
F = 92°C
$[\alpha]^{23}_D$ = - 41° (c = 0,31 ; $CH_3OH$)

**Exemple 114**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridinepropanoïque**

On prépare une solution de 0,48 g ($0,61.10^{-3}$ mole) du composé obtenu selon la préparation XLIII dans 30 ml de dichlorométhane et on ajoute, à 0°C, 66 mg ($0,61.10^{-3}$ mole) d'anisole et, goutte à goutte, 10 ml d'acide trifluoroacétique. Le mélange réactionnel est ensuite maintenu sous agitation à température ambiante pendant 20 heures, puis concentré sous pression réduite. Le résidu est trituré dans 10 ml d'éther diéthylique et le produit solide obtenu est séparé par filtration puis purifié par chromatographie sur gel de silice en éluant à l'aide d'un mélange dichlorométhane/méthanol/ammoniaque (80/20/2 ; v/v/v). On obtient ainsi 0,2 g du produit attendu sous forme d'un solide blanc (Rendement = 45 %).
F = 140°C
$[\alpha]^{22}_D$ = - 52° (c = 0,35 ; $CH_3OH$)

**Exemple 115**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridinepropanoïque, tartrate**

En opérant de façon analogue à l'exemple 2, au départ du composé obtenu à l'exemple 114, on obtient le produit attendu sous forme d'un solide blanc (Rendement = 86 %).
F=138°C
$[\alpha]^{23}_D$ = - 32° (c = 0,39 ; $CH_3OH$)

**PREPARATION XLIV**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridinebutanoïque, méthyl ester**

En opérant de façon analogue à la préparation XIII, au départ du composé obtenu selon l'exemple 9 et de 4-bromobutanoate de méthyle, on obtient le produit attendu sous forme d'un solide blanc écru (Rendement = 58 %).
F = 98°C
$[\alpha]^{22}_D$ = - 40° (c = 0,43 ; $CH_3OH$)

**Exemple 116**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridinebutanoïque**

On prépare une solution de 0,49 g (0,65.$10^{-3}$ mole) de l'ester obtenu selon la préparation XLIV dans 10 ml de dioxane et on ajoute 1,3 ml d'une solution de soude N. Le mélange réactionnel est chauffé à reflux pendant 10 heures puis concentré sous pression réduite. Le résidu est repris dans l'eau et acidifié jusqu'à pH 4,5 à l'aide d'une solution diluée d'acide chlorhydrique. L'eau est éliminée par lyophilisation et le solide obtenu est purifié par chromatographie sur gel de silice greffée RP18 en éluant à l'aide d'un mélange acétonitrile/eau (2/1 ;v/v). On obtient ainsi 0,24 g du produit attendu sous forme d'un solide blanc (Rendement = 50 %).
F=178°C
$[\alpha]^{20}_D$ = - 16° (c = 0,5 ; $CH_3OH$)

**Exemple 117**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-1-pipéridinebutanoïque, tartrate**

En opérant de façon analogue à l'exemple 2, au départ du composé obtenu selon l'exemple 116, on obtient le produit attendu sous forme d'un solide blanc (Rendement = 87 %).
F = 158°C
$[\alpha]^{24}_D$ = - 7° (c = 0,34 ; $CH_3OH$)

**PREPARATION XLV**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-β-oxo-1-pipéridinepropanoïque, 1,1-diméthyléthyl ester**

En opérant de façon analogue à l'exemple 1, au départ du composé obtenu selon l'exemple 9 et de malonate de mono-t-butyle, on obtient le produit attendu sous forme d'un solide blanc (Rendement = 59 %).
F = 101°C
$[\alpha]^{29}_D$ = - 34° (c = 0,33 ; $CH_3OH$)

**Exemple 118**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-β-oxo-1-pipéridinepropanoïque, trifluororacétate**

En opérant de façon analogue à l'exemple 114, au départ du composé obtenu selon la préparation XLV, on obtient le produit attendu sous forme d'un solide jaune (Rendement = 87 %).
F = 130°C
$[\alpha]^{22}_D$ = - 22° (c = 0,56 ; $CH_3OH$)

**Exemple 119**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-γ-oxo-1-pipéridinebutanoïque**

On prépare une suspension de 0,5 g (0,76.$10^{-3}$ mole) du composé obtenu selon l'exemple 9, dans 15 ml d'acétone et on ajoute 76 mg (0,76.$10^{-3}$ mole) d'anhydride succinique. Le mélange réactionnel est chauffé à reflux pendant 8 heures et le solvant est éliminé sous pression réduite. Le résidu est purifié par chromatographie sur gel de silice en éluant à l'aide d'un mélange dichlorométhane/méthanol/ammoniaque (90/10/1 ; v/v/v). On obtient ainsi 0,26 g du produit attendu sous forme d'un solide blanc (Rendement = 45 %).
F = 125°C
$[\alpha]^{22}_D$ = - 33° (c = 0,38 ; $CH_3OH$)

**Exemple 120**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-γ-oxo-1-pipéridinebutanoïque, tartrate**

En opérant de façon analogue à l'exemple 2, au départ du composé obtenu selon l'exemple 119, on obtient le produit attendu sous forme d'un solide jaune (Rendement = 75 %).
F=130°C
$[\alpha]^{19}_D$ = - 22° (c = 0,50 ; $CH_3OH$)

**PREPARATION XLVI**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-δ-oxo-1-pipéridinepentanoïque, 1,1-diméthyléthyl ester**

En opérant de façon analogue à l'exemple 1, au départ du composé obtenu selon l'exemple 9 et de glutarate de mono t-butyle, on obtient le produit attendu sous forme d'un solide blanc (Rendement = 44 %).
F = 112°C
$[\alpha]^{22}_D$ = - 41° (c = 0,30 ; $CH_3OH$)

**Exemple 121**

**Acide 4-[[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]méthyl]-δ-oxo-1-pipéridinepentanoïque, trifluoroacétate**

En opérant de façon analogue à l'exemple 13, au départ du composé obtenu selon la préparation XLVI, on obtient le produit attendu sous forme d'un solide fin jaune (Rendement = 77 %).
F = 131°C
$[\alpha]^{23}_D$ = - 15° (c = 0,37 ; $CH_3OH$)

**PREPARATION XLVII**

**N-[2-[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl](méthyl)amino]-éthyl]-N-méthyl-glycine, 1,1-diméthyléthyl ester**

En opérant de façon analogue à la préparation XVI, au départ du composé obtenu selon l'exemple 98, on obtient le produit attendu sous forme d'un solide blanc (Rendement = 67 %).
F = 71°C
$[\alpha]^{25}_D$ = - 20° (c = 0,37 ; $CH_3OH$)

**Exemple 122**

**N-[2-[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl](méthyl)amino]-éthyl]-N-méthyl-glycine, trifluoroacétate**

En opérant de façon analogue à l'exemple 13, au départ du composé obtenu selon la préparation XLVII, on obtient le produit attendu sous forme d'un solide jaune (Rendement = 84 %).
F = 120°C
$[\alpha]^{22}_D$ = - 29° (c = 0,49 ; $CH_3OH$)

**PREPARATION XLVIII**

**Acide [3-[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]amino]propyl] (méthyl)carbamique, 1,1-diméthyléthyl ester**

En opérant de façon analogue à l'exemple 1, au départ de (3-aminopropyl)(méthyl)carbamate de t-butyle, on obtient le produit attendu sous forme d'un solide blanc (Rendement = 69 %).
F = 75°C

$[\alpha]^{25}_D$ = - 26,5° (c = 0,35 ; $CH_3OH$)

**Exemple 123**

**1-[[2,4-dichloro-3-[[[4-(1*H*-imidazol-1-yl)-2-méthyl-8-quinolinyl]oxy]méthyl]-phényl]sulfonyl]-N-[3-(méthylamino)propyl]-2(S)-pyrrolidinecarboxamide, trifluoroacétate**

En opérant de façon analogue à l'exemple 13, au départ du composé obtenu selon la préparation XLVIII, on obtient le produit attendu sous forme d'un solide jaune (Rendement = 99 %).
F = 120°C
$[\alpha]^{24}_D$ = - 49° (c = 0,48 ; $CH_3OH$)

**PREPARATION IL**

**N-[3-[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl](méthyl)amino]-propyl]-N-méthyl-glycine, 1,1-diméthyléthyl ester**

En opérant de façon analogue à la préparation XVI, au départ du composé obtenu selon l'exemple 99, on obtient le produit attendu sous forme d'un solide blanc (Rendement = 73 %).
F = 72°C
$[\alpha]^{25}_D$ = - 12° (c = 0,45 ; $CH_3OH$)

**Exemple 124**

**N-[3-[[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl](méthyl)amino]-propyl]-N-méthyl-glycine, bis-trifluoroacétate**

En opérant de façon analogue à l'exemple 13, au départ du composé obtenu selon la préparation IL, on obtient le produit attendu sous forme d'un solide jaune (Rendement = 92 %).
F=110°C
$[\alpha]^{22}_D$ = - 34° (c = 0,34 ; $CH_3OH$)

**Exemple 125**

**1-[[2,4-dichloro-3-[[[2-méthyl-4-(1*H*-1,2,4-triazol-1-yl)-8-quinolinyl]oxy]-méthyl]phényl]sulfonyl]-N-[[1-(2-pyridinylméthyl)-4-pipéridinyl]méthyl]-2(S)-pyrrolidinecarboxamide**

On prépare un mélange de 0,5 g ($0,563.10^{-3}$ mole) du composé obtenu selon l'exemple 26 dans 10 ml d'acétonitrile. On ajoute 0,39 g ($2,81.10^{-3}$ mole) de carbonate de potassium puis 0,111 g ($0,676.10^{-3}$ mole) du chlorure de 2-picolyle (sous forme de chlorhydrate). Le mélange réactionnel est agité à 80°C pendant 45 mn puis refroidi et filtré. Les sels minéraux sont rincés par du dichlorométhane que l'on joint au filtrat. Cette solution est concentrée sous pression réduite et le résidu est purifié par chromatographie sur gel de silice en éluant à l'aide d'un mélange dichlorométhane/méthanol (9/1 ; v/v). On obtient ainsi le produit attendu sous forme d'une poudre blanche (Rendement = 71 %).
F = 118°C
$[\alpha]^{28}_D$ = - 46° (c = 0,36 ; $CHCl_3$)

**Exemple 126**

**1-[[2,4-dichloro-3-[[[2-méthyl-4-(1*H*-1,2,4-triazol-1-yl)-8-quinolinyl]oxy]-méthyl]phényl]sulfonyl]-N-[[1-(2-pyridinylméthyl)-4-pipéridinyl]méthyl]-2(S)-pyrrolidinecarboxamide, méthanesulfonate**

En opérant de façon analogue à l'exemple 6, au départ du composé obtenu selon l'exemple 125, on obtient le produit attendu sous forme d'un solide beige (Rendement = 91 %).
F = 139°C
$[\alpha]^{28}_D$ = - 76° (c = 0,59 ; $CH_3OH$)

**Exemple 127**

**1-[[2,4-dichloro-3-[[[4-(1*H*-imidazol-1-yl)-2-méthyl-8-quinolinyl]oxy]méthyl]-phényl]sulfonyl]-N-[[1-(phénylméthyl)-4-pipéridinyl]méthyl]-2(S)-pyrrolidinecarboxamide**

On prépare un mélange de 1 g ($1,12.10^{-3}$ mole) du composé obtenu selon l'exemple 9 dans 4 ml de diméthylformamide et 100 ml de dichlorométhane. On ajoute 0,787 ml ($5,64.10^{-3}$ mole) de triéthylamine, puis, après avoir refroidi le milieu à 0°C, 0,148 ml ($1,24.10^{-3}$ mole) de bromure de benzyle. Le mélange réactionnel est maintenu sous agitation à température ambiante pendant 24 heures puis concentré sous pression réduite. Le résidu est repris en solution dans l'acétate d'éthyle en présence d'eau ; le mélange est amené à pH alcalin (pH 9-10) à l'aide d'une solution de soude. La phase aqueuse est extraite à l'acétate d'éthyle et les phases organiques réunies sont lavées à l'eau, séchées sur sulfate de magnésium puis concentrées sous pression réduite. La purification du produit brut par chromatographie sur gel de silice en éluant à l'aide d'un mélange dichloro-méthane/méthanol (95/5 ; v/v) permet d'obtenir le produit attendu sous forme d'un solide fin blanc (Rendement = 56%).
F = 123°C
$[\alpha]^{28}_D$ = - 43° (c = 0,73 ; $CH_3OH$)

**Exemple 128**

**1-[[2,4-dichloro-3-[[[4-(1*H*-imidazol-1-yl)-2-méthyl-8-quinolinyl]oxy]méthyl]-phényl]sulfonyl]-N-[[1-(phénylméthyl)-4-pipéridinyl]méthyl]-2(S)-pyrrolidinecarboxamide, méthanesulfonate**

En opérant de façon analogue à l'exemple 6, au départ du composé obtenu selon l'exemple 127, on obtient le produit attendu sous forme d'un solide blanc (Rendement = 95 %).
F = 161°C
$[\alpha]^{26}_D$ = - 44° (c = 0,63 ; $CH_3OH$)

**Exemple 129**

**N-[(1-benzoyl-4-pipéridinyl)méthyl]-1-[[2,4-dichloro-3-[[[4-(1*H*-imidazol-1-yl)-2-méthyl-8-quinolinyl]oxy]méthyl]phényl]sulfonyl]-2(S)-pyrrolidine carboxamide**

En opérant de façon analogue à l'exemple 127, au départ de chlorure de benzoyle, on obtient le produit attendu sous forme d'un solide blanc (Rendement = 67 %).
F =102°C
$[\alpha]^{28}_D$ = - 48° (c = 0,66 ; $CHCl_3$)

**Exemple 130**

**N-[(1-benzoyl-4-pipéridinyl)méthyl]-1-[[2,4-dichloro-3-[[[4-(1*H*-imidazol-1-yl)-2-méthyl-8-quinolinyl]oxy]méthyl]phényl]sulfonyl]-2(S)-pyrrolidine carboxamide, méthanesulfonate**

En opérant de façon analogue à l'exemple 6, au départ du composé obtenu selon l'exemple 129, on obtient le produit attendu sous forme d'un solide jaune pâle (Rendement = 95 %).
F=153°C
$[\alpha]^{26}_D$ = -24° (c = 0,55 ; $CH_3OH$)

**Exemple 131**

**1-[[2,4-dichloro-3-[[[4-(1*H*-imidazol-1-yl)-2-méthyl-8-quinolinyl]oxy]méthyl]-phényl]sulfonyl]-N-[[1-(4-pyridinylcarbonyl)-4-pipéridinyl]méthyl]-2(S)-pyrrolidinecarboxamide**

En opérant de façon analogue à l'exemple 127, au départ du chlorhydrate du chlorure d'isonicotinoyle, on obtient le produit attendu sous forme d'un solide fin blanc (Rendement = 34 %).
F=134°C
$[\alpha]^{28}_D$ = - 56° (c = 0,64 ; $CHCl_3$)

**Exemple 132**

**1-[[2,4-dichloro-3-[[[4-(1*H*-imidazol-1-yl)-2-méthyl-8-quinolinyl]oxy]méthyl]-phényl]sulfonyl]-N-[[1-(4-pyridinylcarbonyl)-4-pipéridinyl]méthyl]-2(S)-pyrrolidinecarboxamide, méthanesulfonate**

En opérant de façon analogue à l'exemple 6, au départ du composé obtenu selon l'exemple 131, on obtient le produit attendu sous forme d'un solide blanc (Rendement = 90 %).
F = 165°C
$[\alpha]^{26}_D$ = - 29° (c = 0,48 ; $CH_3OH$)

**Exemple 133**

**1-[[2,4-dichloro-3-[[[4-(1*H*-imidazol-1-yl)-2-méthyl-8-quinolinyl]oxy]méthyl]-phényl]sulfonyl]-N-[[1-(3-pyridinylcarbonyl)-4-pipéridinyl]méthyl]-2(S)-pyrrolidinecarboxamide**

En opérant de façon analogue à l'exemple 127, au départ du chlorure de nicotinoyle, on obtient le produit attendu sous forme d'un solide fin blanc (Rendement = 79%).
F = 109°C
$[\alpha]^{24}_D$ = - 45° (c = 0,88 ; $CHCl_3$)

**Exemple 134**

**1-[[2,4-dichloro-3-[[[4-(1*H*-imidazol-1-yl)-2-méthyl-8-quinolinyl]oxy]méthyl]-phényl]sulfonyl]-N-[[1-(3-pyridinylcarbonyl)-4-pipéridinyl]méthyl]-2(S)-pyrrolidinecarboxamide, méthanesulfonate**

En opérant de façon analogue à l'exemple 6, au départ du composé obtenu selon l'exemple 133, on obtient le produit attendu sous forme d'un solide blanc (Rendement = 82%).
F = 150°C
$[\alpha]^{24}_D$ = - 38° (c = 0,59 ; $CH_3OH$)

**Exemple 135**

**1-[[2,4-dichloro-3-[[[4-(1*H*-imidazol-1-yl)-2-méthyl-8-quinolinyl]oxy]méthyl]-phényl]sulfonyl]-N-[[1-(2-pyridinylcarbonyl)-4-pipéridinyl]méthyl]-2(S)-pyrrolidinecarboxamide**

En opérant de façon analogue à l'exemple 1, au départ d'acide picolinique, on obtient le produit attendu sous forme d'un solide blanc (Rendement = 52 %).
F = 103°C
$[\alpha]^{24}_D$ = - 58° (c = 0,90 ; $CHCl_3$)

**Exemple 136**

**1-[[2,4-dichloro-3-[[[4-(1*H*-imidazol-1-yl)-2-méthyl-8-quinolinyl]oxy]méthyl]-phényl]sulfonyl]-N-[[1-(2-pyridinylcarbonyl)-4-pipéridinyl]méthyl]-2(S)-pyrrolidinecarboxamide, méthanesulfonate**

En opérant de façon analogue à l'exemple 6, au départ du composé obtenu selon l'exemple 135, on obtient le produit attendu sous forme d'un solide jaune pâle (Rendement = 89 %).
F = 152°C
$[\alpha]^{24}_D$ = - 25° (c = 0,76 ; $CH_3OH$)

**PREPARATION L**

**1-[[2,4-dichloro-3-[[[2-méthyl-4-(1*H*-1,2,4-triazol-1-yl)-2-méthyl-8-quinolinyl]oxy]méthyl]phényl]sulfonyl]-L-proline, méthyl ester**

En opérant de façon analogue à la préparation I, au départ de 8-hydroxy-2-méthyl-4-(1*H*-1,2,4-triazol-1-yl) quinoléine, on obtient le produit attendu sous forme d'un solide jaune pâle (Rendement = 98%).
F = 130°C

$[\alpha]^{22}_D$ = - 35° (c = 0,68 ; $CHCl_3$)

**PREPARATION LI**

**1-[[2,4-dichloro-3-[[[2-méthyl-4-(1*H*-1,2,4-triazol-1-yl)-2-méthyl-8-quinolinyl]oxy]méthyl]phényl]sulfonyl]-L-proline**

En opérant de façon analogue à la préparation II, au départ du composé obtenu selon la préparation L, on obtient le produit attendu sous forme d'un solide jaune pâle (Rendement = 72 %).
F = 146°C
$[\alpha]^{24}_D$ = - 5° (c = 0,68 ; $CH_3OH$)

**Exemple 137**

**1-[[2,4-dichloro-3-[[[2-méthyl-4-(1*H*-1,2,4-triazol-1-yl)-2-méthyl-8-quinolinyl]oxy]méthyl]phényl]sulfonyl] -N-[3-(diméthylamino)propyl]-2(S)-pyrrolidinecarboxamide**

En opérant de façon analogue à l'exemple 1, au départ de l'acide obtenu selon la préparation LI et de N-N-diméthylpropanediamine, on obtient le produit attendu sous forme d'un solide amorphe beige (Rendement = 74 %).
F = 105°C
$[\alpha]^{24}_D$ = - 51° (c = 0,75 ; $CHCl_3$)

**Exemple 138**

**1-[[2,4-dichloro-3-[[[2-méthyl-4-(1*H*-1,2,4-triazol-1-yl)-2-méthyl-8-quinolinyl]oxy]méthyl]phényl]sulfonyl] -N-[3-(diméthylamino)propyl]-2(S)-pyrrolidinecarboxamide, hémi-sulfate**

En opérant de façon analogue à l'exemple 44, au départ du composé obtenu selon l'exemple 137, on obtient le produit attendu sous forme d'un solide jaune pâle (Rendement = 98 %).
F=154°C
$[\alpha]^{23}_D$ = - 26° (c = 0,77 ; $CH_3OH$)

**Exemple 139**

**1-[[2,4-dichloro-3-[[[2-méthyl-4-(1*H*-1,2,4-triazol-1-yl)-2-méthyl-8-quinolinyl]oxy]méthyl]phényl]sulfonyl]-N-méthyl-2(S)-pyrrolidinecarboxamide**

En opérant de façon analogue à l'exemple 137, au départ du chlorhydrate de méthylamine, on obtient le produit attendu sous forme d'un solide blanc (Rendement = 60 %).
F=131°C
$[\alpha]^{28}_D$ = - 37° (c = 0,94 ; $CHCl_3$)

**Exemple 140**

**1-[[2,4-dichloro-3-[[[2-méthyl-4-(1*H*-1,2,4-triazol-1-yl)-2-méthyl-8-quinolinyl]oxy]méthyl]phényl]sulfonyl] -N-[(2-pyridinyl)méthyl]-2(S)-pyrrolidinecarboxamide**

En opérant de façon analogue à l'exemple 137, au départ de 2(aminométhyl)pyridine, on obtient le produit attendu sous forme d'un solide fin blanc (Rendement = 85 %).
F = 95°C
$[\alpha]^{28}_D$ = - 31° (c = 0,53 ; $CHCl_3$)

**Exemple 141**

**1-[[2,4-dichloro-3-[[[2-méthyl-4-(1*H*-1,2,4-triazol-1-yl)-2-méthyl-8-quinolinyl]oxy]méthyl]phényl]sulfonyl] -N-[(2-pyridinyl)méthyl]-2(S)-pyrrolidinecarboxamide, méthanesulfonate**

En opérant de façon analogue à l'exemple 4, au départ du composé obtenu selon l'exemple 140, on obtient

le produit attendu sous forme d'un solide jaune pâle (Rendement = 85 %).
F = 108°C
$[\alpha]^{28}_D$ = - 33° (c = 0,52 ; $CH_3OH$)

**Exemple 142**

**8-[[2,6-dichloro-3-[[2(S)-[[4-(2-pyridinyl)-1-pipérazinyl]carbonyl]-1-pyrrolidinyl]sulfonyl]phényl]méthoxy]-2-méthyl-4-(1*H*-1,2,4-triazol-1-yl)quinoléine**

En opérant de façon analogue à l'exemple 137, au départ de 1-(2-pyridyl)pipérazine, on obtient le produit attendu sous forme d'une poudre blanche (Rendement = 75 %).
F=108°C
$[\alpha]^{26}_D$ = + 9° (c = 0,47 ; $CHCl_3$)

**Exemple 143**

**8-[[2,6-dichloro-3-[[2(S)-[[4-(2-pyridinyl)-1-pipérazinyl]carbonyl]-1-pyrrolidinyl]sulfonyl]phényl]méthoxy]-2-méthyl-4-(1*H*-1,2,4-triazol-1-yl)quinoléine, méthanesulfonate**

En opérant de façon analogue à l'exemple 6, au départ du composé obtenu selon l'exemple 142, on obtient le produit attendu sous forme d'un solide jaune (Rendement = 88 %).
F=160°C
$[\alpha]^{26}_D$ = - 12° (c = 0,65 ; $CH_3OH$)

**Exemple 144**

**8-[[2,6-dichloro-3-[[2(S)-(4-morpholinylcarbonyl)-1-pyrrolidinylsulfonyl]-phényl]méthoxy]-4-(1*H*-imidazol-1-yl)-2-méthyl-quinoléïne**

En opérant de façon analogue à l'exemple 1, au départ de morpholine, on obtient le produit attendu sous forme d'un solide blanc (Rendement = 76 %).
F = 50°C
$[\alpha]^{27}_D$ = + 15° (c = 0,54 ; $CHCl_3$)

**Exemple 145**

**8-[[2,6-dichloro-3-[[2(S)-(4-morpholinylcarbonyl)-1-pyrrolidinyl]sulfonyl]-phényl]méthoxy]-4-(1*H*-imidazol-1-yl)-2-méthyl-quinoléïne, tartrate**

En opérant de façon analogue à l'exemple 2, au départ du composé obtenu selon l'exemple 144, on obtient le produit attendu sous forme d'un solide jaune pâle (Rendement = 95 %).
F = 138°C
$[\alpha]^{27}_D$ = - 10° (c = 0,72 ; $CH_3OH$)

**Exemple 146**

**8-[[2,6-dichloro-3-[[2(S)-[(4-méthyl-1-pipérazinyl)carbonyl]-1-pyrrolidinyl]-sulfonyl]phényl]méthoxy]-4-(1*H*-imidazol-1-yl)-2-méthyl-quinoléïne**

En opérant de façon analogue à l'exemple 1, au départ de 1-méthylpipérazine, on obtient le produit attendu sous forme d'une huile incolore (Rendement = 34 %).
$[\alpha]^{27}_D$ = + 11° (c = 0,62 ; $CHCl_3$)
RMN $^1H$ (300 MHz ;DMSOd6) 8.13 (t, J=8.6Hz, 1H); 8.09 (s, 1H); 7.80 (d, J=8.6Hz, 1H) ; 7.67 (s, 1H) ; 7.6-7.50 (m, 3H) ; 7.35-7.20 (m, 2H) ; 5.56 (s, 2H) ; 5.0-4.95 (m, 1H) ; 3.6-3.3 (m, 6H) ; 2.67 (s, 3H) ; 2.3-2.1 (m, 5H) ; 2.16 (s, 3H) ; 2.0-1.80 (m, 3H).

**Exemple 147**

**8-[[2,6-dichloro-3-[[2(S)-[(4-méthyl-1-pipérazinyl)carbonyl]-1-pyrrolidinyl]-sulfonyl]phényl]méthoxy]-4-(1*H*-imidazol-1-yl)-2-méthyl-quinoléïne, tartrate**

En opérant de façon analogue à l'exemple 2 au départ du composé obtenu selon l'exemple 146, on obtient le produit attendu sous forme d'un solide jaune pâle (Rendement = 94 %).
F = 138°C
$[\alpha]^{27}_D$ = - 13° (c = 0,60 ; $CH_3OH$)

**Exemple 148**

**8-[[2,6-dichloro-3-[[2(S)-[(4-phényl-1-pipérazinyl)carbonyl]-1-pyrrolidinyl]-sulfonyl]phényl]méthoxy]-4-(1*H*-imidazol-1-yl)-2-méthyl-quinoléïne**

En opérant de façon analogue à l'exemple 1, au départ de 1-phénylpipérazine, on obtient le produit attendu sous forme d'un solide blanc (Rendement = 77 %).
F = 88°C
$[\alpha]^{28}_D$ = + 15° (c = 0,58 ; $CHCl_3$)

**Exemple 149**

**8-[[2,6-dichloro-3-[[2(S)-[(4-phényl-1-pipérazinyl)carbonyl]-1-pyrrolidinyl]-sulfonyl]phényl]méthoxy]-4-(1*H*-imidazol-1-yl)-2-méthyl-quinoléïne, méthanesulfonate**

En opérant de façon analogue à l'exemple 2, au départ du composé obtenu selon l'exemple 148, on obtient le produit attendu sous forme d'un solide jaune pâle (Rendement = 93 %).
F=147°C
$[\alpha]^{28}_D$ = - 3° (c = 0,50 ; $CH_3OH$)

**Exemple 150**

**1-[[2,4-dichloro-3-[[[4-(1*H*-imidazol-1-yl)-2-méthyl-8-quinolinyl]oxy]méthyl]-phényl]sulfonyl]-N-[2-(2-pyridinyl) éthyl]-2(S)-pyrrolidinecarboxamide**

En opérant de façon analogue à l'exemple 1, au départ de 2-(2-pyridyl)éthylamine, on obtient le produit attendu sous forme d'un solide blanc (Rendement = 87%).
F = 82°C
$[\alpha]^{28}_D$ = - 29° (c = 1,13 ; $CHCl_3$)

**Exemple 151**

**1-[[2,4-dichloro-3-[[[4-(1*H*-imidazol-1-yl)-2-méthyl-8-quinolinyl]oxy]méthyl]-phényl]sulfonyl]-N-[2-(2-pyridinyl) éthyl]-2(S)-pyrrolidinecarboxamide, méthanesulfonate**

En opérant de façon analogue à l'exemple 6, au départ du composé obtenu selon l'exemple 150, on obtient le produit attendu sous forme d'un solide jaune pâle (Rendement = 85%).
F = 110°C
$[\alpha]^{26}_D$ = - 31° (c = 0,61 ; $CH_3OH$)

**Exemple 152**

**1-[[2,4-dichloro-3-[[[4-(1*H*-imidazol-1-yl)-2-méthyl-8-quinolinyl]oxy]méthyl]-phényl]sulfonyl]-N-[2-(3-pyridinyl) éthyl]-2(S)-pyrrolidinecarboxamide**

En opérant de façon analogue à l'exemple 1, au départ de 2-(3-pyridinyl)éthylamine, on obtient le produit attendu sous forme d'un solide blanc (Rendement = 87%).
F = 117°C

$[\alpha]^{29}_D$ = - 41° (c = 0,59 ; $CHCl_3$)

**Exemple 153**

**1-[[2,4-dichloro-3-[[[4-(1*H*-imidazol-1-yl)-2-méthyl-8-quinolinyl]oxy]méthyl]-phényl]sulfonyl]-N-[2-(3-pyridinyl) éthyl]-2(S)-pyrrolidinecarboxamide, méthanesulfonate**

En opérant de façon analogue à l'exemple 6, au départ du composé obtenu selon l'exemple 152, on obtient le produit attendu sous forme d'un solide jaune pâle (Rendement = 92 %).
F = 128°C
$[\alpha]^{29}_D$ =- 23° (c = 0,74 ; $CH_3OH$)

**Exemple 154**

**1-[[2,4-dichloro-3-[[[4-(1*H*-imidazol-1-yl)-2-méthyl-8-quinolinyl]oxy]méthyl]-phényl]sulfonyl]-N-[2-(4-pyridinyl) éthyl]-2(S)-pyrrolidinecarboxamide**

En opérant de façon analogue à l'exemple 1, au départ de 2-(4-pyridinyl)éthylamine, on obtient le produit attendu sous forme d'un solide beige (Rendement = 94%).
F = 120°C
$[\alpha]^{27}_D$ = - 45° (c = 0,56 ; $CHCl_3$)

**Exemple 155**

**1-[[2,4-dichloro-3-[[[4-(1*H*-imidazol-1-yl)-2-méthyl-8-quinolinyl]oxy]méthyl]-phényl]sulfonyl]-N-[2-(4-pyridinyl) éthyl]-2(S)-pyrrolidinecarboxamide, méthanesulfonate**

En opérant de façon analogue à l'exemple 6, au départ du composé obtenu selon l'exemple 154, on obtient le produit attendu sous forme d'un solide blanc (Rendement = 93%).
F = 136°C
$[\alpha]^{27}_D$ = - 18° (c = 0,76 ; $CH_3OH$)

**Exemple 156**

**1-[[2,4-dichloro-3-[[[4-(1*H*-imidazol-1-yl)-2-méthyl-8-quinolinyl]oxy]méthyl]-phényl]sulfonyl]-N-(phénylméthyl)-2(S)-pyrrolidinecarboxamide**

En opérant de façon analogue à l'exemple 1, au départ de benzylamine, on obtient le produit attendu sous forme d'un solide blanc (Rendement = 97%).
F=116°C
$[\alpha]^{27}_D$ = - 31° (c = 0,77 ; $CHCl_3$)

**Exemple 157**

**1-[[2,4-dichloro-3-[[[4-(1*H*-imidazol-1-yl)-2-méthyl-8-quinolinyl]oxy]méthyl]-phényl]sulfonyl]-N-(phénylméthyl)-2(S)-pyrrolidinecarboxamide, méthanesulfonate**

En opérant de façon analogue à l'exemple 6, au départ du composé obtenu selon l'exemple 156, on obtient le produit attendu sous forme d'un solide jaune pâle (Rendement = 91%).
F = 135°C
$[\alpha]^{27}_D$ = - 103° (c = 0,83 ; $CH_3OH$)

**PREPARATION LII**

**Acide 4-[[1-[[2,4-dichloro-3-[[[4-(1*H*-imidazol-1-yl)-2-méthyl-8-quinolinyl]-oxy]méthyl]phényl]sulfonyl]-2(S)-pyrrolidinyl]carbonyl]-1-pipérazinecarboxylique, 1,1-diméthyléthyl ester**

En opérant de façon analogue à l'exemple 1, au départ de l'ester t-butylique de l'acide 1-pipérazine carboxylique (N-boc-pipérazine), on obtient le produit attendu sous forme d'un solide blanc (Rendement = 33%).
F = 98°C
$[\alpha]^{20}_D$ = + 4° (c = 0,79 ; $CHCl_3$)

**Exemple 158**

**8-[[2,6-dichloro-3-[[2(S)-(1-pipérazinylcarbonyl)-1-pyrrolidinyl]sulfonyl]-phényl]méthoxy]-4-(1*H*-imidazol-1-yl)-2-méthyl-quinoléïne, bis trifluoroacétate**

En opérant de façon analogue à l'exemple 13, au départ du composé obtenu selon la préparation LII, on obtient le produit attendu sous forme d'un solide jaune pâle (Rendement = 99 %).
F = 143°C
$[\alpha]^{19}_D$ = + 22° (c = 0,47 ; $CH_3OH$)

**Exemple 159**

**8-[[2,6-dichloro-3-[[2(S)-[[4-(2-pyridinylméthyl)-1-pipérazinyl]carbonyl]-1-pyrrolidinyl]sulfonyl]phényl]méthoxy]-4-(1*H*-imidazol-1-yl)-2-méthylquinoléïne**

En opérant de façon analogue à l'exemple 127, au départ du composé obtenu selon l'exemple 158 et de chlorure de 2-picolyle, on obtient le produit attendu sous forme d'une huile jaune (Rendement = 54 %).
$[\alpha]^{20}_D$ = + 11° (c = 0,54 ; $CHCl_3$)
RMN $^1H$(250 MHz ; DMSOd6)
8.5-8.45 (m, 1H) ; 8.2-8.05 (m, 2H) ; 7.85-7.70 (m, 2H) ; 7.68-7.64 (m, 1H) ; 7.6-7.5 (m, 3H) ; 7.42 (d, J=7.8 Hz, 1H) ; 7.35-7.20 (m, 3H) ; 5.56 (s, 2H) ; 5-4.95 (m, 1H) ; 3.60 (s, 2H) ; 3.55-3.30 (m, 6H) ; 2.66 (s, 3H) ; 2.45-2.15 (m, 5H) ; 2-1.8 (m, 3H).

**Exemple 160**

**8-[[2,6-dichloro-3-[[2(S)-[[4-(2-pyridinylméthyl)-1-pipérazinyl]carbonyl]-1-pyrrolidinyl]sulfonyl]phényl]méthoxy]-4-(1*H*-imidazol-1-yl)-2-méthylquinoléïne, méthanesulfonate**

En opérant de façon analogue à l'exemple 6, au départ du composé obtenu selon l'exemple 159, on obtient le produit attendu sous forme d'un solide jaune pâle (Rendement = 91 %).
F = 143°C
$[\alpha]^{26}_D$ = - 12° (c = 0,56 ; $CH_3OH$)

**Exemple 161**

**8-[[2,6-dichloro-3-[[2(S)-[[4-(3-pyridinylméthyl)-1-pipérazinyl]carbonyl]-1-pyrrolidinyl]sulfonyl]phényl]méthoxy]-4-(1*H*-imidazol-1-yl)-2-méthylquinoléine**

En opérant de façon analogue à l'exemple 159, au départ de chlorure de 3-picolyle, on obtient le produit attendu sous forme d'un solide blanc (Rendement = 26 %).
F = 102°C
$[\alpha]^{22}_D$ = + 12° (c = 0,40 ; $CHCl_3$)

**Exemple 162**

**8-[[2,6-dichloro-3-[[2(S)-[[4-(3-pyridinylméthyl)-1-pipérazinyl]carbonyl]-1-pyrrolidinyl]sulfonyl]phényl]méthoxy]-4-(1*H*-imidazol-1-yl)-2-méthylquinoléine, méthanesulfonate**

En opérant de façon analogue à l'exemple 6, au départ du composé obtenu selon l'exemple 161, on obtient le produit attendu sous forme d'un solide jaune pâle (Rendement = 95 %).
F=154°C
$[\alpha]^{26}_D$ = - 8° (c = 0,72 ; $CH_3OH$)

**Exemple 163**

**8-[[2,6-dichloro-3-[[2(S)-[[4-(4-pyridinylméthyl)-1-pipérazinyl]carbonyl]-1-pyrrolidinyl]sulfonyl]phényl]méthoxy]-4-(1*H*-imidazol-1-yl)-2-méthylquinoléïne**

En opérant de façon analogue à l'exemple 159, au départ de chlorure de 4-picolyle, on obtient le produit attendu sous forme d'un solide fin beige (Rendement = 52 %).
F = 108°C
$[\alpha]^{22}_D$ = + 12° (c = 0,40 ; $CHCl_3$)

**Exemple 164**

**8-[[2,6-dichloro-3-[[2(S)-[[4-(4-pyridinylméthyl)-1-pipérazinyl]carbonyl]-1-pyrrolidinyl]sulfonyl]phényl]méthoxy]-4-(1*H*-imidazol-1-yl)-2-méthylquinoléïne, méthanesulfonate**

En opérant de façon analogue à l'exemple 6, au départ du composé obtenu selon l'exemple 163, on obtient le produit attendu sous forme d'un solide jaune (Rendement = 97 %).
F = 156°C
$[\alpha]^{23}_D$ = - 14° (c = 0,77 ; $CH_3OH$)

**PREPARATION LIII**

**1-[[2,4-dichloro-3-[[[2-méthyl-4-(1*H*-1,2,4-triazol-1-yl)-2-méthyl-8-quinolinyl]-oxy]méthyl]phényl]sulfonyl] -N-[[1-[2-(acétoxy)éthyl]-4-pipéridinyl]**-méthyl]-2(S)-pyrrolidinecarboxamide

En opérant de façon analogue à la préparation XIII, au départ du composé obtenu selon l'exemple 26 et d'acétate de 2-bromoéthyle, on obtient le produit attendu sous forme d'une huile incolore (Rendement = 31%).
$[\alpha]^{29}_D$ = - 47° (c = 0,55 ; $CHCl_3$)
RMN $^1$H (250 MHz ; DMSOd6)
9.18 (s, 1H); 8.44 (s, 1H); 8.10 (d, J=8.6 Hz, 1H); 7.93 (t, J=5.4 Hz, NH); 7.81 (d, J=8.7Hz, 1H); 7.73 (s, 1H); 7.6-7.5 (m, 3H); 5.58 (s, 2H) ; 4.4-4.3 (m, 1H); 4.06 (t, J=6 Hz, 2H); 3.6-3.5 (m, 1H); 3.45-3.30 (m, 1H); 2.95-2.75 (m, 4H) ; 2.69 (s, 3H) ; 2.50-2.45 (m, 2H) ; 2.25-1.75 (6H) ; 1.99 (s, 3H) ; 1.55-1.45 (m, 2H) ; 1.35-1.20 (m, 1H); 1.15-0.95 (m, 2H).

**Exemple 165**

**1-[[2,4-dichloro-3-[[[2-méthyl-4-(1*H*-1,2,4-triazol-1-yl)-2-méthyl-8-quinolinyl]oxy]méthyl]phényl]sulfonyl] -N-[[1-[(2-hydroxyéthyl)-4-pipéridinyl]-méthyl]-2(S)-pyrrolidinecarboxamide**

En opérant de façon analogue à l'exemple 11, au départ du composé obtenu selon la préparation LIII, on obtient le produit attendu sous forme d'un solide blanc (Rendement = 90 %).
F = 115°C
$[\alpha]^{29}_D$ = - 43° (c = 0,58 ; $CHCl_3$)

**Exemple 166**

**1-[[2,4-dichloro-3-[[[2-méthyl-4-(1*H*-1,2,4-triazol-1-yl)-2-méthyl-8-quinolinyl]oxy]méthyl]phényl]sulfonyl] -N-[[1-(2-(hydroxyéthyl)-4-pipéridinyl]-méthyl]-2(S)-pyrrolidinecarboxamide, méthanesulfonate**

En opérant de façon analogue à l'exemple 6, au départ du composé obtenu selon l'exemple 165, on obtient le produit attendu sous forme d'un solide blanc (Rendement = 82 %).
F=137°C
$[\alpha]^{26}_D$ = - 60° (c =0,14 ; $CH_3OH$)

**Exemple 167**

**1-[[2,4-dichloro-3-[[[4-(1*H*-imidazol-1-yl)-2-méthyl-8-quinolinyl]oxy]méthyl]-phényl]sulfonyl]-N-[5-(diméthylamino)pentyl]-2(S)-pyrrolidinecarboxamide**

En opérant de façon analogue à l'exemple 1, au départ de N,N-diméthyl-1,5-pentanediamine, on obtient le produit attendu sous forme d'un solide blanc (Rendement = 53 %).
F = 85°C
$[\alpha]^{28}_D$ = - 31° (c = 0,37 ; $CH_3OH$)

**Exemple 168**

**1-[[2,4-dichloro-3-[[[4-(1*H*-imidazol-1-yl)-2-méthyl-8-quinolinyl]oxy]méthyl]-phényl]sulfonyl]-N-[5-(diméthylamino)pentyl]-2(S)-pyrrolidinecarboxamide, tartrate**

En opérant de façon analogue à l'exemple 2, au départ du composé obtenu selon l'exemple 167, on obtient le produit attendu sous forme d'un solide blanc (Rendement = 97 %).
F = 126°C
$[\alpha]^{28}_D$ = - 31,6° (c = 0,38 ; $CH_3OH$)

**PREPARATION LIV**

**1-[[3-(bromométhyl)-2,4-dichlorophényl]sulfonyl]-3(R)-pyrrolidinol**

En opérant de façon analogue à la préparation VII, au départ de 3(R)-pyrrolidinol, on obtient le produit attendu sous forme de cristaux blancs (Rendement = 63 %).
F = 121°C
$[\alpha]^{25}_D$ = + 7,9° (c = 0,51; $CH_3OH$)

**Exemple 169**

**1-[[2,4-dichloro-3-[[[4-(1*H*-imidazol-1-yl)-2-méthyl-8-quinolinyl]oxy]méthyl]-phényl]sulfonyl]-3(R)-pyrrolidinol**

En opérant de façon analogue à la préparation I, au départ du composé obtenu selon la préparation LIV, on obtient le produit attendu sous forme de cristaux blancs (Rendement = 28 %).
F=166°C
$[\alpha]^{25}_D$ = - 2,1° (c = 0,66 ; $CH_3OH$)

**Exemple 170**

**1-[[2,4-dichloro-3-[[[4-(1*H*-imidazol-1-yl)-2-méthyl-8-quinolinyl]oxy]méthyl]-phényl]sulfonyl]-3(R)-pyrrolidinol, tartrate**

En opérant de façon analogue à l'exemple 2, au départ du composé obtenu selon l'exemple 169, on obtient le produit attendu sous forme d'un solide fin jaune pâle (Rendement = 97 %).
F = 162°C
$[\alpha]^{25}_D$ = + 1,65° (c = 0,59 ; $CH_3OH$)

**PREPARATION LV**

**1-[[3-(bromométhyl)-2,4-dichlorophényl]sulfonyl]-3(S)-pyrrolidinol**

En opérant de façon analogue à la préparation VII, au départ de 3(S)-pyrrolidinol, on obtient le produit attendu sous forme de cristaux blancs (Rendement = 49 %).
F = 120°C
$[\alpha]^{25}_D$ = - 6° (c = 0,61 ; $CH_3OH$)

**Exemple 171**

**1-[[2,4-dichloro-3-[[[4-(1*H*-imidazol-1-yl)-2-méthyl-8-quinolinyl]oxy]méthyl]-phényl]sulfonyl]-3(S)-pyrrolidinol**

En opérant de façon analogue à la préparation I, au départ du composé obtenu selon la préparation LV, on obtient le produit attendu sous forme de cristaux blancs (Rendement = 31 %).
F = 166°C
$[\alpha]^{25}_D$ = + 2,3° (c = 0,54 ; $CH_3OH$)

**Exemple 172**

**1-[[2,4-dichloro-3-[[[4-(1*H*-imidazol-1-yl)-2-méthyl-8-quinolinyl]oxy]méthyl]-phényl]sulfonyl]-3(S)-pyrrolidinol, tartrate**

En opérant de façon analogue à l'exemple 2, au départ du composé obtenu selon l'exemple 171, on obtient le produit attendu sous forme d'un solide fin beige (Rendement = 99 %).
F = 163°C
$[\alpha]^{25}_D$ = + 3,45° (c = 0,67 ; $CH_3OH$)

**PREPARATION LVI**

**N-[1-[[3-(bromométhyl)-2,4-dichlorophényl]sulfonyl]-3(R)-pyrrolidinyl]-acétamide**

En opérant de façon analogue à la préparation VII, au départ de N-[3(R)-pyrrolidinyl]acétamide, on obtient le produit attendu sous forme d'un solide blanc (Rendement = 81 %).
F = 222°C
$[\alpha]^{25}_D$ = - 1,3° (c = 1,12; $CHCl_3$)

**Exemple 173**

**N-[1-[[2,4-dichloro-3-[[[4-(1*H*-imidazol-1-yl)-2-méthyl-8-quinolinyl]oxy]-méthyl]phényl]sulfonyl]-3(R)-pyrrolidinyl]acétamide**

En opérant de façon analogue à la préparation I, au départ du composé obtenu selon la préparation LVI, on obtient le produit attendu sous forme d'un solide blanc (Rendement = 69 %).
F = 246°C
$[\alpha]^{25}_D$ = + 26,2° (c = 0,80 ; $CH_3OH$)

**PREPARATION LVII**

**N-[1-[[3-(bromométhyl)-2,4-dichlorophényl]sulfonyl]-3(S)-pyrrolidinyl]-acétamide**

En opérant de façon analogue à la préparation VIII, au départ de N-[3(S)-pyrrolidinyl]acétamide, on obtient le produit attendu sous forme d'un solide blanc (Rendement = 86 %).
F=221°C
$[\alpha]^{25}_D$ = + 1,7° (c = 0,98; $CHCl_3$)

**Exemple 174**

**N-[1-[[2,4-dichloro-3-[[[4-(1*H*-imidazol-1-yl)-2-méthyl-8-quinolinyl]oxy]-méthyl]phényl]sulfonyl]-3(S)-pyrrolidinyl]acétamide**

En opérant de façon analogue à la préparation I, au départ du composé obtenu selon la préparation LVII, on obtient le produit attendu sous forme d'un solide blanc (Rendement = 69 %).
F = 246°C
$[\alpha]^{25}_D$ = - 26,6° (c = 1,2; $CH_3OH$)

**PREPARATION LVIII**

**N-[3-[[[1-[[2,4-dichloro-3-[[[4-(1*H*-imidazol-1-yl)-2-méthyl-8-quinolinyl]oxy]-méthyl]phényl]sulfonyl]-2(S)-pyrrolidinyl]carbonyl]amino]propyl]-N-méthylglycine, 1-1diméthyléthyl ester**

En opérant de façon analogue à la préparation XVI, au départ du composé obtenu selon l'exemple 123, on obtient le produit attendu sous forme d'un solide blanc (Rendement = 67 %).
F = 74°C
$[\alpha]^{24}_D$ = - 33° (c = 0,36; $CH_3OH$)

**Exemple 175**

**N-[3-[[[1-[[2,4-dichloro-3-[[[4-(1*H*-imidazol-1-yl)-2-méthyl-8-quinolinyl]oxy]-méthyl]phényl]sulfonyl]-2(S)-pyrrolidinyl]carbonyl]amino]propyl]-N-méthylglycine, bis trifluoroacétate**

En opérant de façon analogue à l'exemple 13, au départ du composé obtenu selon la préparation LVIII, on obtient le produit attendu sous forme d'une poudre jaune (Rendement = 78 %).
F = 115°C
$[\alpha]^{25}_D$ = - 31° (c = 0,40; $CH_3OH$)

**PREPARATION LIX**

**Acide 1[[3-(bromométhyl)-2,4-dichlorophényl]sulfonyl]-2(S)-pipéridinecarboxylique, méthyl ester**

On prépare une solution de 0,68 g ($3,78.10^{-3}$ mole) du chlorhydrate de l'ester méthylique de l'acide 2(S)-pipéridinecarboxylique dans 30 ml d'acétonitrile et on ajoute 1,14 g ($11,4.10^{-3}$ mole) de bicarbonate de potassium en solution dans 10 ml d'eau, puis 1,28 g ($3,78.10^{-3}$ mole) de chlorure de 3-(bromométhyl)-2,4-dichlorobenzènesulfonyle. Le mélange réactionnel est maintenu sous agitation pendant 20 heures à température ambiante puis concentré sous pression réduite. Le résidu est repris par du dichlorométhane et cette phase organique est lavée à l'eau, séchée sur sulfate de magnésium et concentrée sous pression réduite. Le produit brut est purifié par chromatographie sur gel de silice en éluant à l'aide d'un mélange toluène/acétate d'éthyle (95/5 ;v/v). On obtient ainsi 1,02 g du produit attendu sous forme d'un solide blanc (Rendement = 61 %).
F = 91°C
$[\alpha]^{25}_D$ = + 4° (c = 0,56; $CH_3OH$)
Note : le produit attendu contient une proportion d'analogue chlorométhylé en position 3 qui peut réagir comme le produit attendu lors de l'étape suivante et n'a pas été séparé.

**PREPARATION LX**

**Acide 1[[2,4-dichloro-3-[[[4-(1H-imidazol-1-yl)-2-méthyl-8-quinolinyl]oxy]-méthyl]phényl]sulfonyl]-2(S)-pipéridinecarboxylique, méthyl ester**

En opérant de façon analogue à la préparation I, au départ du composé obtenu selon la préparation LIX, on obtient le produit attendu sous forme d'un solide blanc écru (Rendement = 72 %).
F = 81°C
$[\alpha]^{25}_D$ = + 13° (c = 0,380; $CH_3OH$)

**PREPARATION LXI**

**Acide 1-[[2,4-dichloro-3-[[[4-(1*H*-imidazol-1-yl)-2-méthyl-8-quinolinyl]-oxy]méthyl]phényl]sulfonyl]-2(S)-pipéridinecarboxylique**

En opérant de façon analogue à la préparation II, au départ du composé obtenu selon la préparation LX, on obtient le produit attendu sous forme d'un solide blanc (Rendement = 73 %).
F = 208°C
$[\alpha]^{26}_D$ = - 5° (c = 0,30; DMSO)

**Exemple 176**

**1-[[2,4-dichloro-3-[[[4-(1*H*-imidazol-1-yl)-2-méthyl-8-quinolinyl]oxy]méthyl]-phényl]sulfonyl]-N-méthyl-2(S)-pipéridinecarboxamide**

En opérant de façon analogue à l'exemple 1, au départ du composé obtenu selon la préparation LXI, on obtient le produit attendu sous forme d'un solide blanc (Rendement = 49 %).
F = 74°C
$[\alpha]^{24}_D$ = + 3° (c = 0,30; $CH_3OH$)

**Exemple 177**

**1-[[2,4-dichloro-3-[[[4-(1*H*-imidazol-1-yl)-2-méthyl-8-quinolinyl]oxy]méthyl]-phényl]sulfonyl]-N-méthyl-2(S)-pipéridinecarboxamide, méthanesulfonate**

En opérant de façon analogue à l'exemple 6, au départ du composé obtenu selon l'exemple 176, on obtient le produit attendu sous forme d'un solide fin jaune (Rendement = 77 %).
F = 153°C
$[\alpha]^{24}_D$ = + 5,2° (c = 0,32; $CH_3OH$)

**PREPARATION LXII**

**Acide 3-[[3-(bromométhyl)-2,4-dichlorophényl]sulfonyl]-4(R)-thiazolidinecarboxylique, méthyl ester**

En opérant de façon analogue à la préparation LIX, au départ de l'ester méthylique de l'acide 4(R)-thiazolidinecarboxylique, on obtient le produit attendu sous forme d'un solide beige (Rendement = 15 %).
F = 48-50°C
$[\alpha]^{24}_D$ = - 40,2° (c = 1,48; $CH_3OH$)

**PREPARATION LXIII**

**Acide 3-[[2,4-dichloro-3-[[[4-(1*H*-imidazol-1-yl)-2-méthyl-8-quinolinyl]oxy]-méthyl]phényl]sulfonyl]-4(R)-thiazolidinecarboxylique, méthyl ester**

En opérant de façon analogue à la préparation I, au départ du composé obtenu selon la préparation LXII, on obtient le produit attendu sous forme d'un solide blanc cassé (Rendement = 50 %).
F = 60°C
$[\alpha]^{27}_D$ = - 31,4° (c = 0,28; $CH_3OH$)

**PREPARATION LXIV**

**Acide 3-[[2,4-dichloro-3-[[[4-(1*H*-imidazol-1-yl)-2-méthyl-8-quinolinyl]oxy]-méthyl]phényl]sulfonyl]-4(R)-thiazolidinecarboxylique**

En opérant de façon analogue à la préparation II, au départ du composé obtenu selon la préparation LXIII, on obtient le produit attendu sous forme d'un solide beige (Rendement = 60 %).
F = 130°C
$[\alpha]^{27}_D$ = - 31,8° (c = 0,33; DMSO)

**Exemple 178**

**3-[[2,4-dichloro-3-[[[4-(1*H*-imidazol-1-yl)-2-méthyl-8-quinolinyl]oxy]méthyl]-phényl]sulfonyl]-N-méthyl-4(R)-thiazolidinecarboxamide**

En opérant de façon analogue à l'exemple 1, au départ du composé obtenu selon la préparation LXIV, on obtient le produit attendu sous forme d'un solide blanc (Rendement = 80 %).
F = 120°C
$[\alpha]^{27}_D$ = - 65,5° (c = 0,36 ; $CH_3OH$)

**Exemple 179**

**3-[[2,4-dichloro-3-[[[4-(1*H*-imidazol-1-yl)-2-méthyl-8-quinolinyl]oxy]méthyl]-phényl]sulfonyl]-N-méthyl-4(R)-thiazolidinecarboxamide, méthanesulfonate**

En opérant de façon analogue à l'exemple 6, au départ du composé obtenu selon l'exemple 178, on obtient le produit attendu sous forme d'un solide jaune (Rendement = 99 %).
F = 143°C
$[\alpha]^{27}_D$ = - 56° (c = 0,33 ; $CH_3OH$)

**PREPARATION LXV**

**Acide 1-[3-(bromométhyl)-2,4-dichlorophényl]-3-pyrrolidinecarboxylique, méthyl ester**

En opérant de façon analogue à la préparation LIX, au départ de l'ester méthylique de l'acide 3-pyrrolidinecarboxylique, on obtient le produit attendu sous forme d'une poudre beige (Rendement = 76 %).
F = 94°C

**PREPARATION LXVI**

**Acide 1-[[2,4-dichloro-3-[[[4-(1*H*-imidazol-1-yl)-2-méthyl-8-quinolinyl]-oxy]méthyl]phényl]sulfonyl]-3-pyrrolidinecarboxylique, méthyl ester**

En opérant de façon analogue à la préparation LX, au départ du composé obtenu selon la préparation LXV, on obtient le produit attendu sous forme d'un solide blanc écru (Rendement = 84 %).
F = 180°C

**PREPARATION LXVII**

**Acide 1-[[2,4-dichloro-3-[[[4-(1*H*-imidazol-1-yl)-2-méthyl-8-quinolinyl]oxy]-méthyl]phényl]sulfonyl]-3-pyrrolidinecarboxylique**

En opérant de façon analogue à la préparation LXI, au départ du composé obtenu selon la préparation LXVI, on obtient le produit attendu sous forme d'un solide blanc (Rendement = 99 %).
F=145°C

**Exemple 180**

**1-[[2,4-dichloro-3-[[[4-(1*H*-imidazol-1-yl)-2-méthyl-8-quinolinyl]oxy]méthyl]-phényl]sulfonyl]-N-méthyl-3-pyrrolidinecarboxamide**

En opérant de façon analogue à l'exemple 1, au départ du composé obtenu selon la préparation LXVII, on obtient le produit attendu sous forme d'un solide blanc (Rendement = 80 %).
F = 108°C

**Exemple 181**

**1-[[2,4-dichloro-3-[[[4-(1*H*-imidazol-1-yl)-2-méthyl-8-quinolinyl]oxy]méthyl]-phényl]sulfonyl]-N-méthyl-3-pyrrolidinecarboxamide, méthanesulfonate**

En opérant de façon analogue à l'exemple 6, au départ du composé obtenu selon l'exemple 180, on obtient le produit attendu sous forme d'un solide jaune (Rendement = 92 %).
F = 137°C

**PREPARATION LXVIII**

**1-[[3-(bromométhyl)-2,4-dichlorophényl]sulfonyl]pyrrolidine**

En opérant de façon analogue à la préparation LIX, au départ de pyrrolidine, on obtient le produit attendu sous forme d'une poudre blanche (Rendement = 94 %).
F = 115°C

**Exemple 182**

**8-[[2,6-dichloro-3-(1-pyrrolidinylsulfonyl)phényl]méthoxy]-4-(1*H*-imidazol-1-yl)-2-méthyl-quinoléïne**

En opérant de façon analogue à la préparation I, au départ du composé obtenu selon la préparation LXVIII, on obtient le produit attendu sous forme d'un solide blanc (Rendement = 67 %).
F=193°C

**Exemple 183**

**8-[[2,6-dichloro-3-(1-pyrrolidinylsulfonyl)phényl]méthoxy]-4-(1*H*-imidazol-1-yl)-2-méthyl-quinoléïne, chlorhydrate**

En opérant de façon analogue à l'exemple 69, au départ du composé obtenu selon l'exemple 182, on obtient le produit attendu sous forme d'une poudre jaune (Rendement = 99 %).
F = 142°C

**Exemple 184**

**1-[[2,4-dichloro-3-[[[4-(1*H*-imidazol-1-yl)-2-méthyl-8-quinolinyl]oxy]méthyl]-phényl]sulfonyl]-4(R)-hydroxy-N-méthyl)-2(S)-pyrrolidinecarboxamide**

En opérant de façon analogue à l'exemple 1, au départ du composé obtenu selon la préparation XXVII, on obtient le produit attendu sous forme d'un solide blanc (Rendement = 49 %).
F = 134°C
$[\alpha]^{24}_D$ = + 5° (c = 0,32; $CH_3OH$)

**Exemple 185**

**1-[[2,4-dichloro-3-[[[4-(1*H*-imidazol-1-yl)-2-méthyl-8-quinolinyl]oxy]méthyl]-phényl]sulfonyl]-4(R)-hydroxy-N-méthyl)-2(S)-pyrrolidinecarboxamide, tartrate**

En opérant de façon analogue à l'exemple 2, au départ du composé obtenu selon l'exemple 184, on obtient le produit attendu sous forme d'un solide fin jaune pâle (Rendement = 82 %).
F = 125°C
$[\alpha]^{24}_D$ = + 10° (c = 0,40 ; $CH_3OH$)

**PREPARATION LXIX**

**Acide 4(R)-méthoxy-2(S)-[(méthylamino)carbonyl]-1-pyrrolidinecarboxylique, phénylméthyl ester**

En opérant de façon analogue à l'exemple 1, au départ de la 1-(phénylméthoxycarbonyl)-4(E)méthoxy-L-proline, on obtient le produit attendu sous forme d'un solide jaune (Rendement = 65 %).
F = 45-47°C

**PREPARATION LXX**

**4(R)-méthoxy-N-méthyl-2(S)-pyrrolidinecarboxamide**

On prépare une solution de 1,27 g ($4{,}34.10^{-3}$ mole) du composé obtenu selon la préparation LXIX dans 100

ml de méthanol et on ajoute 0,13 g de charbon palladié à 10 %. Le mélange est agité sous atmosphère d'hydrogène pendant 2 heures à pression atmosphérique, puis filtré pour éliminer le catalyseur. L'élimination du solvant sous pression réduite permet d'obtenir 0,64 g du produit attendu sous forme d'une huile qui est utilisée sans purification complémentaire à l'étape suivante (Rendement = 93 %)

**PREPARATION LXXI**

**1-[[3-(bromométhyl)-2,4-dichlorophényl]sulfonyl]-4(R)-méthoxy-N-méthyl-2(S)-pyrrolidinecarboxamide**

En opérant de façon analogue à la préparation VII, au départ du composé obtenu selon la préparation LXX, on obtient le produit attendu sous forme d'un solide blanc (Rendement = 80 %).
F = 75°C
$[\alpha]^{27}_D$ = + 16° (c = 0,31; $CH_3OH$)

**Exemple 186**

**1-[[2,4-dichloro-3-[[[4-(1*H*-imidazol-1-yl)-2-méthyl-8-quinolinyl]oxy]méthyl]-phényl]sulfonyl]-4(R)-méthoxy-N-méthyl)-2(S)-pyrrolidinecarboxamide**

En opérant de façon analogue à la préparation I, au départ du composé obtenu selon la préparation LXXI, on obtient le produit attendu sous forme d'un solide blanc (Rendement = 40 %).
F=93°C
$[\alpha]^{27}_D$ = + 19° (c = 0,45; $CH_3OH$)

**Exemple 187**

**1-[[2,4-dichloro-3-[[[4-(1*H*-imidazol-1-yl)-2-méthyl-8-quinolinyl]oxy]méthyl]-phényl]sulfonyl]-4(R)-méthoxy-N-méthyl)-2(S)-pyrrolidinecarboxamide, méthanesulfonate**

En opérant de façon analogue à l'exemple 6, au départ du composé obtenu selon l'exemple 186, on obtient le produit attendu sous forme d'un solide jaunâtre (Rendement = 86 %).
F=143°C
$[\alpha]^{27}_D$ = + 17° (c = 0,36; $CH_3OH$)

**PREPARATION LXXII**

**4(E)-éthoxy-1-(phénylméthoxycarbonyl)-L-proline, éthyl ester**

On prépare une solution de 3 g (11,3.$10^{-3}$ mole) de 4(E)-hydroxy-1-(phénylméthoxycarbonyl)-L-proline dans 15 ml de diméthylformamide et on ajoute 1,12 g (28,2.$10^{-3}$ mole) d'hydrure de sodium (à 60 % dans l'huile). Après 30 mn sous agitation à température ambiante, on ajoute 2,10 ml (26.$10^{-3}$ mole) d'iodoéthane. Le mélange est maintenu sous agitation pendant 24 heures à température ambiante, puis versé sur 250 ml d'eau et extrait par l'acétate d'éthyle. La phase organique est séchée sur sulfate de magnésium puis concentrée sous pression réduite. Le résidu est purifié par chromatographie sur gel de silice en éluant à l'aide d'un mélange toluène/acétate d'éthyle (95/5 ; v/v). On obtient ainsi 2,3 g du produit attendu sous forme d'une huile jaune (Rendement = 63 %).
$[\alpha]^{25}_D$ = - 42,1° (c = 0,42; $CH_3OH$)

**PREPARATION LXXIII**

**4(E)-éthoxy-1-(phénylméthoxycarbonyl)-L-proline**

En opérant de façon analogue à la préparation II, au départ du composé obtenu selon la préparation LXXII, on obtient le produit attendu sous forme d'une huile incolore (Rendement = 99 %).
$[\alpha]^{25}_D$ = - 41,9° (c = 0,52; $CH_3OH$)

**PREPARATION LXXIII**

**4(R)-éthoxy-1-(phénylméthoxycarbonyl)-N-méthyl-2(S)-pyrrolidinecarboxamide**

En opérant de façon analogue à l'exemple 1, au départ du composé obtenu selon la préparation LXXII, on obtient le produit attendu sous forme d'une huile incolore (Rendement = 64 %).
$[\alpha]^{25}_D$ = - 31,7° (c = 0,35; $CH_3OH$)

**PREPARATION LXXIV**

**4(R)-éthoxy-N-méthyl-2(S)-pyrrolidinecarboxamide**

En opérant de façon analogue à la préparation LXX, au départ du composé obtenu selon la préparation LXXIII, on obtient le produit attendu sous forme d'une huile incolore (Rendement = 97 %).
$[\alpha]^{25}_D$ = - 44,2° (c = 0,29; $CH_3OH$)

**PREPARATION LXXV**

**1-[[3-(bromométhyl)-2,4-dichlorophényl]sulfonyl]-4(R)-éthoxy-N-méthyl**

**2(S)-pyrrolidinecarboxamide**

En opérant de façon analogue à la préparation VII, au départ du composé obtenu selon la préparation LXXIV, on obtient le produit attendu sous forme d'un solide beige (Rendement = 89 %).
F = 122°C
$[\alpha]^{25}_D$ = - 5,1° (c = 0,25; $CH_3OH$)

**Exemple 188**

**1-[[2,4-dichloro-3-[[[4-(1*H*-imidazol-1-yl)-2-méthyl-8-quinolyl]oxy]méthyl]-phényl]sulfonyl]-4(R)-éthoxy-N-méthyl-2-(S)-pyrrolidinecarboxamide**

En opérant de façon analogue à la préparation I, au départ du composé obtenu selon la préparation LXXV, on obtient le produit attendu sous forme d'un solide blanc (Rendement = 34 %).
F = 80°C
$[\alpha]^{25}_D$ = + 19,2° (c = 0,22; $CH_3OH$)

**Exemple 189**

**1-[[2,4-dichloro-3-[[[4-(1*H*-imidazol-1-yl)-2-méthyl-8-quinolyl]oxy]méthyl]-phényl]sulfonyl]-4(R)-éthoxy-N-méthyl-2-(S)-pyrrolidinecarboxamide, méthanesulfonate**

En opérant de façon analogue à l'exemple 6, au départ du composé obtenu selon l'exemple 188, on obtient le produit attendu sous forme d'un solide jaune pâle (Rendement = 92 %).
F = 138°C
$[\alpha]^{25}_D$ = + 21,9° (c = 0,30; $CH_3OH$)

**PREPARATION LXXVI**

**4(E)-propoxy-1-(phénylméthoxycarbonyl)-L-proline, propyl ester**

En opérant de façon analogue à la préparation LXXII, au départ d'iodopropane, on obtient le produit attendu sous forme d'une huile jaune (Rendement = 35 %).
$[\alpha]^{25}_D$ = - 52,4° (c = 0,56; $CH_3OH$)

**PREPARATION LXXVII**

**4(E)-propoxy-1-(phénylméthoxycarbonyl)-L-proline**

En opérant de façon analogue à la préparation II, au départ du composé obtenu selon la préparation LXXVI, on obtient le produit attendu sous forme d'une huile jaune (Rendement = 99 %).
$[\alpha]^{25}_D$ = - 38,3° (c = 0,29 ; $CH_3OH$)

**PREPARATION LXXVIII**

**4(R)-propoxy-1-(phénylméthoxycarbonyl)-N-méthyl-2(S)-pyrrolidinecarboxamide**

En opérant de façon analogue à l'exemple 1, au départ du composé obtenu selon la préparation LXXVII, on obtient le produit attendu sous forme d'une huile jaune (Rendement = 75 %).
$[\alpha]^{25}_D$ = - 33° (c = 0,28 ; $CH_3OH$)

**PREPARATION LXXIX**

**4(R)-propoxy-N-méthyl-2(S)-pyrrolidinecarboxamide**

En opérant de façon analogue à la préparation LXX, au départ du composé obtenu selon la préparation LXXVIII, on obtient le produit attendu sous forme d'une huile incolore (Rendement = 90 %).
$[\alpha]^{25}_D$ = - 45,4° (c =0,37 ; $CH_3OH$)

**PREPARATION LXXX**

**1-[[3-(bromométhyl)-2,4-dichlorophényl]sulfonyl]-4(R)-propoxy-N-méthyl-2(S)-pyrrolidinecarboxamide**

En opérant de façon analogue à la préparation VII, au départ du composé obtenu selon la préparation LXXIX, on obtient le produit attendu sous forme d'un solide beige (Rendement = 93 %).
F = 62°C
$[\alpha]^{25}_D$ = - 6,9° (c = 0,27 ; $CH_3OH$)

**Exemple 190**

**1-[[2,4-dichloro-3-[[[4-(1*H*-imidazol-1-yl)-2-méthyl-8-quinolyl]oxy]méthyl]-phényl]sulfonyl]-4(R)-propoxy-N-méthyl-2-(S)-pyrrolidinecarboxamide**

En opérant de façon analogue à la préparation I, au départ du composé obtenu selon la préparation LXXX, on obtient le produit attendu sous forme d'un solide blanc (Rendement = 31 %).
F = 84°C
$[\alpha]^{25}_D$ = + 25,3° (c = 0,22; $CH_3OH$)

**Exemple 191**

**1-[[2,4-dichloro-3-[[[4-(1*H*-imidazol-1-yl)-2-méthyl-8-quinolyl]oxy]méthyl]-phényl]sulfonyl]-4(R)-propoxy-N-méthyl-2-(S)-pyrrolidinecarboxamide, méthanesulfonate**

En opérant de façon analogue à l'exemple 6, au départ du composé obtenu selon l'exemple 190, on obtient le produit attendu sous forme d'un solide jaune pâle (Rendement = 94 %).
F = 141°C
$[\alpha]^{25}_D$ = + 13,7° (c = 0,30; $CH_3OH$)

**PREPARATION LXXXI**

**4(E)-(cyclopropylméthoxy)-1-(phénylméthoxycarbonyl)-L-proline, cyclopropylméthyl ester**

En opérant de façon analogue à la préparation LXXII, au départ de bromométhyl-cyclopropane, on obtient le

produit attendu sous forme d'une huile jaune (Rendement = 27 %).
$[\alpha]^{25}_D$ = - 28,7° (c = 0,33; $CH_3OH$)

**PREPARATION LXXXII**

**4(E)-(cyclopropylméthoxy)-1-(phénylméthoxycarbonyl)-L-proline,**

En opérant de façon analogue à la préparation II, au départ du composé obtenu selon la préparation LXXXI, on obtient le produit attendu sous forme d'une huile incolore (Rendement = 98 %).
$[\alpha]^{25}_D$ = - 31,1° (c = 0,25; $CH_3OH$)

**PREPARATION LXXXIII**

**4(R)-(cyclopropylméthoxy)-1-(phénylméthoxycarbonyl)-N-méthyl-2(S)-pyrrolidinecarboxamide**

En opérant de façon analogue à l'exemple 1, au départ du composé obtenu selon la préparation LXXXII, on obtient le produit attendu sous forme d'une huile incolore (Rendement = 74 %).
$[\alpha]^{25}_D$ = - 28,8° (c = 0,28; $CH_3OH$)

**PREPARATION LXXXIV**

**4(R)-(cyclopropylméthoxy)-N-méthyl-2(S)-pyrrolidinecarboxamide**

En opérant de façon analogue à la préparation LXX, au départ du composé obtenu selon la préparation LXXXIII, on obtient le produit attendu sous forme d'une huile incolore (Rendement = 82 %).
$[\alpha]^{25}_D$ = - 34,2° (c = 0,24; $CH_3OH$)

**PREPARATION LXXXV**

**1-[[3-(bromométhyl)-2,4-dichlorophényl]sulfonyl]-4(R)-(cyclopropylméthoxy)-N-méthyl-2(S)-pyrrolidinecarboxamide**

En opérant de façon analogue à la préparation VII, au départ du composé obtenu selon la préparation LXXXIV, on obtient le produit attendu sous forme d'un solide blanc (Rendement = 90 %).
F = 161°C
$[\alpha]^{25}_D$ = - 3,9° (c = 0,27; $CH_3OH$)

**Exemple 192**

**1-[[2,4-dichloro-3-[[[4-(1*H*-imidazol-1-yl)-2-méthyl-8-quinolyl]oxy]méthyl]-phényl]sulfonyl]-4(R)-(cyclopropylméthoxy)-N-méthyl-2-(S)-pyrrolidinecarboxamide.**

En opérant de façon analogue à la préparation I, au départ du composé obtenu selon la préparation LXXXV, on obtient le produit attendu sous forme d'un solide blanc (Rendement = 59 %).
-F = 98°C
$[\alpha]^{25}_D$ = + 21.2° (c = 0,23; $CH_3OH$)

**Exemple 193**

**1-[[2,4-dichloro-3-[[[4-(1*H*-imidazol-1-yl)-2-méthyl-8-quinolyl]oxy]méthyl]-phényl)sulfonyl]-4(R)-(cyclopropylméthoxy)-N-méthyl-2-(S)-pyrrolidinecarboxamide, méthanesulfonate**

En opérant de façon analogue à l'exemple 6, au départ du composé obtenu selon l'exemple 192, on obtient le produit attendu sous forme d'un solide jaune pâle (Rendement = 87 %).
F=149°C
$[\alpha]^{25}_D$ = +22,9° (c = 0,29; $CH_3OH$)

**PREPARATION LXXXVI**

**4(R)-(1,1-diméthyléthoxy)-1-(phénylméthoxycarbonyl)-N-méthyl-2(S)-pyrrolidinecarboxamide**

En opérant de façon analogue à l'exemple 1, au départ de 4(E)-(1,1-diméthyléthoxy)-1-(phénylméthoxycarbonyl)-L-proline, on obtient le produit attendu sous forme d'une huile incolore (Rendement = 86 %).
$[\alpha]^{25}_D$ = - 6,2° (c = 0,43; $CH_3OH$)

**PREPARATION LXXXVII**

**4(R)-(1,1-diméthyléthoxy)-N-méthyl-2(S)-pyrrolidinecarboxamide**

En opérant de façon analogue à la préparation LXX, au départ du composé obtenu selon la préparation LXXXVI, on obtient le produit attendu sous forme d'une huile incolore (Rendement = 99 %).
$[\alpha]^{25}_D$ = - 34,8° (c = 0,68; $CH_3OH$)

**PREPARATION LXXXVIII**

**1-[[3-(bromométhyl)-2,4-dichlorophényl]sulfonyl]-4(R)-(1,1-diméthyléthoxy)-N-méthyl-2(S)-pyrrolidinecarboxamide**

En opérant de façon analogue à la préparation VII, au départ du composé obtenu selon la préparation LXXVII, on obtient le produit attendu sous forme d'un solide blanc (Rendement = 91 %).
F=73°C
$[\alpha]^{25}_D$ = - 6,4° (c = 0,44; $CH_3OH$)

**Exemple 194**

**1-[[2,4-dichloro-3-[[[4-(1*H*-imidazol-1-yl)-2-méthyl-8-quinolyl]oxy]méthyl]-phényl]sulfonyl]-4(R)-(1,1-diméthyléthoxy)-N-méthyl-2-(S)-pyrrolidinecarboxamide**

En opérant de façon analogue à la préparation I, au départ du composé obtenu selon la préparation LXXXVIII, on obtient le produit attendu sous forme d'un solide blanc (Rendement = 65 %).
F = 84°C
$[\alpha]^{25}_D$ = + 19,4° (c = 0,26; $CH_3OH$)

**Exemple 195**

**1-[[2,4-dichloro-3-[[[4-(1*H*-imidazol-1-yl)-2-méthyl-8-quinolyl]oxy]méthyl]-phényl]sulfonyl]-4(R)-(1,1-diméthyléthoxy)-N-méthyl-2-(S)-pyrrolidinecarboxamide, méthanesulfonate**

En opérant de façon analogue à l'exemple 6, au départ du composé obtenu selon l'exemple 194, on obtient le produit attendu sous forme d'un solide jaune pâle (Rendement = 96 %).
F = 150°C
$[\alpha]^{25}_D$ = + 21,6° (c = 0,26; $CH_3OH$)

**PREPARATION LXXXIX**

**1-[(1,1-diméthyléthoxy)carbonyl]-4(R)-(phénylméthoxy)-N-méthyl-2(S)-pyrrolidinecarboxamide**

En opérant de façon analogue à l'exemple 1, au départ de 1-[(1,1-diméthyléthoxy)carbonyl]-4(E)-(phénylméthoxy)-L-proline, on obtient le produit attendu sous forme d'une huile incolore (Rendement = 82 %).
$[\alpha]^{25}_D$ = - 13,4° (c = 0,14; $CH_3OH$)

**PREPARATION XC**

**4(R)-(phénylméthoxy)-N-méthyl-2(S)-pyrrolidinecarboxamide, trifluoroacétate**

En opérant de façon analogue à l'exemple 9, au départ du composé obtenu selon la préparation LXXXIX, on obtient le produit attendu sous forme d'un solide jaune (Rendement = 98 %).
F = 54°C
$[\alpha]^{25}_D$ = - 3,1° (c = 0,37; $CH_3OH$)

**PREPARATION XCI**

**1-[[3-(bromométhyl)-2,4-dichlorophényl]sulfonyl]-4(R)-(phénylméthoxy)-N-méthyl-2(S)-pyrrolidinecarboxamide**

En opérant de façon analogue à la préparation VII, au départ du composé obtenu selon la préparation XC, on obtient le produit attendu sous forme d'un solide blanc (Rendement = 73 %).
F=62-64°C
$[\alpha]^{25}_D$ = - 14,2° (c = 0,37; $CH_3OH$)

**Exemple 196**

**1-[[2,4-dichloro-3-[[[4-(1*H*-imidazol-1-yl)-2-méthyl-8-quinolyl]oxy]méthyl]-phényl]sulfonyl]-4(R)-(phénylméthoxy)-N-méthyl-2-(S)-pyrrolidinecarboxamide**

En opérant de façon analogue à la préparation I, au départ du composé obtenu selon la préparation XCI, on obtient le produit attendu sous forme d'un solide blanc (Rendement = 29 %).
F=100°C
$[\alpha]^{25}_D$ = + 6,1° (c = 0,29; $CH_3OH$)

**Exemple 197**

**1-[[2,4-dichloro-3-[[[4-(1*H*-imidazol-1-yl)-2-méthyl-8-quinolyl]oxy]méthyl]-phényl]sulfonyl]-4(R)-(phénylméthoxy)-N-méthyl-2-(S)-pyrrolidinecarboxamide, méthanesulfonate**

En opérant de façon analogue à l'exemple 6, au départ du composé obtenu selon l'exemple 196, on obtient le produit attendu sous forme d'un solide blanc (Rendement = 96 %).
F=140-142°C
$[\alpha]^{25}_D$ = + 20,1° (c = 0,32; $CH_3OH$)

**PREPARATION XCII**

**2,5-dihydro-1-[(1,1-diméthyléthoxy)carbonyl]-N-méthyl-1*H*-pyrrole-2-(S)-carboxamide**

En opérant de façon analogue à l'exemple 1, au départ d'acide 2,5-dihydro-1-[(1,1-diméthyléthoxy)carbonyl]-1*H*-pyrrole-2(S)-carboxylique, on obtient le produit attendu sous forme d'un solide blanc (Rendement = 77 %).
F = 47-48°C
$[\alpha]^{19}_D$ = - 166° (c = 0,4 ; $CH_3OH$)

**PREPARATION XCIII**

**2,5-dihydro-N-méthyl-1*H*-pyrrole-2-(S)-carboxamide, trifluoroacétate**

En opérant de façon analogue à l'exemple 9, au départ du composé obtenu selon la préparation XCII, on obtient le produit attendu sous forme d'une huile (Rendement = 98 %).
$[\alpha]^{19}_D$ = - 67° (c = 0,50 ; $CH_3OH$)

**PREPARATION XCIV**

**1-[[3-(bromométhyl)-2,4-dichlorophényl]sulfonyl]-2,5-dihydro-N-méthyl-1*H*-pyrrole-2(S)-carboxamide**

En opérant de façon analogue à la préparation VII, au départ du composé obtenu selon la préparation XCIII, on obtient le produit attendu sous forme d'un solide blanc (Rendement = 86 %).
F = 66°C
$[\alpha]^{25}_D$ = - 111° (c = 0,43; $CH_3OH$)

**Exemple 198**

**1-[[2,4-dichloro-3-[[[4-(1*H*-imidazol-1-yl)-2-méthyl-8-quinolinyl]oxy]méthyl]-phényl]sulfonyl]-2,5-dihydro-N-méthyl-1*H*-pyrrole-2(S)-carboxamide**

En opérant de façon analogue à la préparation I, au départ du composé obtenu selon la préparation XCIV, on obtient le produit attendu sous forme d'un solide blanc (Rendement = 54 %).
F = 132°C
$[\alpha]^{25}_D$ = - 92° (c = 0,33; $CH_3OH$)

**Exemple 199**

**1-[[2,4-dichloro-3-[[[4-(1*H*-imidazol-1-yl)-2-méthyl-8-quinolinyl]oxy]méthyl]-phényl]sulfonyl]-2,5-dihydro-N-méthyl-1*H*-pyrrole-2(S)-carboxamide, méthanesulfonate**

En opérant de façon analogue à l'exemple 6, au départ du composé obtenu selon l'exemple 198, on obtient le produit attendu sous forme d'un solide fin jaune (Rendement = 99 %).
F = 139°C
$[\alpha]^{25}_D$ = - 76° (c = 0,44; $CH_3OH$)

**PREPARATION XCV**

**Acide 1-[[3-(bromométhyl)-2,4-dichlorophényl]sulfonyl]-2(S)-azetidinecarboxylique, méthyl ester**

En opérant de façon analogue à la préparation VII, au départ du 2(S)-azetidinecarboxylate de méthyle, on obtient le produit attendu sous forme d'un solide blanc (Rendement = 33 %).
F = 150°C
$[\alpha]^{28}_D$ = + 6° (c =0,38; $CH_3OH$)

**PREPARATION XCVI**

**Acide 1-[[2,4-dichloro-3-[[[4-(1*H*-imidazol-1-yl)-2-méthyl-8-quinolinyl]oxy]-méthyl]phényl]sulfonyl]-2(S)-azetidinecarboxylique, méthyl ester**

En opérant de façon analogue à la préparation I, au départ du composé obtenu selon la préparation XCV, on obtient le produit attendu sous forme d'un solide blanc (Rendement = 77 %).
F = 80°C
$[\alpha]^{28}_D$ = + 73° (c = 0,32; $CH_3OH$)

**PREPARATION XCVII**

**Acide 1-[[2,4-dichloro-3-[[[4-(1*H*-imidazol-1-yl)-2-méthyl-8-quinolinyl]oxy]-méthyl]phényl]sulfonyl]-2(S)-azetidinecarboxylique**

En opérant de façon analogue à la préparation II, au départ du composé obtenu selon la préparation XCVI, on obtient le produit attendu sous forme d'un solide blanc écru (Rendement = 68 %).
F = 160°C
$[\alpha]^{28}_D$ = + 11,6° (c = 0,32; $CH_3OH$)

**Exemple 200**

**1-[[2,4-dichloro-3-[[[4-(1*H*-imidazol-1-yl)-2-méthyl-8-quinolinyl]oxy]méthyl]-phényl]sulfonyl]-N-méthyl-2(S)-azetidinecarboxamide**

En opérant de façon analogue à l'exemple 1, au départ du composé obtenu selon la préparation CXVII, on obtient le produit attendu sous forme d'un solide beige (Rendement = 98 %).
F = 118°C
$[\alpha]^{28}_D$ = - 37,8° (c = 0,33; $CH_3OH$)

**Exemple 201**

**1-[[2,4-dichloro-3-[[[4-(1*H*-imidazol-1-yl)-2-méthyl-8-quinolinyl]oxy]méthyl]-phényl]sulfonyl]-N-méthyl-2(S)-azetidinecarboxamide, méthanesulfonate**

En opérant de façon analogue à l'exemple 6, au départ du composé obtenu selon l'exemple 200, on obtient le produit attendu sous forme d'un solide jaunâtre (Rendement = 81 %).
F = 135°C
$[\alpha]^{28}_D$ = - 21,1° (c = 0,35; $CH_3OH$)

**PREPARATION XCVIII**

**1-[[3-(bromométhyl)-2,4-dichlorophényl]sulfonyl]-4(E)-phénoxy-L-proline, méthyl ester**

En opérant de façon analogue à l'exemple 127, au départ du chlorure de 3-(bromométhyl)-2,4-dichlorobenzènesulfonyle et de l'ester méthylique de la 4(trans)-phénoxy-L-proline, on obtient le produit attendu sous forme d'une huile jaune (Rendement = 75 %).
$[\alpha]^{24}_D$ = - 16 (c = 0,55; $CHCl_3$)

**PREPARATION IC**

**1-[[2,4-dichloro-3-[[[4-(1*H*-imidazol-1-yl)-2-méthyl-8-quinolinyl]oxy]méthyl]-phényl]sulfonyl]-4(E)-phénoxy-L-proline, méthyl ester**

En opérant de façon analogue à la préparation I, au départ du composé obtenu selon la préparation XCVIII, on obtient le produit attendu sous forme d'un solide jaune (Rendement = 75 %).
F = 88°C
$[\alpha]^{23}_D$ = - 1,36° (c = 0,5; $CHCl_3$)

**PREPARATION C**

**1-[[2,4-dichloro-3-[[[4-(1*H*-imidazol-1-yl)-2-méthyl-8-quinolinyl]oxy]méthyl]-phényl]sulfonyl]-4(E)-phénoxy-L-proline**

En opérant de façon analogue à la préparation II, au départ du composé obtenu selon la préparation IC, on obtient le produit attendu sous forme d'un solide beige (Rendement = 77 %).
F = 150°C
$[\alpha]^{27}_D$ = + 20,9° (c = 0,58; DMSO)

**Exemple 202**

**1-[[2,4-dichloro-3-[[[4-(1*H*-imidaxol-1-yl)-2-méthyl-8-quinolinyl]oxy]méthyl]-phényl]sulfonyl]-N-méthyl-4(R)-phénoxy-2(S)-pyrrolidinecarboxamide**

En opérant de façon analogue à l'exemple 1, au départ du composé obtenu selon la préparation C, on obtient le produit attendu sous forme d'un solide blanc (Rendement = 37 %).
F = 97°C
$[\alpha]^{27}_D$ = -2,9° (c = 0,55; $CH_3OH$)

## Exemple 203

**1-[[2,4-dichloro-3-[[[4-(1*H*-imidazol-1-yl)-2-méthyl-8-quinolinyl]oxy]méthyl]-phényl]sulfonyl]-N-méthyl-4(R)-phénoxy-2(S)-pyrrolidinecarboxamide, méthanesulfonate**

En opérant de façon analogue à l'exemple 6, au départ du composé obtenu selon l'exemple 202, on obtient le produit attendu sous forme d'un solide blanc (Rendement = 92 %).
F = 147°C
$[\alpha]^{23}_D$ = - 4,8° (c = 0,47 ; $CH_3OH$)

## PREPARATION CI

**4(S)-méthoxy-N-méthyl-1-[(phénylméthoxy)carbonyl]-2(S)-pyrrolidine carboxamide**

En opérant de façon analogue à l'exemple 1, au départ de 4(cis)-méthoxy-1-[(phénylméthoxy)-carbonyl]-L-proline, on obtient le produit attendu sous forme d'une huile incolore (Rendement = 76 %).
$[\alpha]^{27}_D$ = - 38° (c = 0,81; $CH_3OH$)

## PREPARATION CII

**4(S)-méthoxy-N-méthyl-2(S)-pyrrolidinecarboxamide**

En opérant de façon analogue à la préparation LXX, au départ du composé obtenu selon la préparation CI, on obtient le produit attendu sous forme d'une huile incolore (Rendement = 95 %).

## PREPARATION CIII

**1-[[3-(bromométhyl)-2,4-dichlorophényl]sulfonyl]-4(S)-méthoxy-N-méthyl-2(S)-pyrrolidinecarboxamide**

En opérant de façon analogue à la préparation XCVIII, au départ du composé obtenu selon la préparation CII, on obtient le produit attendu sous forme d'un solide jaunâtre (Rendement = 90 %).
F = 64°C
$[\alpha]^{27}_D$ = - 17° (c = 0,69; $CHCl_3$)

## Exemple 204

**1-[[2,4-dichloro-3-[[[4-(1*H*-imidazol-1-yl)-2-méthyl-8-quinolinyl]oxy]méthyl]-phényl]sulfonyl]-4(S)-méthoxy-N-méthyl-2(S)-pyrrolidinecarboxamide**

En opérant de façon analogue à la préparation I, au départ du composé obtenu selon la préparation CIII, on obtient le produit attendu sous forme d'un solide blanc (Rendement = 72 %).
F = 64°C
$[\alpha]^{23}_D$ = - 22,7° (c = 0,51; $CHCl_3$)

## Exemple 205

**1-[[2,4-dichloro-3-[[[4-(1*H*-imidazol-1-yl)-2-méthyl-8-quinolinyl]oxy]méthyl]-phényl]sulfonyl]-4(S)-méthoxy-N-méthyl-2(S)-pyrrolidinecarboxamide, méthanesulfonate**

En opérant de façon analogue à l'exemple 6, au départ du composé obtenu selon l'exemple 204, on obtient le produit attendu sous forme d'un solide jaune (Rendement = 90 %).
F = 135°C
$[\alpha]^{27}_D$ = - 5,3° (c = 0,4; $CH_3OH$)

**Exemple 206**

**1-[[2,4-dichloro-3-[[[2-méthyl-4-(1*H*-1,2,4-triazol-1-yl)-2-méthyl-8-quinolinyl]oxy]méthyl]phényl]sulfonyl]-4(R)-méthoxy-N-méthyl-2(S)-pyrrolidine carboxamide**

En opérant de façon analogue à l'exemple 186, au départ de 8-hydroxy-2-méthyl-4-(lH-1,2,4-triazol-1-yl)quinoléine, on obtient le produit attendu sous forme d'un solide blanc (Rendement = 62 %).
F = 73°C
$[\alpha]^{27}_D$ = + 17,2° (c = 0,68 ; $CH_3OH$)

**Exemple 207**

**1-[[2,4-dichloro-3-[[[2-méthyl-4-(1*H*-1,2,4-triazol-1-yl)-2-méthyl-8-quinolinyl]oxy]méthyl]phényl]sulfonyl]-4(R)-méthoxy-N-méthyl-2(S)-pyrrolidinecarboxamide, méthanesulfonate**

En opérant de façon analogue à l'exemple 6, au départ du composé obtenu selon l'exemple 206, on obtient le produit attendu sous forme d'un solide jaune (Rendement = 87 %).
F = 134°C
$[\alpha]^{23}_D$ = + 38° (c = 0,52 ; $CH_3OH$)

L'activité des produits selon l'invention a été évaluée, selon un premier aspect, en fonction de leur aptitude à se lier aux récepteurs $B_2$ de la bradykinine. On sait que les kinines, dont l'un des principaux représentants est la bradykinine, forment un groupe de petits peptides qui contribuent de façon importante à la réponse inflammatoire et apparaissent de ce fait impliqués dans la pathologie des maladies inflammatoires. On sait également que la bradykinine est un des agents algésiants parmi les plus puissants connus. Le mode d'action des kinines et plus particulièrement de la bradykinine fait intervenir un couplage des peptides aux deux types de récepteurs appelés respectivement $B_1$ et $B_2$. Le récepteur $B_2$ appartient à la grande famille des récepteurs à sept domaines transmembranaires couplés aux G-protéines et semble plus particulièrement impliqué dans le domaine des pathologies citées précédemment. C'est la raison pour laquelle les produits de l'invention, qui ont la propriété de pouvoir se fixer sur le récepteur $B_2$, inhibent la fixation de la bradykinine et, par conséquence, suppriment son activité néfaste. Le test mis en oeuvre pour mesurer cette propriété est un test de fixation compétitive sur des membranes de cellules CHO exprimant le récepteur humain $B_2$ utilisant la bradykinine marquée au tritium ([$^3$H]-bradykinine) en tant que ligand.

Les résultats sont exprimés par la valeur du Ki, telles que calculée selon la méthode préconisée avec la description de l'essai mis en oeuvre et décrite selon D. Pruneau et col. Dans Br. J, Pharmacol. 1998, 125 p 365-372.

Selon un second aspect du contrôle de l'activité, il était important de vérifier que les produits de l'invention possèdent bien un caractère antagoniste de la bradykinine vis à vis du récepteur $B_2$, c'est à dire que le composé, après fixation sur le récepteur $B_2$, ne provoque pas les symptômes analogues à ceux provoqués par la fixation de la bradykinine sur ledit récepteur $B_2$. Cette caractéristique antagoniste est exprimée par la valeur $pA_2$, calculée d'après un essai biologique mis en oeuvre pour mesurer l'inhibition de la contraction de la veine ombilicale humaine isolée, par les composés selon l'invention en présence de bradykinine. La procédure du test et la méthode de calcul de $pA_2$ sont décrits dans les articles de D. Pruneau et col. Publiés dans Br. J. Pharmacol. 1998, 125, p 365-372 et JL. Paquet et col. B. J. Pharmacol. 1999, 126 (en impression).

Les valeurs obtenues par certains composés de l'invention sont rassemblées dans le tableau 1 ci-après. Les valeurs trouvées pour le Ki montrent des valeurs inférieures à 1 nM, témoignant d'une excellente affinité des composés pour le récepteur $B_2$ de la bradykinine. Les valeurs trouvées pour $pA_2$ sont représentatives du caractère antagoniste des composés vis à vis du récepteur $B_2$ de la bradykinine.

Les composés de la présente invention, en raison de leur propriété antagoniste de la bradykinine vis à vis de son récepteur $B_2$, sont utiles dans le traitement des algies, et dans le traitement de nombreuses pathologies impliquant la bradykinine ou ses homologues. Parmi ces pathologies, on inclut les chocs septiques et hémorragiques, les réactions anaphylactiques, l'arthrose, la polyarthrite rhumatoïde, les rhinites, l'asthme, les maladies inflammatoires du tractus gastro-intestinal (par ex. colites, rectites, maladie de Crohn), la pancréatite, certains carcinomes, l'angiooedème héréditaire, la migraine, l'encéphalomyélite, la méningite, les accidents vasculaires cérébraux (notamment ceux provoqués par un choc traumatique cérébral), certains désordres neurologiques, les états inflammatoires vasculaires (par exemple : athérosclérose et artérite des membres inférieurs), les états douloureux (par exemple les céphalalgies, les douleurs dentaires, les douleurs menstruelles), les contractions utérines prématurées, la cystite et les brûlures. Les composés selon l'invention peuvent également être utiles pour la potentialisation d'agents antiviraux.

Les composés de la présente invention, qui peuvent être utilisés sous forme de base libre ou de leurs sels d'addition non toxiques, en association avec un excipient physiologiquement acceptable, sont en général prescrits en thérapeutique humaine à des doses d'environ 1 à 1000 mg/jour, sous une forme administrable par voie orale, par

injection intraveineuse, intramusculaire ou sous-cutanée, par voie transdermique, par le moyen d'aérosols ou par le moyen de suppositoires. Ces composés sont également administrables par voie topique, notamment sous forme de gel ou de pommade.

Les composés de la présente invention trouvent également leur utilité dans le domaine de la cosmétique pour traiter des pathologies de la peau ou du cuir chevelu.

TABLEAU I

| **Exemples** | **Activité biologique** | |
|---|---|---|
| | **Ki (nM)** | **pA2** |
| 4 | 0,24 | 10 |
| 10 | 1,0 | 8,5 |
| 12 | 0,47 | 8,7 |
| 23 | 0,45 | 9,1 |
| 30 | 0,73 | 8,7 |
| 32 | 1,4 | 9,1 |
| 42 | 77 | 8,3 |
| 48 | 32 | 8,5 |
| 50 | 30 | 8,3 |
| 61 | 21 | 8,1 |
| 64 bis | 0,034 | 9,3 |
| 73 | 10 | 8,4 |
| 75 | 2.8 | 8,3 |
| 77 | 6.1 | 8,6 |
| 83 | 14 | 7,9 |
| 87 | 15 | 8,2 |
| 89 | 55 | 8,0 |
| 101 | 50 | 8,4 |
| 103 | 21 | 7,9 |
| 105 | 7,7 | 8,3 |
| 109 | 15 | 8,3 |
| 115 | 35 | 8,5 |
| 123 | 8.1 | 8,4 |
| 166 | 5,8 | 8,2 |
| 170 | 7,8 | 8,0 |
| 174 | 8,8 | 8,1 |

## Revendications

**1.** Composé hétérocyclique de benzènesulfonamide, **caractérisé en ce qu'**il est choisi parmi l'ensemble constitué par :

(i) les composés de formule I :

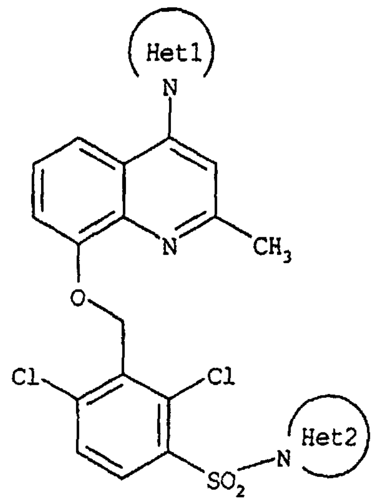

dans laquelle :

Het1 représente un hétérocycle azoté à 5 sommets, notamment l'imidazole, le pyrazole ou le triazole,
Het2 représente un hétérocycle azoté à 4, 5 ou 6 sommets de structure :

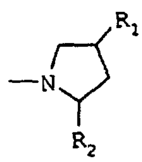 , 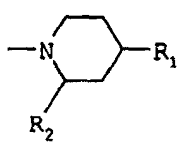 , 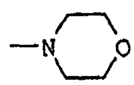 ,

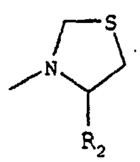 , 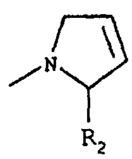 ou 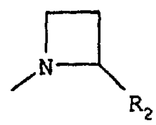

dans lesquels
$R_1$ représente un atome d'hydrogène ou un groupe hydroxy, alcoxy en $C_1$-$C_4$, phénoxy, phénylméthoxy, $-CH_2OH$, cycloalkyloxy, cycloalkylalcoxy (où chaque fragment cycloalkyle est en $C_3$-$C_8$ et le fragment alcoxy en $C_1$-$C_4$), $-NH-CO-CH_3$, $-CO-NH_2$ ou $-CO-NH-CH_3$,
$R_2$ représente un atome d'hydrogène ou un groupe $-CH_2OH$, $-CH_2-O-CH_3$, $-CONR_3R_4$,

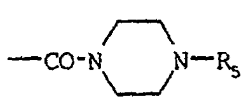 ou 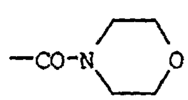

$R_3$ représente un atome d'hydrogène, un groupe alkyle en $C_1$-$C_3$, un groupe cycloalkyle en $C_3$-$C_8$, un groupe cycloalkyl (en $C_3$-$C_8$)-alkyle (en $C_1$-$C_3$), un groupe phényle, ou un groupe phénylméthyle,
$R_4$ représente un atome d'hydrogène, un groupe alkyle en $C_1$-$C_3$, $-(CH_2)n-CH_2OH$, $-(CH_2)n-COOH$, $-(CH_2)n-CH_2-NR_5R_6$,

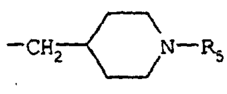 , 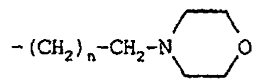 ou 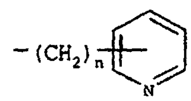

$R_5$ représente un atome d'hydrogène, un groupe alkyle en $C_1$-$C_3$, phényle, phénylméthyle, pyridinyle, pyridinylméthyle, pyridinyléthyle, benzoyle, 4-(aminoiminométhyl)benzoyle, $-(CH_2)_m-CH_2OH$, $-(CH_2)_m-COOH$, $-(CH_2)_mCH_2-O-(CH_2)_m-CH_2OH$, $-CO-(CH_2)_m-COOH$, ou

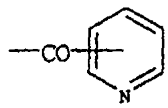

$R_6$ représente un atome d'hydrogène ou un groupe alkyle en $C_1$-$C_3$,

ou, $R_5$ et $R_6$ considérés ensemble forment, avec l'atome d'azote auquel ils sont attachés, un N-hétérocycle de 5 à 6 sommets,

$$n = 1, 2, 3 \text{ ou } 4,$$

$$m = 1, 2 \text{ ou } 3 \text{ ; et,}$$

(ii) leurs sels d'addition.

2. Composé de formule I selon la revendication 1, **caractérisé en ce que** Het1 représente un groupe 1-(1*H*)-imidazolyle.

3. Composé de formule I selon l'une des revendications 1 ou 2, **caractérisé en ce que** Het2 représente un groupe 2(S)-pyrrolidinecarboxamide

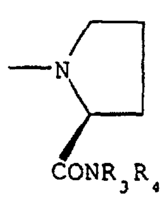

$R_3$ représente un atome d'hydrogène ou un groupe alkyle en $C_1$-$C_3$,
$R_4$ représente un groupe alkyle en $C_1$-$C_3$, un groupe $-(CH_2)n-CH_2-NR_5R_6$, un groupe pyridinylméthyle, ou un groupe

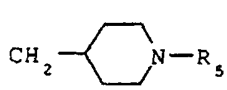

$R_5$ représente un groupe $(CH_2)_m-CH_2OH$, un groupe pyridinylméthyle ou un groupe 4-(aminoiminométhyl) benzoyle,

$R_6$ représente un groupe méthyle ou forme avec $R_5$ et l'azote auquel ils sont liés, un hétérocycle saturé à 5 ou 6 sommets.

4. Composé de formule I selon l'une des revendications 1 ou 2, **caractérisé en ce que** Het2 représente un groupe 2(S)-pyrrolidinecarboxamide de formule

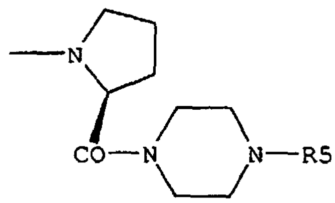

dans lequel $R_5$ représente un groupe pyridinyle ou un groupe pyridinylméthyle

5. Procédé de préparation d'un composé de formule I, **caractérisé en ce qu'**il comprend les étapes consistant à :

(1) faire réagir un dérivé de la 8-hydroxyquinoléine de formule II :

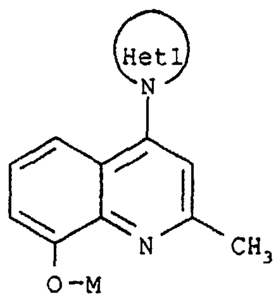

(II)

dans laquelle :

Het1 représente un hétérocycle azoté à cinq sommets comprenant au total 1, 2, 3 ou 4 atomes d'azote et M représente un métal alcalin, notamment le sodium ou le potassium,
avec un composé de formule III :

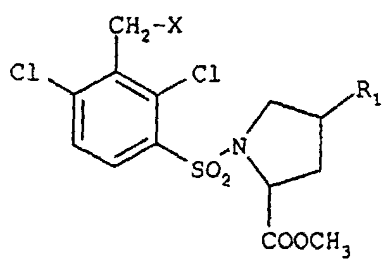

(III)

dans laquelle :

X représente un atome d'halogène, de préférence un atome de brome, et
$R_1$ représente un atome d'hydrogène, un groupe OH, un groupe alcoxy ou un groupe phénoxy,
dans un solvant anhydre, à une température comprise entre 0 et 50°C, pendant 0,5 à 10 heures, pour obtenir un composé de formule IV :

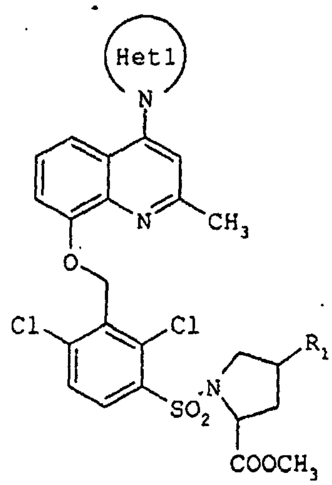

(IV)

dans laquelle :

Het1 et $R_1$ conservent la même signification que précédemment ;

(2) hydrolyser la fonction ester du composé de formule IV, ainsi obtenu, pour obtenir un composé de formule V :

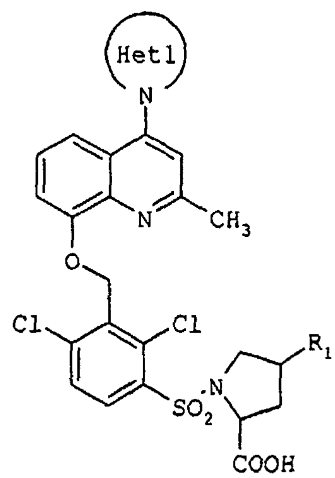

(V)

dans laquelle :

Het1 et $R_1$ conservent la même signification que ci-dessus ;

(3) faire réagir le composé de formule V, ainsi obtenu, avec une amine de formule :

$$HNR_3R_4 \quad (VI)$$

dans laquelle :

$R_3$ représente un atome d'hydrogène ou un groupe alkyle en $C_1$-$C_3$,
$R_4$ représente un atome d'hydrogène, un groupe alkyle en $C_1$-$C_3$, $-(CH_2)_n-CH_2OH$, $-(CH_2)_n-COOR_{11}$,

$-(CH_2)_n-CH_2-NR_5R_6$,

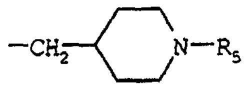 , 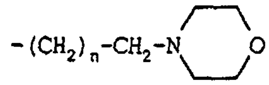 ou 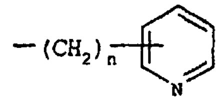

$R_5$ représente un groupe alkyle en $C_1$-$C_3$, $-(CH_2)_m-CH_2OH$, $-(CH_2)_m-COOR_{11}$, $-(CH_2)_m-CH_2-O-(CH_2)_m-CH_2OH$, ou un groupe aminoprotecteur ($R_5$ et $R_6$ n'étant pas simultanément des groupes aminoprotecteurs),
$R_6$ représente un groupe alkyle en $C_1$-$C_3$ ou un groupe aminoprotecteur,
$R_{11}$ représente un groupe protecteur de la fonction acide facilement hydrolysable,

$$n = 1, 2, 3 \text{ ou } 4,$$

$$m = 1, 2 \text{ ou } 3,$$

dans un solvant, en présence d'activateurs, à une température voisine de la température ambiante (0-40°C, de préférence 10-35°C), pendant 2 à 50 heures, pour obtenir un composé de formule :

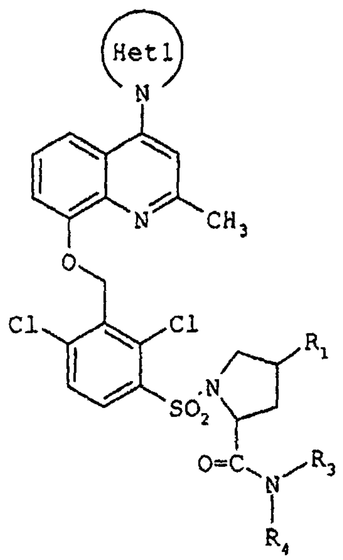

(VII)

dans laquelle :

Het1, $R_1$, $R_3$, $R_4$ conservent la même signification que précédemment ; et,

(4) si nécessaire, faire réagir le composé de formule VII, ainsi obtenu, pour remplacer chaque groupe amino- ou acidoprotecteur par un atome d'hydrogène, de façon à obtenir le composé de formule I':

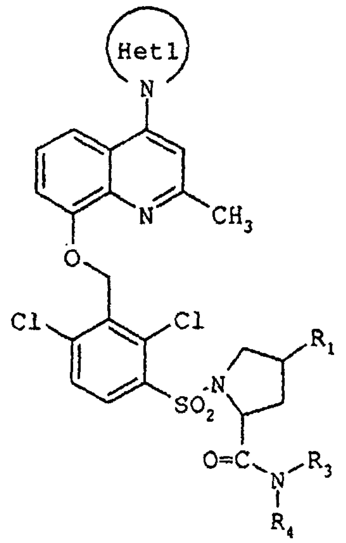

(I')

dans laquelle :

Het1, $R_1$, $R_3$ et $R_4$ conservent la même signification que ci-dessus, à l'exception des groupes protecteurs remplacés par des atomes d'hydrogène ; puis,

(5) si nécessaire, faire réagir le composé de formule $I^{1'}$, ainsi obtenu, avec un acide pour obtenir le sel d'addition d'acide correspondant.

**6.** Procédé de préparation d'un composé de formule I selon la revendication 1, **caractérisé en ce qu'**il comprend les étapes consistant à :

(1) faire réagir un composé de formule I'tel qu'obtenu à l'étape (4) du procédé selon la revendication 5 ci-dessus,

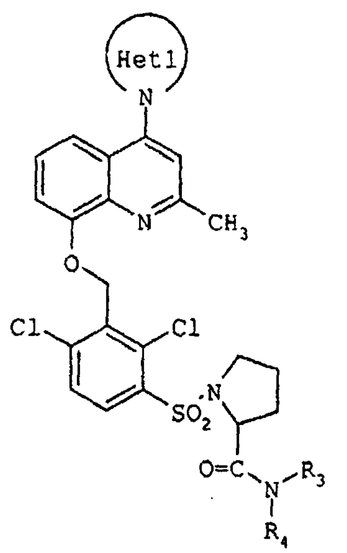

dans laquelle :

Het1 représente un groupe 1-imidazolyle, un groupe 1-pyrazolyle ou un groupe 1-(1,2,4-triazolyle),

$R_3$ représente H, ou un groupe alkyle en $C_1$-$C_3$,
$R_4$ représente un groupe porteur d'une fonction amine primaire ou secondaire choisi parmi : $-(CH_2)_n-CH_2-NHR_6$ ou

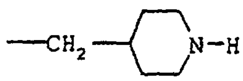

où $R_6$ représente H ou un groupe alkyle et n représente 1, 2, 3 ou 4, avec un composé halogéné de formule :

$$Y-(CH_2)_m-CH_2OR_{13},$$

$$Y-(CH_2)_m-COOR_{11}, \text{ ou}$$

$$Y-(CH_2)_m-CH_2-O-(CH_2)_m-CH_2OR_{13},$$

où

Y est un halogène, préférentiellement Br ou I,
m représente 1, 2, ou 3,
$R_{11}$ est un groupe acidoprotecteur, notamment *t*-butyle, et
$R_{13}$ est un groupe protecteur de la fonction alcool, notamment le groupe acétyle,
dans un solvant, en présence d'un agent alcalin, à température voisine de la température ambiante, pendant 5 à 20 heures, pour obtenir le composé de formule VII :

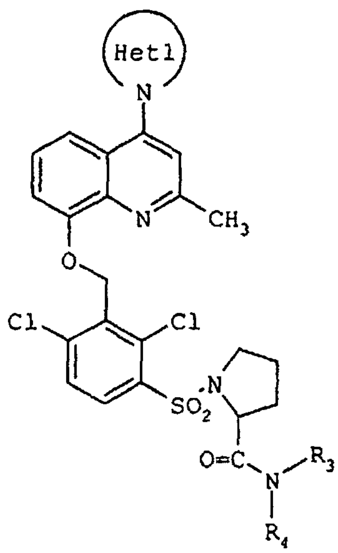
(VII)

dans laquelle :

$R_3$ représente H ou un groupe alkyle en $C_1$-$C_3$,
$R_4$ représente un groupe $-(CH_2)_n-CH_2-NR_5R_6$ ou

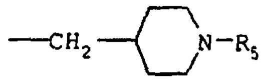

$R_5$ représente un groupe :

- $(CH_2)_m$-$CH_2OR_{13}$,
- $(CH_2)_m$-$COOR_{11}$, ou
- $(CH_2)_m$-$CH_2$-O-$(CH_2)_m$-$CH_2OR_{13}$,

Het1, $R_6$, $R_{11}$ et $R_{13}$ conservant la même signification que ci-dessus ;

(2) effectuer une réaction de déprotection de chaque fonction alcool ou acide afin de remplacer $R_{13}$ et $R_{11}$ par un atome d'hydrogène, et ainsi obtenir les composés de formule I correspondants ; et,
(3) si nécessaire, faire réagir le composé de formule I, ainsi obtenu, avec un acide minéral ou organique pour obtenir le sel correspondant.

**7.** Procédé de préparation d'un composé de formule I selon la revendication 1, **caractérisé en ce qu'**il comprend les étapes consistant à:

(1) faire réagir le chlorure d'acide de formule VIII :

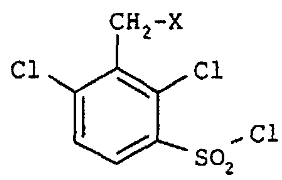

(VIII)

dans laquelle :

X représente un halogène, préférentiellement le brome,
avec un dérivé hétérocyclique répondant à la formule :

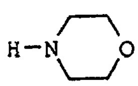

,

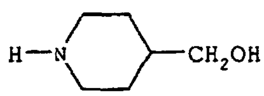

ou

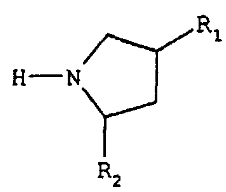

où:

$R_1$ représente H, OH, alcoxy, phénoxy, phénylméthoxy, $CH_2OH$, cycloalkyloxy en $C_3$-$C_8$ ou cycloalkylalcoxy où le fragment cycloalkyle est en $C_3$-$C_8$ et le fragment alcoxy en $C_1$-$C_4$,
$R_2$ représente un groupe -$CH_2OH$, -$CH_2OCH_3$, -CONH$(CH_2)_n$$CH_2NR_5R_{12}$, -CONH$(CH_2)_n$$CH_2OH$, -CONH$(CH_2)_n$$COOR_{11}$ ou

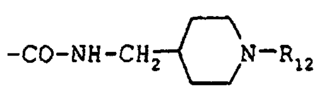

,

n = 1, 2, 3 ou 4,

$R_5$ représente H ou un groupe alkyle,
$R_{11}$ représente un groupe acidoprotecteur, et
$R_{12}$ représente un groupe aminoprotecteur,
dans un solvant, en présence d'une base, à une température proche de la température ambiante, pendant 10 à 30 heures, pour obtenir un composé de formule IX :

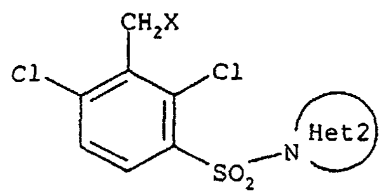

(IX)

dans laquelle :

Het2 représente un groupe

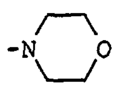 , 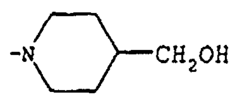 ou 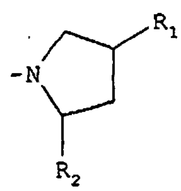

et X, $R_1$, $R_2$, $R_{11}$, $R_{12}$ et n conservent la même signification que ci-dessus ;

(2) faire réagir le composé de formule IX, ainsi obtenu, avec un dérivé de la 8-hydroxyquinoléine de formule II :

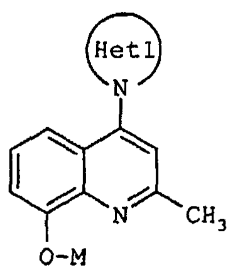

(II)

dans laquelle :

Het1 représente un hétérocycle azoté à cinq sommets comprenant 1, 2, 3 ou 4 atomes d'azote et M représente un métal alcalin,
dans un solvant anhydre, à une température comprise entre 0 et 50°C, pendant 0,5 à 10 heures, pour obtenir un composé de formule X :

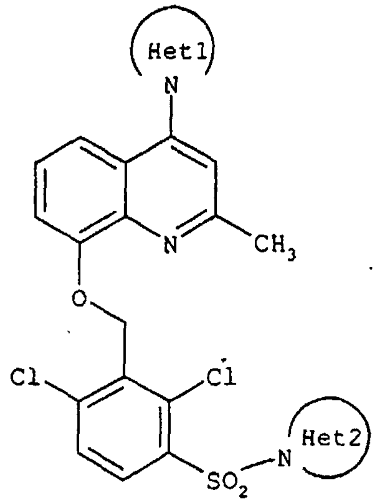

(X)

dans laquelle :

Het1 et Het2 conservent la même signification que ci-dessus ;

(3) si nécessaire, effectuer une réaction de déprotection pour remplacer $R_{11}$ et $R_{12}$ par un atome d'hydrogène, afin d'obtenir un composé de formule I :

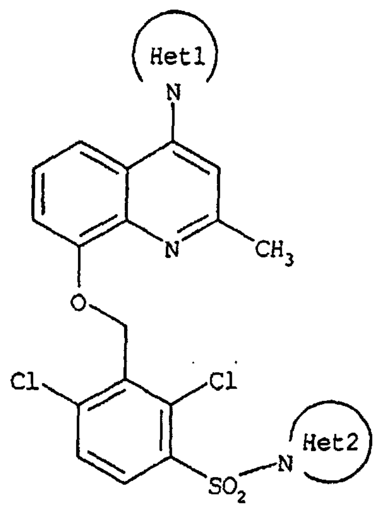

dans laquelle :

Het1 conserve la même signification que ci-dessus, et
Het2 représente un groupe

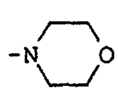

,

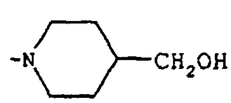

ou

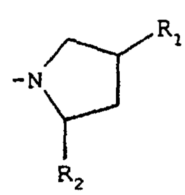

$R_1$ a la même signification que ci-dessus,
$R_2$ représente un groupe $-CH_2OH$, $-CH_2OCH_3$, $-CONH(CH_2)_nCH_2NHR_5$, $-CONH(CH_2)_nCH_2OH$, $-CONH(CH_2)_nCOOH$ ou

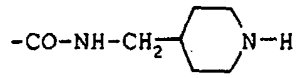

n = 1, 2, 3 ou 4, et

$R_5$ représente H ou un groupe alkyle ; et,

(4) si nécessaire, faire réagir le composé de formule I, ainsi obtenu, avec un acide pour obtenir le sel correspondant.

**8.** Composition thérapeutique **caractérisée en ce qu'**elle renferme, en association avec un excipient physiologiquement acceptable, au moins un composé choisi parmi l'ensemble constitué par les composés de formule I et leurs sels d'addition non toxiques selon la revendication 1.

**9.** Utilisation d'une substance antagoniste d'un récepteur de la bradykinine et des hormones analogues, ladite utilisation étant **caractérisée en ce que** l'on fait appel à une substance antagoniste du récepteur $B_2$ de la bradykinine et choisie parmi l'ensemble constitué par les composés de formule I et leurs sels d'addition non toxiques selon la revendication 1, pour l'obtention d'un médicament destiné à une utilisation en thérapeutique vis-à-vis d'états pathologiques impliquant la bradykinine ou ses homologues.

**10.** Utilisation suivant la revendication 9, **caractérisée en ce que** ledit médicament est destiné à une utilisation en thérapeutique pour le traitement d'états douloureux.

**11.** Utilisation suivant la revendication 9, **caractérisée en ce que** ledit médicament est destiné à une utilisation en thérapeutique pour le traitement d'états inflammatoires.

**12.** Utilisation suivant la revendication 9, **caractérisée en ce que** ledit médicament est destiné à une utilisation en thérapeutique vis-à-vis des traumatismes causés par un choc sévère.

**Patentansprüche**

**1.** Heterocyclische Benzolsulfonamid-Verbindung, **dadurch gekennzeichnet, dass** sie ausgewählt ist aus:

(i) Verbindungen der Formel I:

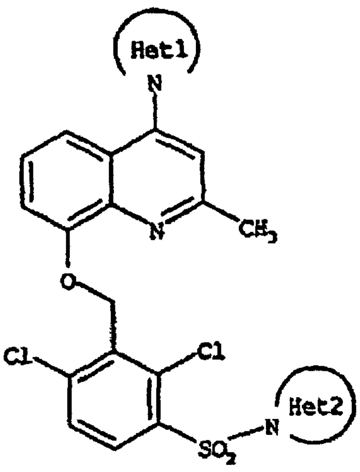

wobei:

Het1 einen 5-gliedrigen Stickstoff-Heterocyclus, insbesondere Imidazol, Pyrazol oder Triazol, darstellt,
Het2 einen 4-, 5- oder 6-gliedrigen Stickstoff-Heterocyclus der Struktur

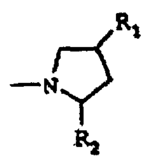 , 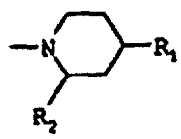 , 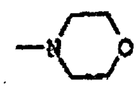 ,

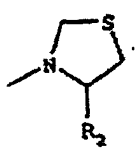 , 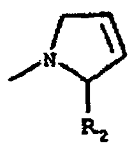 oder 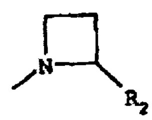

darstellt,
wobei
$R_1$ ein Wasserstoffatom oder eine Hydroxygruppe, einen $C_1$-$C_4$-Alkoxyrest, eine Phenoxygruppe, eine Phenylmethoxygruppe, eine Gruppe $-CH_2OH$, einen Cycloalkyloxyrest, einen Cycloalkylalkoxyrest (wobei jede Cycloalkyleinheit eine $C_3$-$C_8$-Einheit ist und die Alkoxyeinheit eine $C_1$-$C_4$-Einheit ist) oder eine Gruppe $-NH-CO-CH_3$, $-CO-NH_2$ oder $-CO-NH-CH_3$ darstellt,
$R_2$ ein Wasserstoffatom oder eine Gruppe bzw. einen Rest $-CH_2OH$, $-CH_2-O-CH_3$, $-CONR_3R_4$,

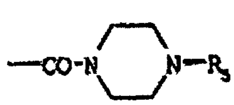 oder 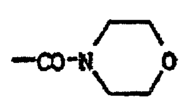

darstellt,
$R_3$ ein Wasserstoffatom, einen $C_1$-$C_3$-Alkylrest, einen $C_3$-$C_8$-Cycloalkylrest, einen $C_3$-$C_8$-Cycloalkyl-$C_1$-$C_3$-alkylrest, eine Phenylgruppe oder eine Phenylmethylgruppe darstellt,

$R_4$ ein Wasserstoffatom, einen $C_1$-$C_3$-Alkylrest oder einen Rest $-(CH_2)_n-CH_2OH$, $-(CH_2)_n-COOH$, $-(CH_2)_n-CH_2-NR_5R_6$,

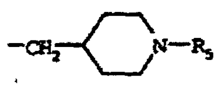 , 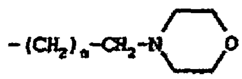 oder 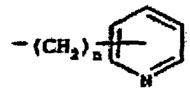

darstellt,
$R_5$ ein Wasserstoffatom, einen $C_1$-$C_3$-Alkylrest oder eine Phenyl-, Phenylmethyl-, Pyridinyl-, Pyridinylmethyl-, Pyridinylethyl-, Benzoyl- oder 4-(Aminoiminomethyl)benzoylgruppe oder einen Rest $-(CH_2)_m-CH_2OH$, $-(CH_2)_m-COOH$, $-(CH_2)_mCH_2-O-(CH_2)_m-CH_2OH$, $-CO-(CH_2)_m-COOH$ oder

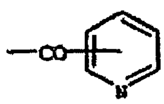

darstellt,
$R_6$ ein Wasserstoffatom oder einen $C_1$-$C_3$-Alkylrest darstellt,
oder $R_5$ und $R_6$ zusammen mit dem Stickstoffatom, an das sie gebunden sind, einen 5- bis 6-gliedrigen N-Hetrocyclus bilden,

$$n = 1, 2, 3 \text{ oder } 4 \text{ ist,}$$

$$m = 1, 2 \text{ oder } 3 \text{ ist; und}$$

(ii) ihren Additionssalzen.

2. Verbindung der Formel I gemäß Anspruch 1, **dadurch gekennzeichnet, dass** Het1 eine 1-(1*H*)-Imidazolylgruppe darstellt.

3. Verbindung der Formel I gemäß einem der Ansprüche 1 oder 2, **dadurch gekennzeichnet, dass** Het2 einen 2(S)-Pyrrolidincarboxamidrest

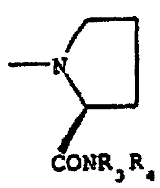

darstellt,

$R_3$ ein Wasserstoffatom oder einen $C_1$-$C_3$-Alkylrest darstellt,
$R_4$ einen $C_1$-$C_3$-Alkylrest, einen Rest $-(CH_2)_n-CH_2-NR_5R_6$, eine Pyridinylmethylgruppe oder einen Rest

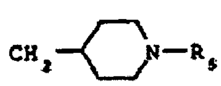

darstellt,
$R_5$ einen Rest $(CH_2)_m$-$CH_2OH$, eine Pyridinylmethylgruppe oder eine 4-(Aminoiminomethyl)benzoylgruppe darstellt,
$R_6$ eine Methylgruppe darstellt oder mit $R_5$ und dem Stickstoffatom, an das sie gebunden sind, einen gesättigten 5- oder 6-gliedrigen Heterocyclus bildet.

**4.** Verbindung der Formel I gemäß einem der Ansprüche 1 oder 2, **dadurch gekennzeichnet, dass** Het2 einen 2(S)-Pyrrolidincarboxamidrest der Formel

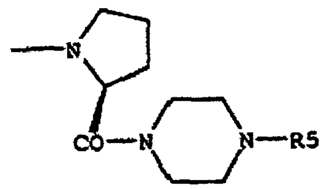

darstellt,
wobei $R_5$ eine Pyridinylgruppe oder eine Pyridinylmethylgruppe darstellt.

**5.** Verfahren zur Herstellung einer Verbindung der Formel I, **dadurch gekennzeichnet, dass** es die folgenden Schritte umfasst:

(1) Umsetzen eines 8-Hydroxychinolin-Derivats der Formel II:

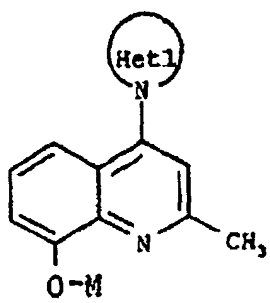

(II)

wobei:

Het1 einen fünfgliedrigen Stickstoff-Heterocyclus darstellt, der insgesamt 1, 2, 3 oder 4 Stickstoffatome umfasst und M ein Alkalimetall, insbesondere Natrium oder Kalium, darstellt,
mit einer Verbindung der Formel III:

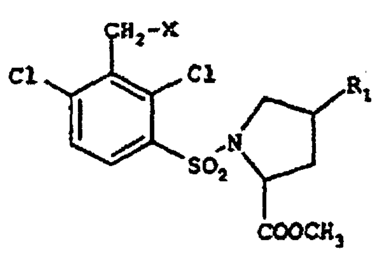

(III)

wobei:

X ein Halogenatom, vorzugsweise ein Bromatom, darstellt und
$R_1$ ein Wasserstoffatom, eine OH-Gruppe, einen Alkoxyrest oder eine Phenoxygruppe darstellt,
in einem wasserfreien Lösungsmittel, bei einer Temperatur zwischen 0 und 50°C, für 0,5 bis 10 Stunden, zu einer Verbindung der Formel IV:

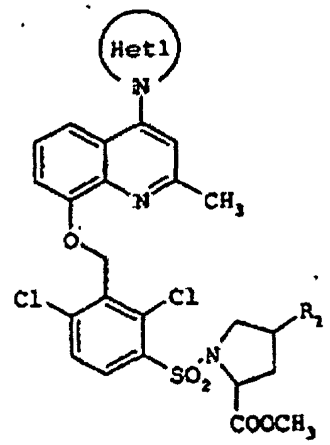

(IV)

wobei:

Het1 und $R_1$ die zuvor angegebene Bedeutung haben;

(2) Hydrolysieren der Esterfunktion der so dargestellten Verbindung der Formel IV zu einer Verbindung der Formel V:

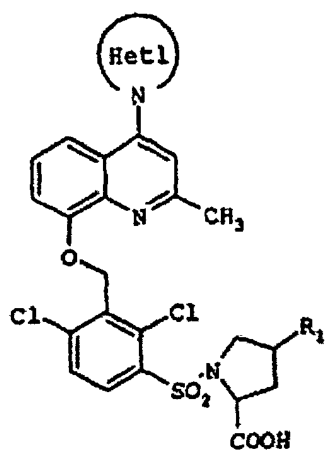

(V)

wobei:

Het1 und $R_1$ die oben angegebene Bedeutung haben;

(3) Umsetzen der so dargestellten Verbindung der Formel V mit einem Amin der Formel:

$$HNR_3R_4 \quad (VI)$$

wobei:

$R_3$ ein Wasserstoffatom oder einen $C_1$-$C_3$-Alkylrest darstellt,
$R_4$ ein Wasserstoffatom, einen $C_1$-$C_3$-Alkylrest oder einen Rest $-(CH_2)_n-CH_2OH$, $-(CH_2)_n-COOR_{11}$, $-(CH_2)_n-CH_2-NR_5R_6$,

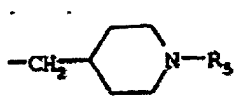 , 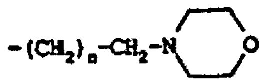 oder 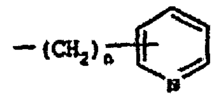

darstellt,
$R_5$ einen $C_1$-$C_3$-Alkylrest, einen Rest $-(CH_2)_m-CH_2OH$, $-(CH_2)_m-COOR_{11}$, $-(CH_2)_m-CH_2-O-(CH_2)_m-CH_2OH$ oder eine Aminoschutzgruppe darstellt (wobei $R_5$ und $R_6$ nicht gleichzeitig als Aminoschutzgruppen fungieren),
$R_6$ einen $C_1$-$C_3$-Alkylrest oder eine Aminoschutzgruppe darstellt,
$R_{11}$ eine leicht hydrolysierbare Schutzgruppe der Säurefunktion darstellt,

n= 1, 2, 3 oder 4 ist,

m = 1, 2 oder 3 ist,

in einem Lösungsmittel, in Gegenwart von Aktivatoren, bei einer Temperatur nahe der Raumtemperatur (0-40°C, vorzugsweise 10-35°C), für 2 bis 50 Stunden, zu einer Verbindung der Formel:

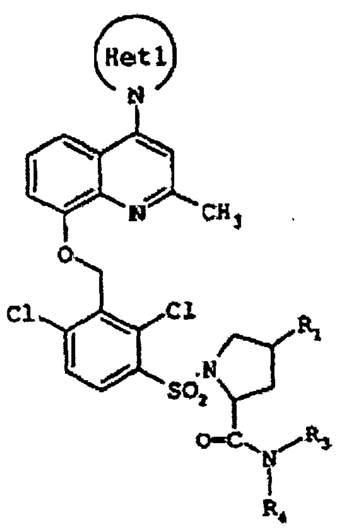 (VII)

wobei:

Het1, $R_1$, $R_3$ und $R_4$ die zuvor angegebene Bedeutung haben; und

(4) gegebenenfalls Umsetzen der so dargestellten Verbindung der Formel VII, um jede der Amino- oder Säureschutzgruppen durch ein Wasserstoffatom zu ersetzen, so dass eine Verbindung der Formel I' erhalten wird:

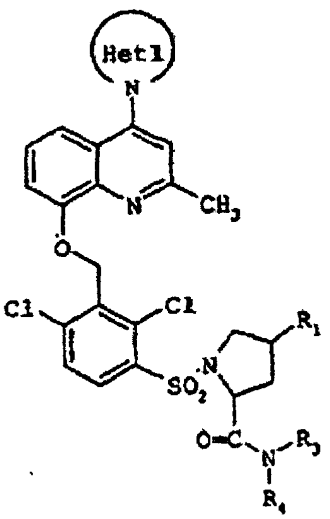

(I')

wobei:

Het1, $R_1$, $R_3$ und $R_4$, mit Ausnahme der durch Wasserstoffatome ersetzten Schutzgruppen die oben angegebene Bedeutung haben; dann,

(5) gegebenenfalls Umsetzen der so dargestellten Verbindung der Formel I' mit einer Säure zum Additionssalz der entsprechenden Säure.

**6.** Verfahren zur Herstellung einer Verbindung der Formel I gemäß Anspruch 1, **dadurch gekennzeichnet, dass** es die folgenden Schritte umfasst:

(1) Umsetzen einer wie in Schritt (4) des Verfahrens gemäß obigem Anspruch 5 gewonnenen Verbindung der Formel I':

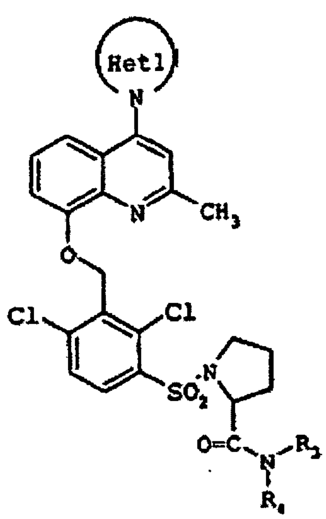

wobei:

Het1 eine 1-Imidazolylgruppe, eine 1-Pyrazolylgruppe oder eine 1-(1,2,4-Triazolyl)gruppe darstellt,
$R_3$ ein Wasserstoffatom oder einen $C_1$-$C_3$-Alkylrest darstellt,

$R_4$ einen Rest darstellt, der eine primäre oder sekundäre Aminfunktion aufweist, ausgewählt aus: $-(CH_2)_n-CH_2-NHR_6$ oder

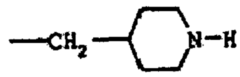

darstellt,

wobei $R_6$ ein Wasserstoffatom oder einen Alkylrest darstellt und n 1, 2, 3 oder 4 ist, mit einer Halogenverbindung der Formel:

$$Y-(CH_2)_m-CH_2OR_{13}$$

$$Y-(CH_2)_m-COOR_{11} \text{ oder}$$

$$Y-(CH_2)_m-CH_2-O-(CH_2)_m-CH_2OR_{13},$$

wobei

Y ein Halogenatom, vorzugsweise ein Brom- oder Iodatom, darstellt,
m 1, 2 oder 3 ist,
$R_{11}$ eine Säureschutzgruppe, insbesondere eine *t*-Butylgruppe, darstellt und
$R_{13}$ eine Schutzgruppe der Alkoholfunktion, insbesondere eine Acetylgruppe, darstellt,
in einem Lösungsmittel, in Gegenwart eines alkalischen Agens, bei einer Temperatur nahe der Raumtemperatur, für 5 bis 20 Stunden, zu einer Verbindung der Formel VII:

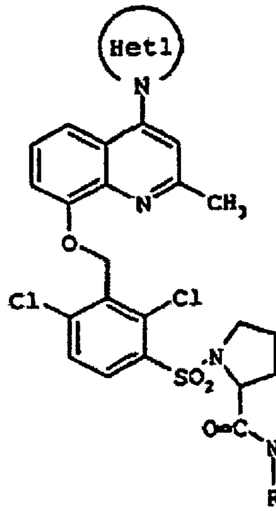

(VII)

wobei:

$R_3$ ein Wasserstoffatom oder einen $C_1$-$C_3$-Alkylrest darstellt,
$R_4$ einen Rest $-(CH_2)_n-CH_2-NR_5R_6$ oder

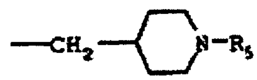

darstellt,
$R_5$ einen Rest:

- $(CH_2)_m$-$CH_2OR_{13}$,
- $(CH_2)_m$-$COOR_{11}$ oder
- $(CH_2)_m$-$CH_2$-O-$(CH_2)_m$-$CH_2OR_{13}$ darstellt,

wobei Het1, $R_6$, $R_{11}$ und $R_{13}$ die oben angegebene Bedeutung haben;

(2) Durchführen einer Reaktion zur Entschützung jeder der Alkohol- oder Säurefunktionen, um $R_{13}$ und $R_{11}$ durch ein Wasserstoffatom zu ersetzen und so die entsprechenden Verbindungen der Formel I zu erhalten; und
(3) gegebenenfalls Umsetzen der so dargestellten Verbindung der Formel I mit einer Mineralsäure oder einer organischen Säure zum entsprechenden Salz.

**7.** Verfahren zur Herstellung einer Verbindung der Formel I gemäß Anspruch 1, **dadurch gekennzeichnet, dass** es die folgenden Schritte umfasst:

(1) Umsetzen eines Säurechlorids der Formel VIII:

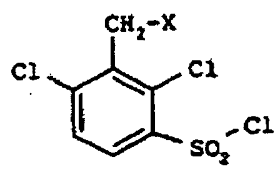

(VIII)

wobei:

X ein Halogenatom, vorzugsweise Brom, darstellt,
mit einem heterocyclischen Derivat der Formel:

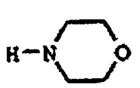 , 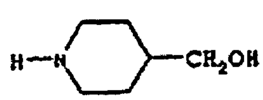 oder 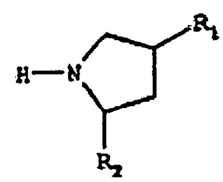

wobei:

$R_1$ ein Wasserstoffatom, eine Hydroxygruppe, einen Alkoxyrest, eine Phenoxygruppe, eine Phenylmethoxygruppe, eine Gruppe $CH_2OH$, einen $C_3$-$C_8$-Cycloalkyloxyrest oder einen Cycloalkylalkoxyrest mit einer $C_3$-$C_8$-Cycloalkyleinheit und einer $C_1$-$C_4$ Alkoxyeinheit darstellt,
$R_2$ eine Gruppe -$CH_2OH$ oder -$CH_2OCH_3$ oder einen Rest -$CONH(CH_2)_nCH_2NR_5R_{12}$, -$CONH(CH_2)_nCH_2OH$, -$CONH(CH_2)_nCOOR_{11}$ oder

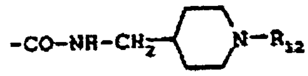

darstellt,

n= 1, 2, 3 oder 4 ist,

$R_5$ ein Wasserstoffatom oder einen Alkylrest darstellt,
$R_{11}$ eine Säureschutzgruppe darstellt und
$R_{12}$ eine Aminoschutzgruppe darstellt,
in einem Lösungsmittel, in Gegenwart einer Base, bei einer Temperatur nahe der Raumtemperatur, für 10 bis 30 Stunden, zu einer Verbindung der Formel IX:

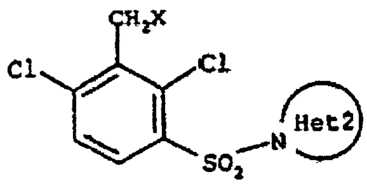

(IX)

wobei:
Het2 eine Gruppe bzw. einen Rest

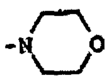

,

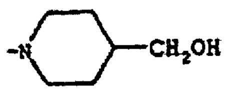

oder

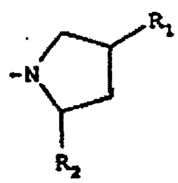

darstellt
und X, $R_1$, $R_2$, $R_{11}$, $R_{12}$ und n die oben angegebene Bedeutung haben;

(2) Umsetzen der so dargestellten Verbindung der Formel IX mit einem 8-Hydrochinolin-Derivat der Formel II:

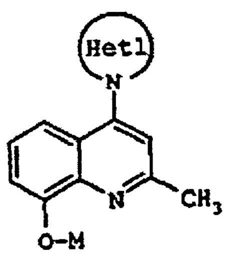

(II)

wobei:

Het1 einen fünfgliedrigen Stickstoff-Heterocyclus darstellt, der 1, 2, 3 oder 4 Stickstoffatome umfasst und
M ein Alkalimetall darstellt,

in einem wasserfreien Lösungsmittel, bei einer Temperatur zwischen 0 und 50°C, für 0,5 bis 10 Stunden, zu einer Verbindung der Formel X:

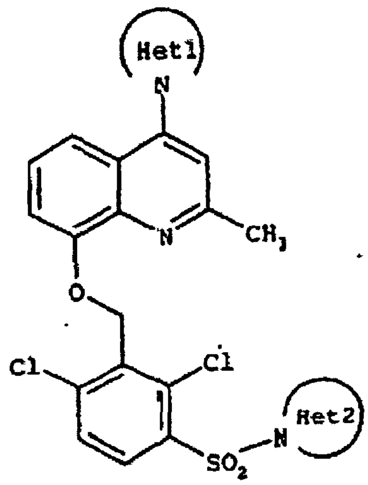

(X)

wobei:

Het1 und Het2 die oben angegebene Bedeutung haben;

(3) gegebenenfalls Durchführen einer Entschützungsreaktion, um $R_{11}$ und $R_{12}$ durch ein Wasserstoffatom zu ersetzen, um eine Verbindung der Formel I zu erhalten:

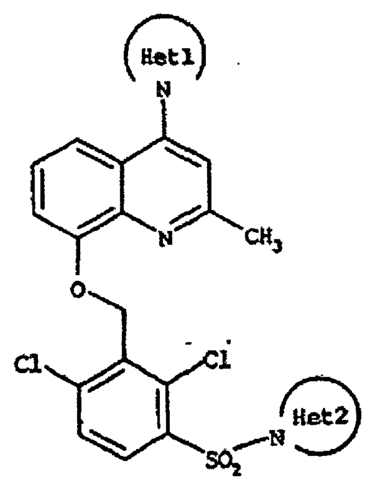

wobei:

Het1 die oben angegebene Bedeutung hat und
Het2 eine Gruppe bzw. einen Rest

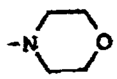 , 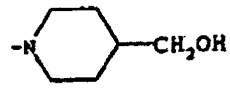 oder 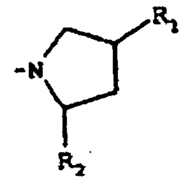

darstellt,

$R_1$ die oben angegebene Bedeutung hat,

$R_2$ eine Gruppe $-CH_2OH$ oder $-CH_2OCH_3$, einen Rest $-CONH(CH_2)_nCH_2NHR_5$, $-CONH(CH_2)_nCH_2OH$ oder $-CONH(CH_2)_nCOOH$ oder eine Gruppe

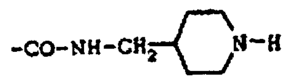

darstellt,

n = 1, 2, 3 oder 4 ist und

$R_5$ ein Wasserstoffatom oder einen Alkylrest darstellt; und

(4) gegebenenfalls Umsetzen der so dargestellten Verbindung der Formel I mit einer Säure zu dem entsprechenden Salz.

**8.** Therapeutische Zusammensetzung, **dadurch gekennzeichnet, dass** sie, in Verbindung mit einem physiologisch verträglichen Exzipienten, mindestens eine Verbindung ausgewählt aus Verbindungen der Formel I gemäß Anspruch 1 und ihren nicht-toxischen Additionssalzen einschließt.

**9.** Verwendung einer antagonistisch auf den Bradykininrezeptor und Rezeptoren analoger Hormone wirkenden Substanz, wobei die Verwendung **dadurch gekennzeichnet ist, dass** man dabei auf eine antagonistisch auf den $B_2$-Rezeptor des Bradykinin wirkende Substanz, ausgewählt aus Verbindungen der Formel I und ihren nicht-toxischen Additionssalzen gemäß Anspruch 1 zurückgreift, zur Herstellung eines Medikaments zur Verwendung in der Therapie von pathologischen Zuständen, in die Bradykinin oder seine Homologen involviert sind.

**10.** Verwendung gemäß Anspruch 9, **dadurch gekennzeichnet, dass** das Medikament zum therapeutischen Einsatz bei der Behandlung schmerzhafter Zustände vorgesehen ist.

**11.** Verwendung gemäß Anspruch 9, **dadurch gekennzeichnet, dass** das Medikament zum therapeutischen Einsatz bei der Behandlung entzündlicher Zustände vorgesehen ist.

**12.** Verwendung gemäß Anspruch 9, **dadurch gekennzeichnet, dass** das Medikament zum Einsatz in der Therapie von durch einen schweren Schock verursachten Traumata vorgesehen ist.

## Claims

**1.** Heterocyclic benzenesulphonamide compound, **characterized in that** it is chosen from the group formed by:

(i) compounds of formula I:

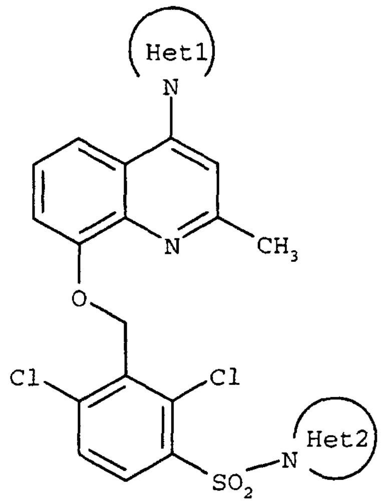

(I)

in which:

Het1 represents a 5-membered nitrogen heterocycle, especially imidazole, pyrazole or triazole,
Het2 represents a 4-, 5- or 6-membered nitrogen heterocycle of structure:

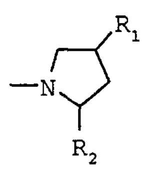 , 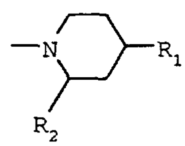 , 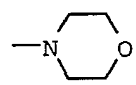 ,

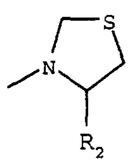 , 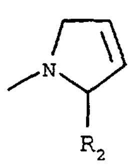 or 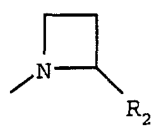

in which
$R_1$ represents a hydrogen atom or a hydroxyl, $C_1$-$C_4$ alkoxy, phenoxy, phenylmethoxy, $-CH_2OH$, cycloalkyloxy, cycloalkylalkoxy (where each cycloalkyl fragment is $C_3$-$C_8$ and the alkoxy fragment is $C_1$-$C_4$), $-NH-CO-CH_3$, $-CO-NH_2$ or $-CO-NH-CH_3$ group,
$R_2$ represents a hydrogen atom or a $-CH_2OH$, $-CH_2-O-CH_3$, $-CONR_3R_4$ group,

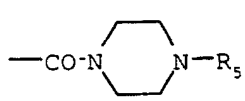 or 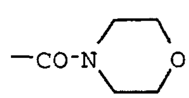

$R_3$ represents a hydrogen atom, a $C_1$-$C_3$ alkyl group, a $C_3$-$C_8$ cycloalkyl group, a ($C_3$-$C_8$) cycloalkyl ($C_1$-$C_3$) alkyl group, a phenyl group, or a phenylmethyl group,
$R_4$ represents a hydrogen atom, a $C_1$-$C_3$ alkyl, $-(CH_2)_n-CH_2OH$, $-(CH_2)n-COOH$, $-(CH_2)n-CH_2-NR_5R_6$,

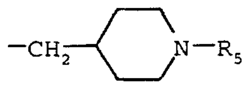 , 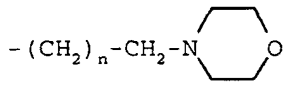 or 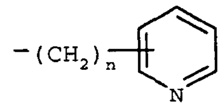

group,
$R_5$ represents a hydrogen atom, a $C_1$-$C_3$ alkyl, phenyl, phenylmethyl, pyridinyl, pyrinylmethyl, pyridinylethyl, benzoyl, 4-(aminoiminomethyl)benzoyl, $-(CH_2)_m-CH_2OH$, $-(CH_2)_m-COOH$, $-(CH_2)_mCH_2-O-(CH_2)_m-CH_2OH$, $-CO-(CH_2)_m-COOH$, or

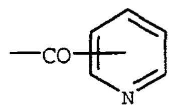

group,
$R_6$ represents a hydrogen atom or a $C_1$-$C_3$ alkyl group, or, $R_5$ and $R_6$ considered together form, with the nitrogen atom to which they are attached, a 5- to 6-membered N heterocycle,

n = 1, 2, 3 or 4,

m = 1, 2 or 3; and

(ii) their addition salts.

**2.** Compound of formula I according to Claim 1, **characterized in that** Het 1 represents a 1-(1*H*)-imidazolyl group.

**3.** Compound of formula I according to one of Claims 1 and 2, **characterized in that** Het2 represents a 2(S)-pyrrolidinecarboxamide group

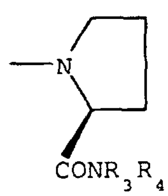

$R_3$ represents a hydrogen atom or a $C_1$-$C_3$ alkyl group,
$R_4$ represents a $C_1$-$C_3$ alkyl group, a $-(CH_2)n-CH_2-NR_5R_6$ group, a pyridinylmethyl group, or a

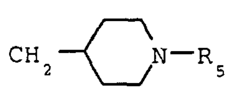

group
$R_5$ represents a $(CH_2)_m-CH_2OH$ group, a pyridinylmethyl group or a 4-(aminoiminomethyl)benzoyl group,
$R_6$ represents a methyl group or forms, with $R_5$ and the nitrogen to which they are bonded, a 5- or 6-membered saturated heterocycle.

**4.** Compound of formula I according to one of Claims 1 and 2, **characterized in that** Het2 represents a 2(S)-pyrrolidinecarboxamide group of formula

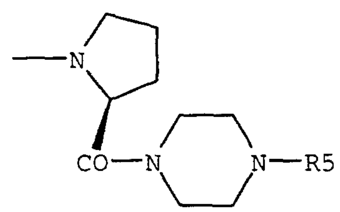

in which $R_5$ represents a pyridinyl group or a pyridinylmethyl group.

**5.** Method of preparation of a compound of formula I, **characterized in that** it comprises the steps consisting of:

(1) reacting an 8-hydroxyquinoline derivative of formula II:

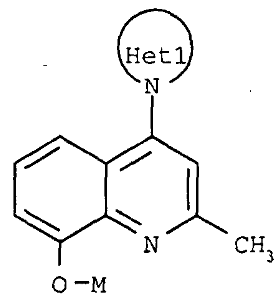

(II)

in which:

Het1 represents a five-membered nitrogen heterocycle comprising in total 1, 2, 3 or 4 nitrogen atoms and M represents an alkali metal, especially sodium or potassium,
with a compound of formula III:

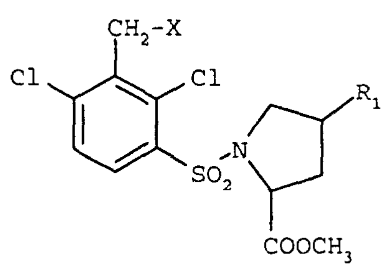

(III)

in which:

X represents a halogen atom, preferably a bromine atom, and
$R_1$ represents a hydrogen atom or an OH group, an alkoxy group or a phenoxy group,
in an anhydrous solvent, at a temperature of between 0 and 50°C, for 0.5 to 10 hours, in order to obtain a compound of formula IV:

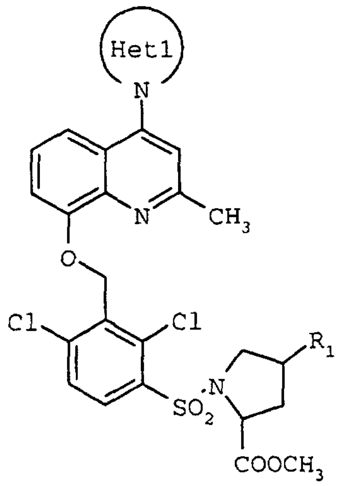

(IV)

in which:

Het1 and $R_1$ retain the same meaning as previously;

(2) hydrolysing the ester function of the compound of formula IV thus obtained in order to obtain a compound of formula V:

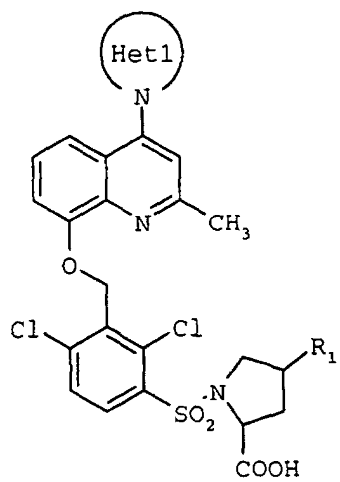

(V)

in which:

Het1 and $R_1$ retain the same meaning as above;

(3) reacting the compound of formula V thus obtained with an amine of formula:

$$HNR_3R_4 \quad (VI)$$

in which:

$R_3$ represents a hydrogen atom or a $C_1$-$C_3$ alkyl group,

$R_4$ represents a hydrogen atom, a $C_1$-$C_3$ alkyl, $-(CH_2)_n-CH_2OH$, $-(CH_2)_n-COOR_{11}$, $-(CH_2)_n-CH_2-NR_5R_6$,

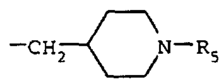 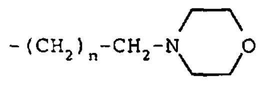 or 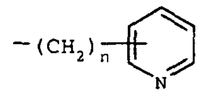

group,

$R_5$ represents a $C_1$-$C_3$ alkyl $-(CH_2)_m-CH_2OH$, $-(CH_2)_m-COOR_{11}$, $-(CH_2)_m-CH_2-O-(CH_2)_m-CH_2OH$ group, or an amino-protective group ($R_5$ and $R_6$ not simultaneously being amino-protective groups),

$R_6$ represents a $C_1$-$C_3$ alkyl group or an amino-protecting group,

$R_{11}$ represents an acid-protecting group of the acid function, which is easily hydrolysable,

$$n = 1, 2, 3 \text{ or } 4,$$

$$m = 1, 2 \text{ or } 3,$$

in a solvent, in the presence of activators, at a temperature close to room temperature (0-40°C, preferably 10-35°C), for 2 to 50 hours, in order to obtain a compound of formula:

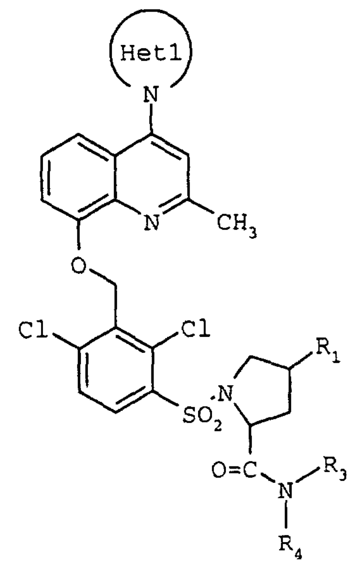

(VII)

in which

Het1, $R_1$, $R_3$, $R_4$ keep the same meaning as previously; and

(4) if necessary, reacting the compound of formula VII thus obtained in order to replace each amino- or acid-protecting group by a hydrogen atom, so as to obtain the compound of formula I:

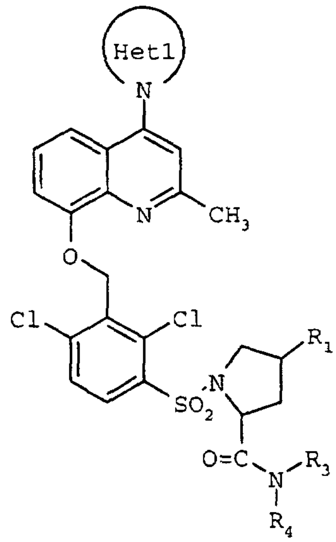

(I)

in which:

Het1, $R_1$, $R_3$ and $R_4$ keep the same meaning as above, with the exception of the protective groups replaced by hydrogen atoms;

then,

(5) if necessary, reacting the compound of formula I thus obtained with an acid in order to obtain the corresponding acid addition salt.

**6.** Method of preparation of a compound of formula I according to Claim 1, **characterized in that** it comprises the steps consisting of:

(1) reacting a compound of formula I such as obtained in step (4) of the method according to Claim 5 above,

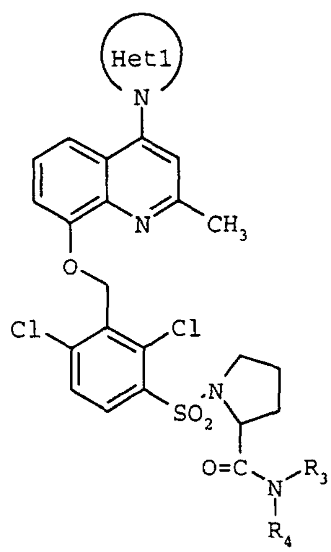

in which:

Hetl represents a 1-imidazolyl group, a 1-pyrazolyl group or a 1-(1,2,4-triazolyl) group,

$R_3$ represents H, or a $C_1$-$C_3$ alkyl group,
$R_4$ represents a group which carries a primary or secondary amino function chosen from: $-(CH_2)_n-CH_2-NHR_6$ or

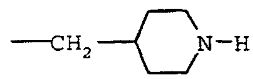

where $R_6$ represents H or an alkyl group and n represents 1, 2, 3 or 4, with a halogenated compound of formula: $Y-(CH_2)_m-CH_2OR_{13}$, $Y-(CH_2)_m-COOR_{11}$, or $Y-(CH_2)_m-CH_2-O-(CH_2)_m-CH_2OR_{13}$, where

Y is a halogen, preferentially Br or I,
m represents 1, 2 or 3
$R_{11}$ is an acid-protecting group, especially *t*-butyl, and
$R_{13}$ is an alcohol protecting group, in particular the acetyl group,
in a solvent, in the presence of an alkaline agent, at a temperature close to room temperature, for 5 to 20 hours, in order to obtain the compound of formula VII:

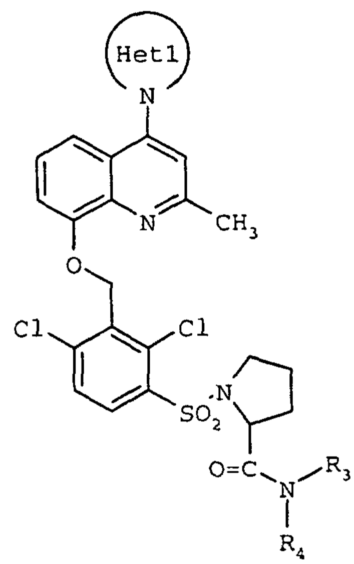

(VII)

in which:

$R_3$ represents H or a $C_1$-$C_3$ alkyl group,
$R_4$ represents a $-(CH_2)_n-CH_2-NR_5R_6$ group or

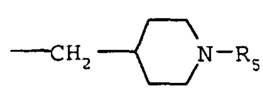

$R_5$ represents a group: $-(CH_2)_m-CH_2OR_{13}$, $-(CH_2)_m-COOR_{11}$, or $-(CH_2)_m-CH_2-O-(CH_2)_m-CH_2OR_{13}$,
Het1, $R_6$, $R_{11}$ and $R_{13}$ keep the same meaning as above;

(2) carrying out a deprotection reaction of each alcohol or acid function in order to replace $R_{13}$ and $R_{11}$ by a hydrogen atom, and thus obtaining the corresponding compounds of formula I;
(3) if necessary, reacting the compound of formula I thus obtained with an inorganic or organic acid in order

to obtain the corresponding salt.

**7.** Method of preparation of a compound of formula I according to Claim 1, **characterized in that** it comprises the steps consisting of:

(1) reacting the acid chloride of formula VIII:

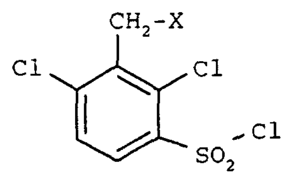

(VIII)

in which:

X represents a halogen, preferentially bromine,
with a heterocyclic derivative corresponding to the formula :

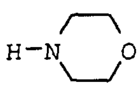 , 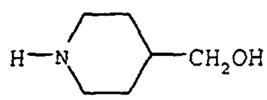 or 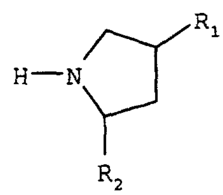

where:

$R_1$ represents H, OH, alkoxy, phenoxy, phenylmethoxy, $CH_2OH$, $C_3$-$C_8$ cycloalkyloxy or cycloalkylalkoxy where the cycloalkyl fragment is $C_3$-$C_8$ and the alkoxy fragment $C_1$-$C_4$,
$R_2$ represents a $-CH_2OH$, $-CH_2OCH_3$, $-CONH(CH_2)_nCH_2NR_5R_{12}$, $-CONH(CH_2)_nCH_2OH$, $-CONH(CH_2)_nCOOR_{11}$ or

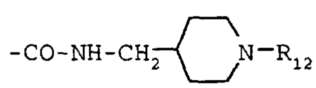

group,

n = 1, 2, 3 or 4,

$R_5$ represents H or an alkyl group,
$R_{11}$ represents an acid-protecting group, and
$R_{12}$ represents an amino-protecting group,
in a solvent, in the presence of a base, at a temperature close to room temperature, for 10 to 30 hours, in order to obtain a compound of formula IX:

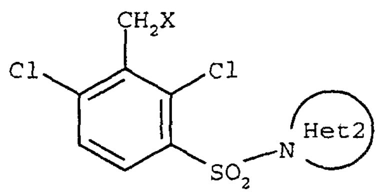

(IX)

in which:

Het2 represents a

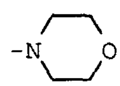 , 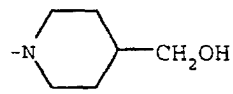 or 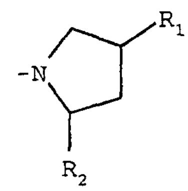

group
and X, $R_1$, $R_2$, $R_{11}$, $R_{12}$ and n keep the same meaning as above;

(2) reacting the compound of formula IX thus obtained with an 8-hydroxyquinoline derivative of formula II:

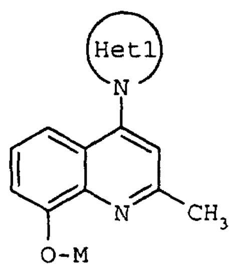

(II)

in which:

Het1 represents a 5-membered nitrogen heterocycle comprising 1, 2, 3 or 4 nitrogen atoms and M represents an alkali metal,
in an anhydrous solvent, at a temperature of between 0 and 50°C, for 0.5 to 10 hours, in order to obtain a compound of formula X:

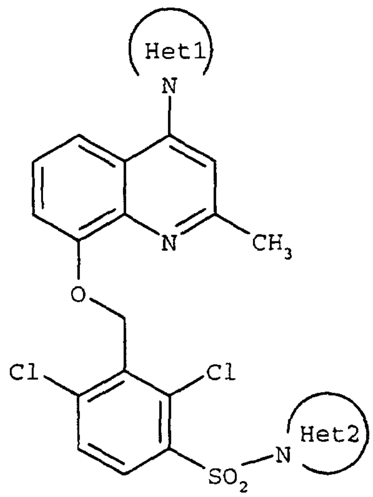

(X)

in which:

Het1 and Het2 retain the same meaning as above;

(3) if necessary, carrying out a deprotection reaction to replace $R_{11}$ and $R_{12}$ by a hydrogen atom, in order to obtain a compound of formula I:

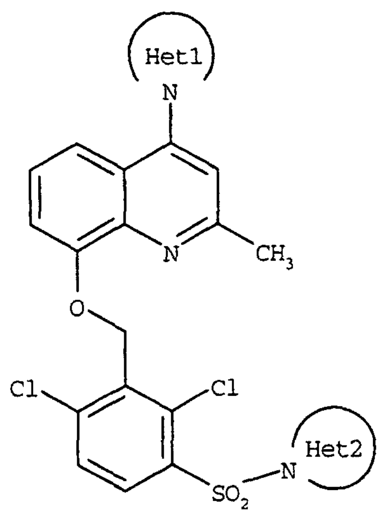

in which:

Het1 retains the same meaning as above, and
Het2 represents a

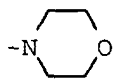 , 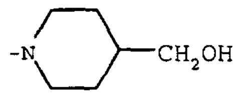 or 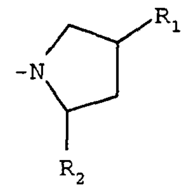

group,
$R_1$ has the same meaning as above,
$R_2$ represents a $-CH_2OH$, $-CH_2OCH_3$, $-CONH(CH_2)_nCH_2NHR_5$, $-CONH(CH_2)_nCH_2OH$, $-CONH(CH_2)_nCOOH$ or

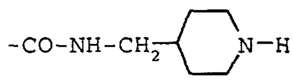

group

n = 1, 2, 3 or 4, and

$R_5$ represents H or an alkyl group; and

(4) if necessary, reacting the compound of formula I thus obtained with an acid in order to obtain the corresponding salt.

**8.** Therapeutic composition, **characterized in that** it comprises, in combination with a physiologically acceptable excipient, at least one compound chosen from the group formed by the compounds of formula I and their non-toxic addition salts according to Claim 1.

**9.** Use of a substance which is an antagonist of a bradykinin receptor and of analogous hormones, the said use being **characterized in that** use is made of a substance which is an antagonist of the bradykinin $B_2$ receptor and is chosen from the group formed by the compounds of formula I and their non-toxic addition salts according to Claim 1 in order to obtain a medicament intended for therapeutic use with respect to pathological states involving bradykinin or its homologues.

**10.** Use according to Claim 9, **characterized in that** the said medicament is intended for therapeutic use for the treatment of painful conditions.

**11.** Use according to Claim 9, **characterized in** the said medicament is intended for therapeutic use for the treatment of inflammatory conditions.

**12.** Use according to Claim 9, **characterized in that** the said medicament is intended for therapeutic use with respect to traumatism caused by a severe shock.